# FASCICULE DE BREVET EUROPEEN

(11) **EP 2 313 412 B1**
(45) Date de publication et mention de la délivrance du brevet: **02.01.2013**
(21) Numéro de dépôt: 09766078.1
(22) Date de dépôt: 16.06.2009
(51) Int. Cl.: C07D 487/04, C07D 471/04, C07D 207/323, C07D 209/40, C07D 233/64, A61K 31/437

(54) **NOUVEAUX DERIVES DE PYRROLOINDOLE INHIBITEURS D'HSP90, COMPOSITIONS LES CONTENANT ET UTILISATION**
NEUE PYRROLOINDOL DERIVATEN ALS HSP90 INHIBITOREN, DEREN ZUSAMMENSETZUNGEN UND VERWENDUNGEN
NOVEL DERIVATIVES OF PYRROLOINDOLE INHIBITORS OF HSP90, COMPOSITIONS CONTAINING SAME, AND USE THEREOF

(30) Priorité: 16.06.2008 FR 0803334
(43) Date de publication de la demande: 27.04.2011
(73) Titulaire: SANOFI, 75008 Paris (FR)
(72) Inventeur: ALASIA, Marcel, F-75013 Paris (FR); MINOUX, Hervé, F-75013 Paris (FR); RUXER, Jean-Marie, F-75013 Paris (FR)
(74) Mandataire: Le Coupanec, Pascale A.M.P.
(86) Numéro de dépôt international: PCT/FR2009/051140
(87) Numéro de publication internationale: WO 2009/153516

(56) Documents cités:
- WO-A-2006/123061
- FR-A- 2 884 252
- JANIN Y L: "Heat Shock Protein 90 Inhibitors. A Text Book Example of Medicinal Chemistry?" JOURNAL OF MEDICINAL CHEMISTRY, AMERICAN CHEMICAL SOCIETY. WASHINGTON.; US, vol. 48, no. 24, 8 novembre 2005 (2005-11-08), pages 7503-7512, XP002373723 ISSN: 0022-2623 cité dans la demande

## Description

La présente invention concerne de nouveaux composés chimiques, dérivés hétérocycliques de pyrrolo[1,2-a]indole, les compositions qui les contiennent, et leur utilisation comme médicaments.

Plus particulièrement, l'invention concerne, selon un premier aspect, de nouveaux dérivés hétérocycliques de pyrrolo[1,2-a]indole présentant une activité anticancéreuse, et en particulier une activité inhibitrice de la protéine chaperone Hsp90, et plus particulièrement via l'inhibition de l'activité catalytique de type ATPasique de la protéine chaperone Hsp90.

### Protéines Chaperones :

Les chaperones moléculaires de la famille « Heat Shock Proteins » (HSPs), classées en fonction de leur masse moléculaire (Hsp27, Hsp70, Hsp90...), sont des éléments clés de l'équilibre entre la synthèse et la dégradation des protéines cellulaires, responsables du repliement correct des protéines. Elles jouent un rôle vital en réponse au stress cellulaire. Les HSPs, et en particulier Hsp90, sont également impliquées dans la régulation de diverses fonctions majeures de la cellule, via leur association avec diverses protéines clients impliquées dans la prolifération cellulaire ou l'apoptose (Jolly C. et Morimoto R.I., J. N. Cancer Inst..(2000), 92, 1564-72 ; Smith D.F. et al., Pharmacological Rev. (1998), 50, 493-513; Smith D.F. , Molecular Chaperones in the Cell, 165-178, Oxford University Press 2001).

### Chaperone Hsp90 et inhibiteurs d'Hsp90 dans le traitement des cancers :

La chaperone Hsp90, qui représente 1 à 2% du contenu protéique de la cellule a été récemment mise en évidence comme une cible particulièrement prometteuse en thérapie anticancéreuse (cf pour revue : Moloney A. et Workman P., Expert Opin. Biol. Ther. (2002), 2(1), 3-24 ; Chiosis et al, Drug Discovery Today (2004), 9, 881-888). Cet intérêt tient en particulier aux interactions cytoplasmiques d'Hsp90 avec les principales protéines clients d'Hsp90, protéines qui sont impliquées dans les six mécanismes de progression des tumeurs, tels que définis par Hanahan D. et Weinberg R.A. (Cell (2002), 100, 57-70), à savoir :
- une capacité à proliférer en l'absence de facteurs de croissance : EGFR-R/HER2, Src, Akt, Raf, MEK, Bcr-Abl, Flt-3 ...
- une capacité à échapper à l'apoptose : forme mutée de p53, Akt, survivine ...
- une insensibilité aux signaux d'arrêt de prolifération : Cdk4, Plk , Wee1 ...
- une capacité à activer l'angiogénèse : VEGF-R, FAK, HIF-1, Akt ...
- une capacité à proliférer sans limite réplicative : hTert ...
- une capacité à envahir de nouveaux tissus et à métastaser : c-Met

Parmi les autres protéines clients de Hsp90, des récepteurs aux hormones stéroïdiennes, tels que le récepteur à l'estrogène ou le récepteur à l'androgène, présentent également un intérêt important dans le cadre des thérapies anticancéreuses.

Il a été montré récemment que la forme alpha d'Hsp90 avait également un rôle extracellulaire via son interaction avec la métalloprotéase MMP-2, elle-même impliquée dans l'invasion tumorale (Eustace B.K. et al, Nature Cell Biology (2004), 6, 507-514).

Hsp90 est constituée de deux domaines N- et C-terminaux séparés par une région fortement chargée. L'interaction dynamique entre ces deux domaines, coordonnée par la fixation de nucléotides et de co-chaperones, détermine la conformation de la chaperone et son état d'activation. L'association des protéines clients dépend principalement de la nature des co-chaperones Hsp70/Hsp40, Hop60 etc ... et de la nature du nucléotide ADP ou ATP liée au domaine N-terminal de Hsp90. Ainsi l'hydrolyse de l'ATP en ADP et le facteur d'échange ADP/ATP contrôlent l'ensemble de la « machinerie » chaperone, et il a été montré qu'il suffit de prévenir l'hydrolyse de l'ATP en ADP - activité ATPasique d'Hsp90 - pour libérer dans le cytoplasme des protéines-clients qui seront alors dégradées au protéasome (Neckers L et Neckers K, Expert Opin. Emerging Drugs (2002), 7, 277-288 ; Neckers L, Current Medicinal Chemistry, (2003), 10, 733-739 ; Piper P.W., Current Opin. Invest. New Drugs (2001), 2, 1606-1610).

### Rôle d'Hsp90 et de ses inhibiteurs dans des pathologies autres que le cancer:

Diverses pathologies humaines sont la conséquence d'un repliement incorrect de protéines clés, conduisant notamment à des maladies neurodégénératives suite à l'agrégation de certaines protéines comme dans les maladies d'Alzheimer et de Huntington ou les maladies liées aux prions (Tytell M. et Hooper P.L., Emerging Ther. Targets (2001), 5, 267- 287). Dans ces pathologies, des approches visant à inhiber Hsp90 dans le but d'activer les voies de stress (Hsp70 par exemple) pourraient être bénéfiques (Nature Reviews Neuroscience 6 : 11, 2005). Quelques exemples sont cités ci-dessous :
i) La maladie de Huntington : Cette maladie neurodégénérative est due à une extension de triplets CAG dans l'exon 1 du gène codant pour la protéine huntingtine. Il a été montré que la geldanamycine inhibe l'agrégation de cette protéine du fait de la surexpression des chaperones Hsp70 et Hsp40 (Human Molecular Genetics 10: 1307, 2001).
ii) La maladie de Parkinson : Cette maladie est due à la perte progressive des neurones dopaminergiques et caractérisée par l'aggrégation de la protéine alpha-synuclein. Il a été montré que la geldanamycine est capable de protéger la drosophile contre la toxicité de l'alpha-synuclein sur les neurones dopaminergiques.
iii) L'ischémie cérébrale focale : Il a été montré dans un modèle animal de rat que la geldanamycine protège le cerveau contre l'ischémie cérébrale, et ce du fait de l'effet de stimulation de la transcription des gènes codant pour les « heat-shock proteins » par un inhibiteur d'Hsp90.
iv) Les maladies d'Alzheimer et la sclérose en plaques : Ces maladies sont dues en partie à l'expression de cytokines pro-inflammatoires et de la forme inductible de la NOS (Nitric oxide synthase) dans le cerveau, et cette expression délétaire est supprimée par la réponse au stress. En particulier, les inhibiteurs d'Hsp90 sont capables d'engranger cette réponse au stress, et il a été montré in vitro que la geldanamycine et le 17-AAG présentent une activité anti-inflammatoire dans des cellules gliales de cerveau (J. Neuroscience Res. 67 : 461, 2002).
v) La sclérose latérale amyotrophigue : Cette maladie neurodégénérative est due à la perte progressive des neurones moteurs. Il a été montré que l'arimoclomol, un inducteur « heat-shock proteins », retarde l'évolution de la maladie dans un modèle animal (Nature Medicine 10: 402, 2004). Etant donné qu'un inhibiteur d'Hsp90 est également un inducteur des protéines heat-shock (Mol. Cell Biol. 19 : 8033, 1999 ; Mol. Cell Biol. 18 : 4949, 1998), il est probable qu'un effet bénéfique pourrait être obtenu également dans cette pathologie pour ce type d'inhibiteurs.

D'autre part, un inhibiteur de la protéine Hsp90 pourrait être potentiellement utile dans diverses maladies, autres que le cancer cité précedemment, telles que des infections parasitaires, virales, fongiques, ou des maladies neurodégénératives et ce par une action directe sur Hsp90 et des protéines clients particulières. Quelques exemples sont présentés ci-dessous :
vi) la malaria : la protéine Hsp90 de Plasmodium falciparum présente 59% d'identité et 69% de similarité avec la protéine Hsp90 humaine, et il a été montré que la geldanamycine inhibe la croissance du parasite in vitro (Malaria Journal 2 : 30, 2003 ; J. Biol. Chem. 278 : 18336, 2003 ; J. Biol. Chem. 279: 46692, 2004).
vii) Les filarioses de Brugia et de Bancroft : ces parasites filaires lymphatiques possèdent une protéine Hsp90 pouvant potentiellement être inhibée par des inhibiteurs de la protéine humaine. En effet, il a été montré pour un autre parasite proche, le Brugia pahangi, que ce dernier est sensible à l'inhibition par la geldanamycine. Les séquences de B. pahangi et humaines sont identiques à 80% et similaires à 87%. (Int. J. for Parasitology 35: 627,2005)
viii) La toxoplasmose : Toxoplasma gondii, le parasite responsable de la toxoplasmose, possède une protéine chaperone Hsp90, pour laquelle il a été montré l'induction au cours de la conversion tachyzoite-bradyzoite, correspondant au passage de l'infection chronique vers la toxoplasmose active. De plus, la geldanamycine bloque in vitro cette conversion tachyzoite-bradyzoite (J. Mol. Biol. 350 : 723, 2005)
ix) Les mycoses résistantes aux traitements : Il est possible que la protéine Hsp90 potentialise l'évolution de la résistance aux drogues, en permettant à de nouvelles mutations de se développer. Par conséquent, un inhibiteur de Hsp90, seul ou en combinaison avec un autre traitement antifongique, pourrait se révéler utile dans le traitement de certaines souches résistantes (Science 309 : 2185, 2005). De plus, l'anticorps anti-Hsp90 développé par Neu Tec Pharma démontre une activité contre C. albicans, sensible et résistant au fluconazole, C. krusei, C. tropicalis, C. glabrata, C. lusitaniae et C. parapsilosis in vivo (Current Molecular Medicine 5 : 403, 2005).
x) L'hépatite B : Hsp90 est l'une des protéines de l'hôte qui interagit avec la transcriptase inverse du virus de l'hépatite B au cours du cycle de réplication du virus. Il a été montré que la geldanamycine inhibe la réplication de l'ADN viral et l'encapsulation de l'ARN viral (Proc. Natl. Acad. Sci. USA 93: 1060, 1996)
xi) L'hépatite C : La protéine Hsp90 humaine participe à l'étape de clivage entre les protéines NS2 et NS3 par la protéase virale. La geldanamycine et le radicicol sont capables d'inhiber ce clivage NS2/3 in vitro (Proc. Natl. Acad. Sci. USA 98 : 13931, 2001)
xii) Le virus de l'Herpes : La geldanamycine a démontré des activités d'inhibition de la réplication du virus HSV-1 in vitro, et ce avec un bon index thérapeutique (Antimicrobial Agents and Chemotherapy 48 : 867, 2004). Les auteurs ont également trouvé une activité de la geldanamycine sur les autres virus HSV-2, VSV, Cox B3, HIV-1 et le coronavirus SARS (données non montrées).
xiii) La dengue (ou grippe tropicale) : Il a été montré que la protéine humaine Hsp90 participe à l'étape d'entrée du virus, en formant un complexe contenant également Hsp70 qui sert de récepteur au virus ; Un anticorps anti-Hsp90 diminue le pouvoir infectieux du virus in vitro (J. of Virology 79 : 4557, 2005)
xiv) L'atrophie musculaire spinale et bulbaire : (SBMA : Spinal and bulbar muscular atrophy), une maladie neurodégénérative héréditaire caractérisée par une extension de triplets CAG dans le gène du récepteur aux androgènes. Il a été montré que le 17-AAG, un dérivé de la Geldanamycine, présente une activité in vivo sur des animaux transgéniques servant de modèles expérimentaux de cette maladie (Nature Medicine 11 : 1088, 2005).

### Inhibiteurs d'Hsp90 :

Les premiers inhibiteurs connus d'Hsp90 sont des composés de la famille des amsamycines, en particulier la Geldanamycine (1) et l'Herbimycine A. Des études de rayon X ont montré que la Geldanamycine se lie au site ATP du domaine N-terminal d'Hsp90 où elle inhibe l'activité ATPasique de la chaperone (Prodromou C. et al, Cell (1997), 90, 65-75)

Actuellement le NIH et Kosan Biosciences assurent le développement clinique du 17-AAG (2), qui est un inhibiteur d'Hsp90 dérivé de la geldanamycine (1), qui bloque l'activité ATPasique d'Hsp90, en se liant au site de reconnaissance N-terminal de l'ATP. Les résultats des essais cliniques de phase 1 du 17-AAG (1) conduisent aujourd'hui à initier des essais de phase 11, mais orientent aussi les recherches vers des dérivés plus solubles tel que l'analogue 3 (17-DMAG de Kosan BioSciences), porteur d'une chaîne diméthylaminée à la place du résidu méthoxy, et vers des formulations optimisées du 17AAG (CNF1010 de Conforma Therapeutics) :

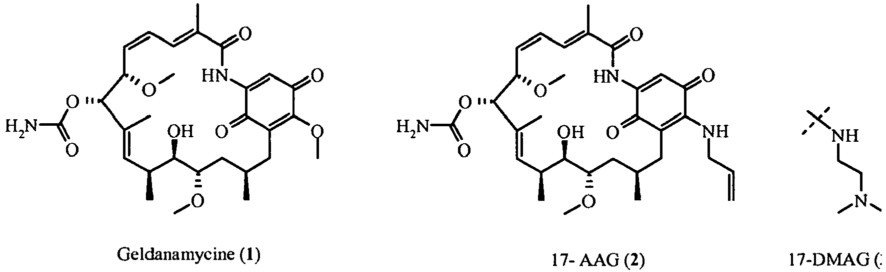

L'analogue réduit du 17-AAG (WO 2005063714 / US 2006019941) est également en études cliniques de phase I depuis peu par la société Infinity Pharmaceuticals. De nouveaux dérivés de geldanamycine ou d'ansamycines ont été décrits récemment (WO2006016773 / US6855705 /US 2005026894/ WO2006050477 / US2006205705 / WO2007001049 /WO2007064926 / WO2007074347 / WO2007098229 / WO2007128827 /W02007128829).

Le Radicicol (4) est également un inhibiteur d'Hsp90 d'origine naturelle (Roe S.M. et al, J. Med Chem. (1999), 42, 260-66). Toutefois, si celui-ci est de loin le meilleur inhibiteur in vitro d'Hsp90, son instabilité métabolique vis à vis des nucléophiles soufrés le rend difficilement utilisable in vivo. Des dérivés oximes bien plus stables tel que le KF 55823 (5) ou le KF 25706 ont été développés par la société Kyowa Hakko Kogyo (Soga et al, Cancer Research (1999), 59, 2931-2938)

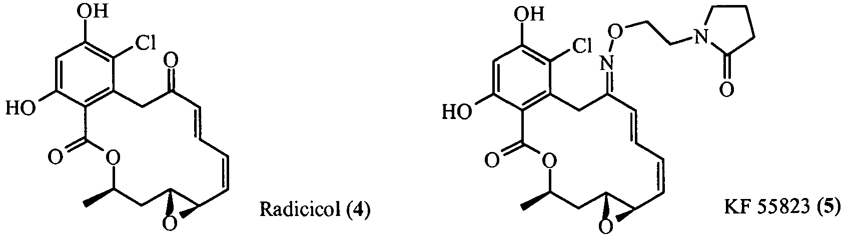

Des structures d'origine naturelle apparentées au radicicol ont également été récemment décrites, comme la zéaralénone (6) par la société Conforma Therapeutics (WO2003041643) ou les composés (7-9).

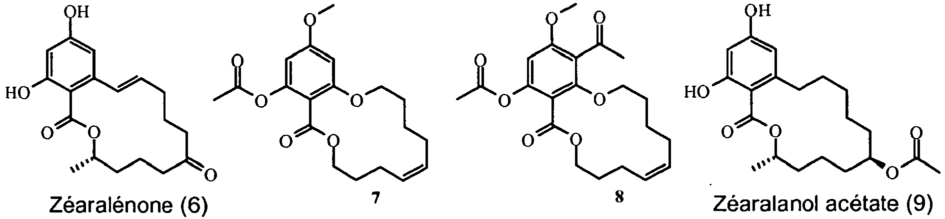

La demande de brevet US2006089495 décrit des composés mixtes comprenant un noyau quinone, comme les dérivés d'amsamycine, et un noyau résorcinol comme les analogues de radicicol, comme inhibiteurs d'Hsp90.

Un inhibiteur d'Hsp90 d'origine naturelle, la novobiocine (10) se lie à un site ATP différent situé dans le domaine C-terminal de la protéine (Itoh H. et al, Biochem J. (1999), 343, 697-703). Récemment des analogues simplifiés de la Novobiocine ont été identifiés comme plus puissants inhibiteurs d'Hsp90 que la Novobiocine elle-même (J. Amer. Chem. Soc(2005), 127(37), 12778-12779). .

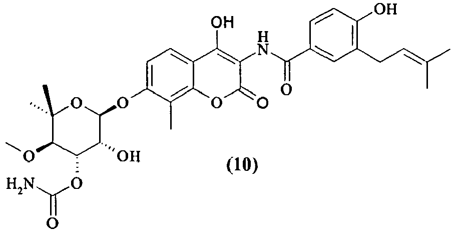

Les demandes de brevet WO2006050501 et US2007270452 revendiquent des analogues de Novobiocine comme inhibiteurs d'Hsp90.

La demande de brevet WO2007117466 revendique des dérivés de Célastrol et de Gédunine comme inhibiteurs d'Hsp90.

Un depsipeptide, nommé Pipalamycin ou ICI101 a également été décrit comme inhibiteur non compétitif du site ATP d'Hsp90 (J. Pharmacol. Exp. Ther. (2004), 310, 1288-1295).

La Sherperdine, nonapeptide KHSSGCAFL, mime une partie de la séquence K79-K90 (KHSSGCAFLSVK) de la Survivine et bloque l'interaction des protéines de la famille IAP avec Hsp90 in vitro (W02006014744).

Des petits peptides, comprenant une séquence de type Otoferline (YSLPGYMVKKLLGA), ont été récemment décrits comme inhibiteurs de Hsp90 (WO2005072766).

Des purines, comme les composés PU3 (11) (Chiosis et al, Chem. Biol. (2001), 8, 289-299) et PU24FCl (12) (Chiosis et al, Curr. Canc. Drug Targets (2003), 3, 371-376 ; WO2002036075) ont également été décrites comme inhibiteurs d'Hsp90 :

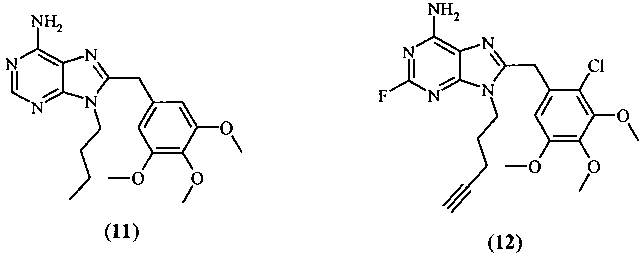

Un dérivé de purine CNF2024 (13) a été récemment introduit en clinique par la société Conforma Therapeutics, en collaboration avec le Sloan Kettering Memorial Institute for Cancer Research (WO2006084030).

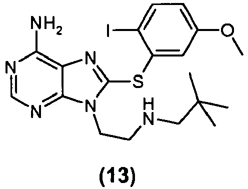

La demande de brevet FR2880540 (Aventis) revendique une autre famille de purines inhibitrices d'Hsp90.

La demande de brevet WO2004072080 (Cellular Genomics) revendique une famille de 8-hétéroaryl-6-phényl-imidazo[1,2-a]pyrazines comme modulateurs de l'activité d'hsp90.

La demande de brevet WO2004028434 (Conforma Therapeutics) revendique des aminopurines, des aminopyrrolopyrimidines, des aminopyrazolopyrimidines et des aminotriazolopyrimidines comme inhibiteurs d'Hsp90.

La demande de brevet WO2004050087 (Ribotarget/Vernalis) revendique une famille de pyrazoles utiles pour traiter des pathologies liées à l'inhibition des « Heat Shock Proteins » tels que la chaperone Hsp90.

La demande de brevet WO2004056782 (Vernalis) revendique une nouvelle famille de pyrazoles utiles pour traiter des pathologies liées à l'inhibition des « Heat Shock Proteins » tels que la chaperone Hsp90.

La demande de brevet WO200407051 (Vernalis) revendique des dérivés d'arylisoxazoles utiles pour traiter des pathologies liées à l'inhibition des « Heat Shock Proteins » tels que la chaperone Hsp90.

La demande de brevet WO2004096212 (Vernalis) revendique une troisième famille de pyrazoles utiles pour traiter des pathologies liées à l'inhibition des « Heat Shock Proteins » tels que la chaperone Hsp90.

La demande de brevet WO200500300 (Vernalis) revendique de manière plus générale des hétérocycles à 5 chaînons, substitués par des radicaux aryles, utiles pour traiter des pathologies liées à l'inhibition des « Heat Shock Proteins » tels que la chaperone Hsp90.

La demande de brevet JP2005225787 (Nippon Kayaku) revendique une autre famille de pyrazoles comme inhibiteurs d'Hsp90.

La demande de brevet WO200500778 (Kyowa Hakko Kogyo) revendique une famille de dérivés de benzophénone comme inhibiteurs d'Hsp90, utiles pour le traitement des tumeurs.

La demande de brevet WO200506322 (Kyowa Hakko Kogyo) revendique une famille de dérivés de résorcinol comme inhibiteurs d'Hsp90.

La demande de brevet WO2005051808 (Kyowa Hakko Kogyo) revendique une famille de dérivés d'acides résorcinyl-benzoïques comme inhibiteurs d'Hsp90.

Les demandes de brevet WO2005021552, WO20050034950, WO200608503 WO2006079789 et WO2006090094 (Vernalis) revendiquent des familles de pyrimidothiophènes ou de pyridothiophènes, utiles pour traiter des pathologies liées à l'inhibition des « Heat Shock Proteins » tels que la chaperone Hsp90.

La demande WO2006018082 (Merck) revendique une autre famille de pyrazoles comme inhibiteurs d'Hsp90.

La demande WO2006010595 (Novartis) revendique une famille d'indazoles comme inhibiteurs d'Hsp90.

La demande WO2006010594 (Novartis) revendique une famille de dihydrobenzimidazolones comme inhibiteurs d'Hsp90.

La demande de brevet WO2006055760 (Synta Pharma) revendique une famille de diaryl-triazoles comme inhibiteurs d'Hsp90.

La demande de brevet WO2006087077 (Merck) revendique une famille de (s-triazol-3-yl)phenols comme inhibiteurs d'Hsp90.

La demande de brevet FR2882361 (Aventis) revendique une famille de 3-aryl-1,2-benzisoxazoles comme inhibiteurs d'Hsp90.

La demande de brevet WO2006091963 (Serenex) revendique des familles de tetrahydroindolones et de tetrahydroindazolone comme inhibiteurs d'Hsp90.

La demande de brevet DE10200509440 (Merck) revendique une famille de thiénopyridines comme inhibiteurs d'Hsp90.

La demande de brevet W02006095783 (Nippon Kayaku) revendique une famille de triazoles comme inhibiteurs d'Hsp90.

La demande de brevet WO2006101052 (Nippon Kayaku) revendique une famille de dérivés acétylèniques comme inhibiteurs d'Hsp90.

La demande de brevet WO2006105372 (Conforma Therapeutics) revendique une famille d'alkynyl pyrrolo[2,3-d]pyrimidines comme inhibiteurs d'Hsp90.

La demande de brevet FR2884252 (Aventis) revendique une famille d'isoindoles comme inhibiteurs d'Hsp90

La demande de brevet WO20061009075 (Astex Therapeutics) revendique une famille de benzamides comme inhibiteurs d'Hsp90.

La demande de brevet WO2006109085 (Astex Therapeutics) revendique une famille d'hydroxybenzamides comme inhibiteurs d'Hsp90.

La demande de brevet WO2006113498 (Chiron) revendique une famille de 2-aminoquinazolin-5-ones comme inhibiteurs d'Hsp90.

La demande de brevet JP200606755 (Nippon Kayaku) revendique une famille de pyrazoles comme inhibiteurs d'Hsp90.

La demande de brevet WO2006117669 (Pfizer) revendique une famille d'hydroxyarylcarboxamides comme inhibiteurs d'Hsp90.

Les demandes de brevet WO2006122631 et DE102006008890 (Merck GmbH) revendiquent une famille d'amino-2-phényl-4-quinazolines comme inhibiteurs d'Hsp90.

La demande de brevet WO2006123061 (Aventis) revendique une famille de dérivés d'azabenzimidazolyl- ou de benzimidazolyl-fluorenes comme inhibiteurs d'Hsp90.

La demande de brevet WO2006123065 (Astex Therapeutics) revendique une famille d'azinamines (amino-2-pyrimidines ou triazines) comme inhibiteurs d'Hsp90.

La demande de brevet WO2006125531 (Merck GmbH) revendique une famille de thiéno[2,3b]pyridines comme inhibiteurs d'Hsp90.

Les demandes de brevet WO2006125813 et WO2006125815 (Altana Pharma) revendique une famille de tétrahydropyridothiophènes comme inhibiteurs d'Hsp90.

La demande de brevet WO2007017069 (Merck GmbH) revendique une famille de dérivés d'adénine comme inhibiteurs d'Hsp90.

Les demandes de brevet WO2007021877 et WO200701966 (Synta Pharma) revendiquent respectivement des familles d'arylpyrazoles et d'arylimidazoles comme inhibiteurs d'Hsp90.

La demande de brevet WO2007022042 (Novartis) revendique une famille de pyrimidylaminobenzamides comme inhibiteurs d'Hsp90.

La demande de brevet WO2007034185 (Vernalis) revendique une famille d'hétéroarylpurines comme inhibiteurs d'Hsp90.

La demande de brevet WO2007041362 (Novartis) revendique une famille de 2-amino-7,8-dihydro-6H-pyrido[4,3-d]pyrimidin-5-ones comme inhibiteurs d'Hsp90.

La demande de brevet WO2007104944 (Vernalis) revendique une famille de pyrrolo[2,3b]pyridines comme inhibiteurs d'Hsp90.

La demande de brevet US2007105862 revendique une famille de dérivés d'azole comme inhibiteurs d'Hsp90.

La demande de brevet WO2007129062 (Astex Therapeutics) revendique une famille de diazoles (aryle pyrazoles) comme inhibiteurs d'Hsp90.

La demande de brevet US2007129334 (Conforma Therapeutics) revendique une famille d'arylthio-purines comme inhibiteurs d'Hsp90, actifs par voie orale.

La demande de brevet WO2007155809 (Synta Pharma) revendique des familles de phényltriazoles comme inhibiteurs d'Hsp90.

La demande de brevet WO2007092496 (Conforma Therapeutics) revendique une famille de 7,9-dihydropurine-8-ones comme inhibiteurs d'Hsp90

La demande de brevet WO2007207984 (Serenex) revendique une famille de dérivés de cyclohexylaminobenzène comme inhibiteurs d'Hsp90.

Les demandes de brevet DE10206023336 et DE10206023337 (Merck GmbH) revendiquent respectivement des familles de 1,5-diphénylpyrazoles et de 1,5-diphényltriazoles comme inhibiteurs d'Hsp90.

La demande de brevet WO2007134298 (Myriad Genetics) revendique une famille de purinamines comme inhibiteurs d'Hsp90.

La demande de brevet WO2007138994 (Chugai) revendique des familles de 2-aminopyrimidines ou de 2-aminotriazines comme inhibiteurs d'Hsp90.

Les demandes de brevet WO2007139951, WO2007139952, WO2007139960, WO2007139967, WO2007139968, WO2007139955 et WO20071400002 (Synta Pharma) revendiquent des familles de triazoles comme inhibiteurs d'Hsp90 et agents pour le traitement des lymphomes non-Hodgkiniens.
La présente invention concerne des dérivés de pyrrolo[1,2-a]indole produits de formule (I) 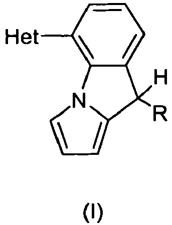 dans lesquels :
Het représente un hétérocycle aromatique ou partiellement insaturé - de type dihydro ou tétrahydro - mono ou bicyclique, de 5 à 11 chaînons, contenant de 1 à 4 hétéroatomes, choisis parmi N, O ou S, éventuellement substitué par un ou plusieurs radicaux R1 ou R'1 identiques ou différents tels que décrits ci-dessous,
R est choisi dans le groupe constitué par X-(A-B)n-CONH2, X-(A-B)n-O-CONH2, X-(A-B)n-NH-CONH2, X-(CH2)m-hétérocycloalkyle, X-(CH2)m-aryle et X-(CH2)m-hétéroaryle avec X représente -O-C(O), -NH-C(O), NH-CS, -NH-CO-CH2-O- ; -NH-CO-CH2-S-CH2-CO-NH-; -NH-CO-(CH2)2-SO2-; -NH-CO-CH2-N(CH3)-CO- ; A et B identiques ou différents représentent indépendamment une simple liaison, CH2, CH-alkyle, CH-aralkyle, n = 1,2 et m=0,1,
R1 et/ou R'1 identiques ou différents sont dans le groupe constitué par H, halogène, CF3, nitro, cyano, alkyle, hydroxy, mercapto, amino, alkylamino, dialkylamino, alkoxy, alkylthio, carboxy libre ou estérifié par un radical alkyle, carboxamide, CO-NH(alkyl), CON(alkyl)2, NH-CO-alkyl, sulfonamide, NH-SO2-alkyl, S(O)2-NHalkyl, S(O2)-N(alkyl)2, tous les radicaux alkyle, alcoxy et alkylthio étant eux-mêmes éventuellement substitués par un ou plusieurs radicaux identiques ou différents choisi(s) parmi halogène, hydroxy, alkoxy, amino, alkylamino et dialkylamino ;
lesdits produits de formule (I) étant sous toutes les formes tautomères et isomères possibles, racémiques, énantiomères et diastéréoisomères, ainsi que comme sels d'addition avec les acides minéraux et organiques ou avec les bases minérales et organiques des produits de formule (i).

Dans les produits de formule (I) et dans ce qui suit, les termes indiqués ont les significations qui suivent :
- le terme halogène désigne les atomes de fluor, de chlore, de brome ou d'iode et de préférence de fluor, chlore ou brome.
- le terme radical alkyle désigne un radical linéaire ou ramifié renfermant au plus 12 atomes de carbone choisi parmi les radicaux méthyle, éthyle, propyle, isopropyle, butyle, isobutyle, sec-butyle, tert-butyle, pentyle, isopentyle, sec-pentyle, tert-pentyle, néo-pentyle, hexyle, isohexyle, sec-hexyle, tert-hexyle et également heptyle, octyle, nonyle, décyle, undécyle et dodécyle, ainsi que leurs isomères de position linéaires ou ramifiés. On cite plus particulièrement les radicaux alkyle ayant au plus 6 atomes de carbone et notamment les radicaux méthyle, éthyle, propyle, isopropyle, n-butyle, isobutyle, terbutyle, pentyle linéaire ou ramifié, hexyle linéaires ou ramifiés.
- le terme radical alcoxy désigne un radical linéaire ou ramifié renfermant au plus 12 atomes de carbone et préférentiellement 6 atomes de carbone choisi par exemple parmi les radicaux méthoxy, éthoxy, propoxy, isopropoxy, butoxy linéaire, secondaire ou tertiaire, pentoxy, hexoxy et heptoxy ainsi que leurs isomères de position linéaires ou ramifiés,
- Le terme alkylthio ou alkyl-S- désigne un radical linéaire ou ramifié renfermant au plus 12 atomes de carbone et représente notamment les radicaux méthylthio, éthylthio, isopropylthio et heptylthio. Dans les radicaux comportant un atome de soufre, l'atome de soufre peut être oxydé en radical SO ou S(O)2
- le terme carboxamide désigne CONH2 .
- le terme sulfonamide désigne SO2NH2
- le terme radical acyle ou r-CO- désigne un radical linéaire ou ramifié renfermant au plus 12 atomes de carbone dans lequel le radical r représente un atome d'hydrogène, un radical alkyle, cycloalkyle, cycloalkényle, hétérocycloalkyle ou aryle, ces radicaux ayant les valeurs indiquées ci-dessus et étant éventuellement substitués comme indiqué : on cite par exemple les radicaux formyle, acétyle, propionyle, butyryle ou benzoyle, ou encore valéryle, hexanoyle, acryloyle, crotonoyle ou carbamoyle
- le terme radical cycloalkyle désigne un radical carbocyclique monocyclique ou bicyclique renfermant de 3 à 10 chaînons et désigne notamment les radicaux cyclopropyle, cyclobutyle, cyclopentyle et cyclohexyle,
- le terme radical cycloalkylalkyle désigne un radical dans lequel cycloalkyle et alkyle sont choisis parmi les valeurs indiquées ci-dessus : ce radical désigne ainsi par exemple les radicaux cyclopropylméthyle, cyclopentylméthyle, cyclohexylméthyle et cycloheptylméthyle.
- par radical acyloxy, on entend les radicaux acyl-O- dans lesquels acyl a la signification indiquée ci-dessus : on cite par exemple les radicaux acétoxy ou propionyloxy.
- par radical acylamino, on entend les radicaux acyl-N- dans lesquels acyl a la signification indiquée ci-dessus.
- le terme radical aryle désigne les radicaux insaturés, monocycliques ou constitués de cycles condensés, carbocycliques. Comme exemples de tel radical aryle, on peut citer les radicaux phényle ou naphtyle.
- Par arylalkyle on entend les radicaux résultant de la combinaison des radicaux alkyle cités précédemment éventuellement substitués et les radicaux aryles également cités ci-dessus, éventuellement substitués : on cite par exemple les radicaux benzyle, phényléthyle, 2-phénéthyle, triphénylméthyle ou naphthlèneméthyle.
- le terme radical hétérocyclique désigne un radical carbocyclique saturé (hétérocycloalkyle) ou partiellement ou totalement insaturé (hétéroaryle) constitué de 4 à 10 chaînons interrompus par un ou plusieurs hétéroatomes, identiques ou différents, choisis parmi les atomes d'oxygène, d'azote ou de soufre.

Comme radicaux hétérocycloalkyles, on peut citer notamment les radicaux dioxolane, dioxane, dithiolane, thiooxolane, thiooxane, oxirannyle, oxolannyle, dioxolannyle, pipérazinyle, pipéridyle, pyrrolidinyle, imidazolidinyle, imidazolidine-2,4-dione, pyrazolidinyle, morpholinyle ou encore tétrahydrofuryle, hexahydropyranne , tétrahydrothiényle, chromanyle, dihydrobenzofuranyle, indolinyle, perhydropyranyle, pyrindolinyle, tétrahydroquinoléinyle, tétrahydroisoquinoléinyle ou thioazolidinyle, tous ces radicaux étant éventuellement substitués.

Parmi les radicaux hétérocycloalkyles, on peut citer notamment les radicaux pipérazinyle éventuellement substitué, N-méthylpipérazinyle, pipéridyle, éventuellement substitués, pyrrolidinyle éventuellement substitué, imidazolidinyle, pyrazolidinyle, morpholinyle, hexahydropyranne ou thiazolidinyle.

Par radical hétérocycloalkylalkyle, on entend les radicaux dans lesquels les restes hétérocycloalkyle et alkyle ont les significations précédentes
parmi les radicaux hétéroaryles à 5 chaînons on peut citer les radicaux furyle, pyrrolyle, tétrazolyle, thiazolyle, isothiazolyle, diazolyle, thiadiazolyle, thiatriazolyle, oxazolyle, oxadiazolyle, isoxazolyle, imidazolyle, pyrazolyle, thiényle, triazolyle,

Parmi les radicaux hétéroaryles à 6 chaînons on peut citer notamment les radicaux pyridyle tel que 2-pyridyle, 3-pyridyle et 4-pyridyle, pyrimidyle, pyridazinyle, pyrazinyle.

Comme radicaux hétéroaryles condensés contenant au moins un hétéroatome choisi parmi le soufre, l'azote et l'oxygène, on peut citer par exemple benzothiényle, benzofuryle, benzopyrrolyle, benzothiazolyle, benzimidazolyle, imidazopyridyle, imidazopyrimidinyle, imidazopyrazinyle, purinyle, pyrrolopyrimidinyle, pyrrolopyridinyle, benzoxazolyle, benzisoxazolyle, benzisothiazolyle, thionaphtyle, chroményle, indolizinyle, quinazolinyle, quinoxalinyle, indolyle, indazolyle, purinyle, quinolyle, isoquinolyle et naphtyridinyle.

Par radical alkylamino, on entend les radicaux dans lesquels le radical alkyl est choisi parmi les radicaux alkyles cités ci-dessus. On préfère les radicaux alkyle ayant au plus 4 atomes de carbone et on peut citer par exemple les radicaux méthylamino, éthylamino, propylamino ou butylamino, linéaire ou ramifié.

Par radical dialkylamino, on entend les radicaux dans lesquels les radicaux alkyle identiques ou différents sont choisis parmi les radicaux alkyle cités ci-dessus. Comme précédemment on préfère les radicaux alkyle ayant au plus 4 atomes de carbone et on peut citer par exemple les radicaux diméthylamino, diéthylamino, méthyléthylamino linéaire ou ramifié.

Le terme patient désigne les êtres humains mais aussi les autres mammifères.

Le terme "Prodrogue" désigne un produit qui peut être transformé in vivo par des mécanismes métaboliques (tel que l'hydrolyse) en un produit de formule (I). Par exemple, un ester d'un produit de formule (I) contenant un groupe hydroxyle peut être converti par hydrolyse in vivo en sa molécule mère. Ou encore un ester d'un produit de formule (I) contenant un groupe carboxy peut être converti par hydrolyse in vivo en sa molécule mère.

On peut citer à titre d'exemples des esters de produits de formule (I) contenant un groupe hydroxyle tels que les acétates, citrates, lactates, tartrates, malonates, oxalates, salicylates, propionates, succinates, fumarates, maléates, méthylene-bis-b-hydroxynaphthoates, gentisates, iséthionates, di-p-toluoyltartrates, méthanesulfonates, éthanesulfonates, benzenesulfonates, p-toluènesulfonates, camphorsulfonates, cyclohexylsulfamates et quinates.

Des esters de produits de formule (I) particulièrement utiles contenant un groupe hydroxyle peuvent être préparés à partir de restes acides tels que ceux décrits par Bundgaard et. al., J. Med. Chem., 1989, 32, page 2503-2507: ces esters incluent notamment des (aminométhyl)-benzoates substitués, dialkylamino-méthylbenzoates dans lesquels les deux groupements alkyle peuvent être liés ensemble ou peuvent être interrompus par un atome d'oxygène ou par un atome d'azote éventuellement substitué soit un atome d'azote alkylé ou encore des morpholino-méthyl)benzoates, e.g. 3- ou 4-(morpholinométhyl)-benzoates, et (4-alkylpiperazin-1-yl)benzoates, e.g. 3- ou 4-(4-alkylpiperazin-1-yl)benzoates.

Le ou les radicaux carboxy des produits de formule (I) peuvent être salifiés ou estérifiés.

On peut rappeler que la stéréoisomérie peut être définie dans son sens large comme l'isomérie de composés ayant mêmes formules développées, mais dont les différents groupes sont disposés différemment dans l'espace, tels que notamment dans des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomère est utilisé dans la présente demande dans son sens le plus large et concerne donc l'ensemble des composés indiqués ci-dessus.

La présente invention concerne ainsi notamment les produits de formule (I) tels que définis ci-dessus dans lesquels Het représente un hétérocycle tel que défini ci-dessus renfermant notamment au moins un atome d'azote et éventuellement substitué par un ou plusieurs radicaux R1 ou R'1 identiques ou différents tels que définis ci-dessus, et R ayant l'une quelconque des définitions indiquées ci-dessus ou ci-après
lesdits produits de formule (I) étant sous toutes les formes tautomères et isomères possibles, racémiques, énantiomères et diastéréoisomères, ainsi que comme sels d'addition avec les acides minéraux et organiques ou avec les bases minérales et organiques des produits de formule (I).

La présente invention concerne ainsi notamment les produits de formule (I) tels que définis ci-dessus dans lesquels :
Het est choisi dans le groupe constitué par : 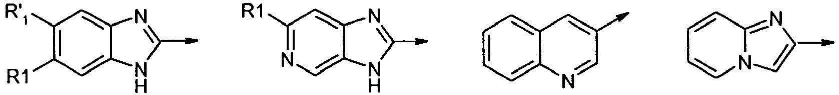 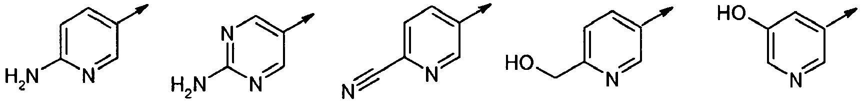 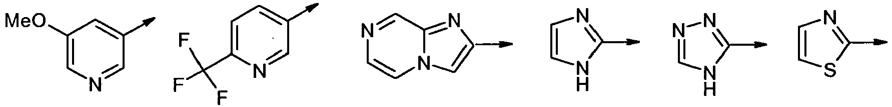
R1 et/ou R'1 identiques ou différents sont dans le groupe constitué par H, halogène, CF3, nitro, cyano, alkyle, hydroxy, mercapto, amino, alkylamino, dialkylamino, alkoxy, alkylthio (méthylthio), carboxy libre ou estérifié par un radical alkyle, carboxamide, CO-NH(alkyl) et CON(alkyl)2, NH-CO-alkyl, sulfonamide, NH-SO2-alkyl, S(O)2-NH(alkyl), S(O)2-N(alkyl)2, tous les radicaux alkyle, alcoxy et alkylthio étant eux-mêmes éventuellement substitués par un ou plusieurs radicaux identiques ou différents choisi(s) parmi halogène, hydroxy, alkoxy, amino, alkylamino et dialkylamino ;
le substituant R desdits produits de formule (I) étant choisi parmi les valeurs définies ci-dessus ou ci-après,
lesdits produits de formule (I) étant sous toutes les formes tautomères et isomères possibles, racémiques, énantiomères et diastéréoisomères, ainsi que comme sels d'addition avec les acides minéraux et organiques ou avec les bases minérales et organiques des produits de formule (I).

La présente invention concerne ainsi notamment les produits de formule (I) tels que définis ci-dessus dans lesquels Het représente un hétérocycle tel que défini ci-dessus éventuellement substitué par un ou plusieurs radicaux R1 ou R'1 identiques ou différents tels que définis ci-dessus, et R représente notamment le groupement X-(CH2)m-hétéroaryle tel que défini ci-dessus ou ci-après avec X représente notamment -NH-CO- et m représente notamment 0. lesdits produits de formule (I) étant sous toutes les formes tautomères et isomères possibles, racémiques, énantiomères et diastéréoisomères, ainsi que comme sels d'addition avec les acides minéraux et organiques ou avec les bases minérales et organiques des produits de formule (I).

La présente invention concerne ainsi notamment les produits de formule (I) tels que définis ci-dessus dans lesquels Het représente un hétérocycle tel que défini ci-dessus éventuellement substitué par un ou plusieurs radicaux R1 ou R'1 identiques ou différents tels que définis ci-dessus, et R est choisi dans le groupe constitué par : 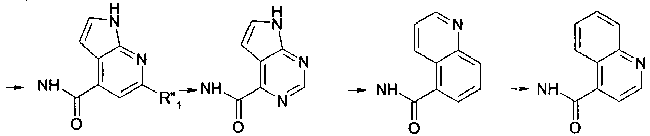 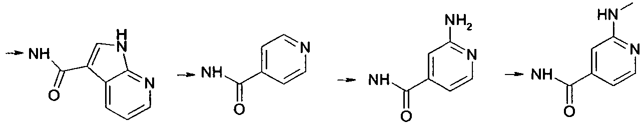 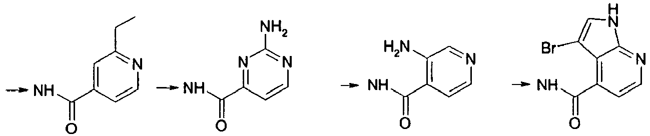 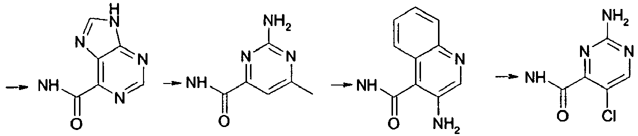 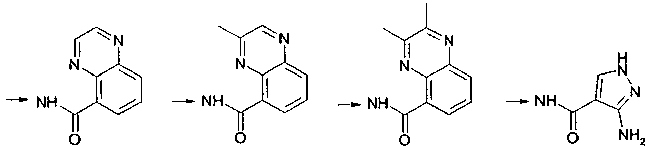 lesdits produits de formule (I) étant sous toutes les formes tautomères et isomères possibles, racémiques, énantiomères et diastéréoisomères, ainsi que comme sels d'addition avec les acides minéraux et organiques ou avec les bases minérales et organiques des produits de formule (I).

La présente invention concerne ainsi notamment les produits de formule (I) tels que définis ci-dessus ou ci-après dans lesquels :
Het est choisi dans le groupe constitué par : 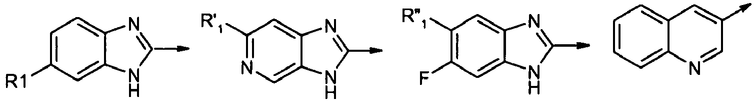 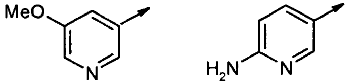
R est choisi dans le groupe constitué par : 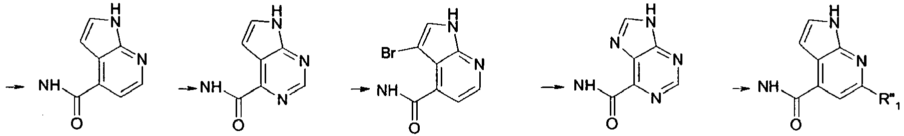
R1 est dans le groupe constitué par H, F, Cl, Br, CF3, NO2, CN, CH3, OH, OCH3, OCF3, CO2Me, CONH2, CONHMe, CONH-(CH2)3-OMe, CONH-(CH2)3-N(Me)2, NHC(O)Me, SO2NH2, SO2N(Me)2 ;
R'1 est dans le groupe constitué par H, CONH2, CONHMe et OMe ;
R"1 est dans le groupe constitué par F, Cl, OH, OMe, CN, O-(CH2)3-OMe et O-(CH2)3-N(Me)2 ;
lesdits produits de formule (I) étant sous toutes les formes tautomères et isomères possibles, racémiques, énantiomères et diastéréoisomères, ainsi que comme sels d'addition avec les acides minéraux et organiques ou avec les bases minérales et organiques des produits de formule (I).

La présente invention concerne ainsi notamment les produits de formule (I) tels que définis ci-dessus ou ci-après dans lesquels :
Het est choisi dans le groupe constitué par : 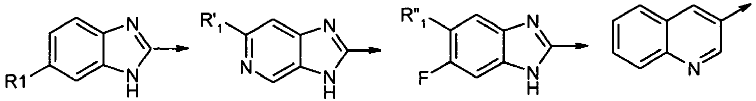 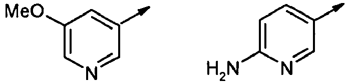
R est choisi dans le groupe constitué par : 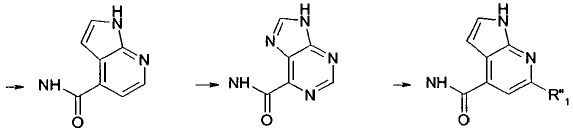
R1 est dans le groupe constitué par H, F, Cl, Br, CF3, NO2, CN, CH3, OH, OCH3, OCF3, CO2Me, CONH2, CONHMe, CONH-(CH2)3-OMe, CONH-(CH2)3-N(Me)2, NHC(O)Me, SO2NH2, SO2N(Me)2,
R'1 est dans le groupe constitué par H, CONH2, CONHMe et OMe,
R"1 est dans le groupe constitué par F, Cl, OH, OMe, CN, O-(CH2)3-OMe et O-(CH2)3-N(Me)2
lesdits produits de formule (I) étant sous toutes les formes isomères possibles racémiques, énantiomères et diastéréo-isomères, ainsi que les sels d'addition avec les acides minéraux et organiques ou avec les bases minérales et organiques.

L'invention a particulièrement pour objet les produits de formule (I) tels que définis ci-dessus dans lesquels :
Het est choisi dans le groupe constitué par : 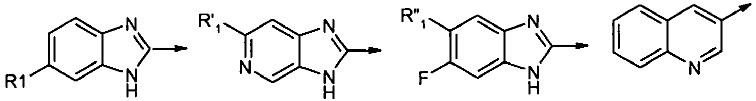 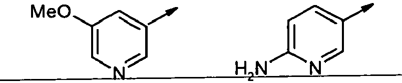 avec :
   R1 représente H, F, Cl, Br, CF3, NO2, CN, CH3, OH, OCH3, OCF3, CO2Me, CONH2, CONHMe, CONH-(CH2)3-OMe, CONH-(CH2)3-N(Me)2, NHC(O)Me, SO2NH2, SO2N(Me)2;
   R'1 représente H, CONH2, CONHMe et OMe;
   R"1 représente F, Cl, OH, OMe, CN, O-(CH2)3-OMe et O-(CH2)3-N(Me)2 ;
et R est choisi dans le groupe constitué par : 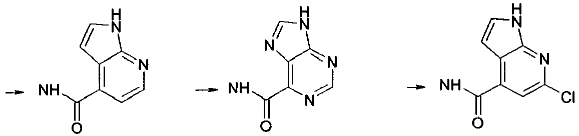 ainsi que leurs prodrogues, lesdits produits de formule (I) étant sous toutes les formes isomères possibles tautomères, racémiques, énantiomères et diastéréo-isomères, ainsi que les sels d'addition avec les acides minéraux et organiques ou avec les bases minérales et organiques desdits produits de formule (I).

La présente invention a plus particulièrement pour objet les produits de formule (I) tels que définis ci-dessus dont les noms suivent :
- le [5-(6-fluoro-1H-benzimidazol-2-yl)-9H-pyrrolo[1,2-a]indol-9-yl]amide de l'acide 1H-pyrrolo[2,3-b]pyridine-4-carboxylique.
- le (5-quinoléin-3-yl-9H-pyrrolo[1,2-a]indol-9-yl)-amide de l'acide 1H-pyrrolo[2,3-b]pyridine-4-carboxylique.
- le [5-(3H-imidazo[4,5-c]pyridin-2-yl)-9H-pyrrolo[1,2-a]indol-9-yl]-amide de l'acide [1H-pyrrolo[2,3-b]pyridine-4-carboxylique.
- le [5-(3H-imidazo[4,5-c]pyridin-2-yl)-9H-pyrrolo[1,2-a]indol-9-yl]-amide de l'acide 6-chloro-1H-pyrrolo[2,3-b]pyridine-4-carboxylique.
ainsi que les sels d'addition avec les acides minéraux et organiques ou avec les bases minérales et organiques desdits produits de formule (I).

Les produits de formule (I) selon la présente invention peuvent être préparés selon les méthodes connues de l'homme du métier et particulièrement selon les méthodes décrites ci-après: la présente invention a ainsi également pour objet les méthodes de synthèse des produits de formule (I) selon la présente invention et notamment les méthodes générales de synthèses décrites dans les schémas ci-après.

### Méthodes de synthèses générales des composés de formule générale (I) :

Les produits de formule générale (I) peuvent être préparés à partir d'un dérivé de formule générale (II), soit par l'intermédiaire d'un dérivé de formule générale (III) (méthode A) soit par l'intermédiaire d'un dérivé de formule générale (IV) (méthode B) selon le schéma général (1) ci-dessous :

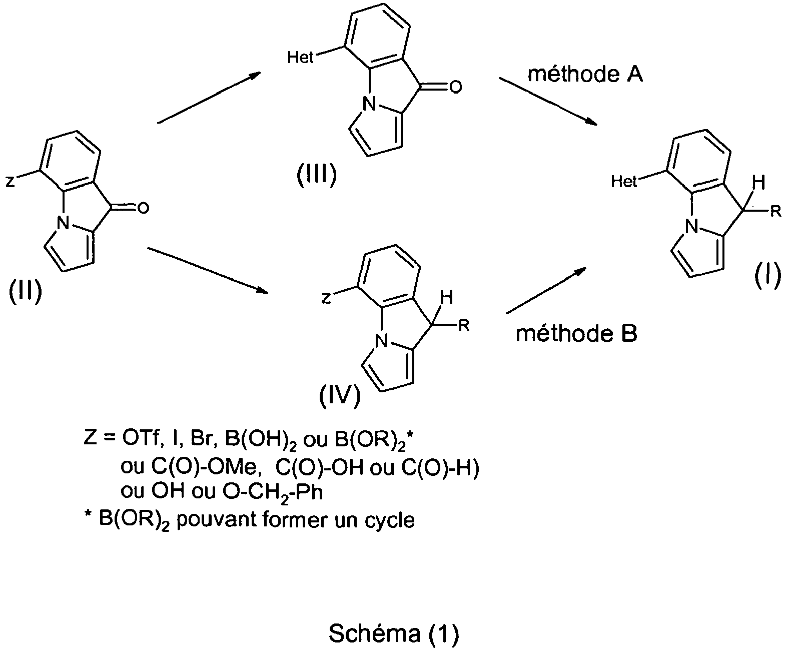

La présente invention a ainsi notamment pour objet un procédé de préparation des produits de formule (I) tels que définis ci-dessus, caractérisé par le schéma (I) ci-dessus, selon lequel les produits de formule générale (I) peuvent être préparés à partir d'un dérivé de formule générale (II), soit par l'intermédiaire d'un dérivé de formule générale (III) (méthode A) soit par l'intermédiaire d'un dérivé de formule générale (IV) (méthode B) et dans lequel les substituants Het et R ont les significations indiquées ci-dessus pour les produits de formule (I) et z a la signification indiquée ci-dessus dans le schéma (1),.

La présente invention a également pour objet les procédés de préparation des produits de formule (I), et d'intermédiaires permettant d'obtenir des produits de formule (I), selon les schémas 2 à 12 ci-après.

### Préparation des composés de formule générale (II)

La présente invention a ainsi également pour objet les méthodes de synthèse des produits de formule (II), dans lesquels Z représente le brome, le radical triflate, un acide boronique ou un boronate, éventuellement cyclique, un radical carboxy, un radical carboxylate de méthyle, le radical hydroxy, ou le radical benzyloxy.

Le produit de formule générale (II) dans lequel Z représente l'atome d'iode peut être obtenu selon J. Med. Chem. 2004, 47(6), 1448.

Le produit de formule générale (II) dans lequel Z représente l'atome de brome peut être obtenu en remplaçant l'atome d'iode par l'atome de brome dans le procédé précédent décrit dans J. Med. Chem. 2004, 47(6), 1448.

Le produit de formule générale (II) dans lequel Z représente le radical benzyloxy peut être obtenu selon les méthodes générales connues de l'homme de l'art et en particulier en adaptant celles décrites dans J.Org.Chem. 1967, 32(2), 486.

Le produit de formule générale (II) dans lequel Z représente le radical hydroxyle peut-être obtenu par débenzylation selon les méthodes générales connues de l'homme de l'art du produit de formule générale (II) dans lequel z représente le radical benzyloxy.

Le produit de formule générale (II) dans lequel Z représente le radical trifluorométhanesulfonyloxy (aussi appelé « triflate » dans la suite de l'invention) peut être obtenu par action d'un agent de trifluorométhylsulfonation, tel que le N-phényl-bis(trifluorométhane-sulfonimide), dans un solvant organique comme le dichlorométhane, en présence d'une base organique comme la triéthylamine, selon le schéma (2) ci-dessous.

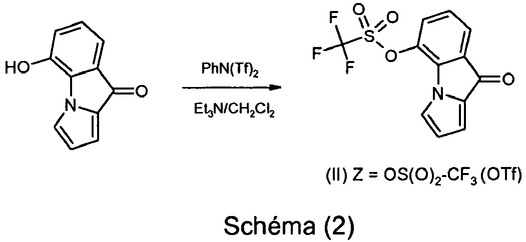

Le produit de formule générale (II) dans lequel Z représente un radical carboxylate de méthyle peut être obtenu soit
- par une réaction de carbonylation dans le méthanol, catalysée par un complexe de palladium tel que l'acétate de palladium en présence de ligand de type phosphine tel que 1,3-diphénylphosphinopropane,
- soit par la réaction de cyclisation avec BBr3 dans le dichlorométhane à partir de l'ester diméthylique de l'acide 2(1-pyrrolyl)isophthalique décrit dans Helv.Chim.Acta 1983, 66(7), 2135,
selon le schéma (3) ci-dessous :

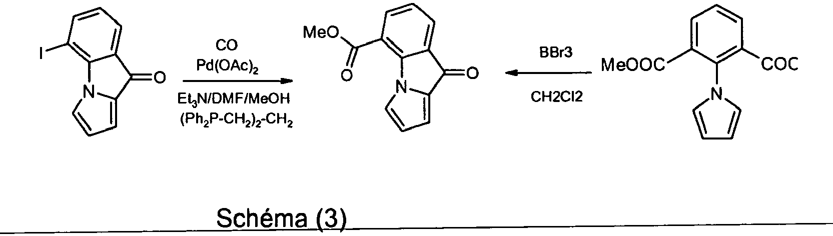

Le produit de formule générale (II) dans lequel Z représente un radical carboxy peut être obtenu par hydrolyse selon les méthodes générales connues de l'homme de l'art du composé de formule générale (II) dans lequel z représente un radical carboxylate de méthyle.

Le produit de formule générale (II) dans lequel Z représente un radical formyle peut être obtenu en opérant selon selon les méthodes générales connues de l'homme de l'art à partir du produit de formule générale (II) dans lequel z représente le radical carboxylate de méthyle.

Le produit de formule générale (II) dans lequel z représente un radical chlorocarbonyle peut être obtenu par choruration du composé de formule générale (II) dans lequel z représente un radical carboxy selon les méthodes générales connues de l'homme de l'art.

Les produits de formule générale (II) dans lesquels Z représente un acide boronique ou un ester boronique, éventuellement cyclique, peuvent être avantageusement préparés par action d'une base lithiée puis d'un borate, tel que le borate de triméthyle, de tri-n.butyle ou de triisopropyle ou le pinacolyle diboronate, sur la 5-bromo-pyrrolo[1,2-a]indol-9-one à basse température dans un solvant organique tel que le tétrahydrofuranne, ou bien encore à partir de la 5-iodo- ou 5-trifluorométhylsulfonyloxy-pyrrolo[1,2-a]indol-9-one, en présence d'un catalyseur au palladium(0), selon le schéma (4).

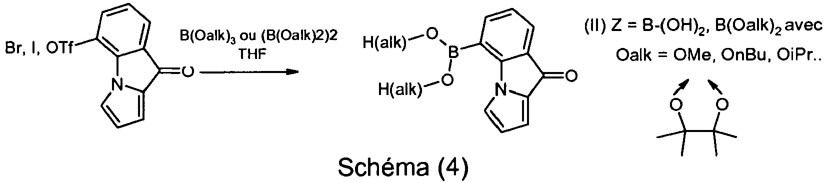

### Préparation des composés de formule générale (III)

La présente invention a ainsi également pour objet les méthodes de synthèse des produits de formule (III), dans lesquels, R1 et/ou R'1 étant tels que définis précédemment, Het est dans le groupe constitué par : 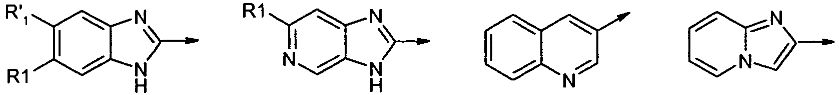 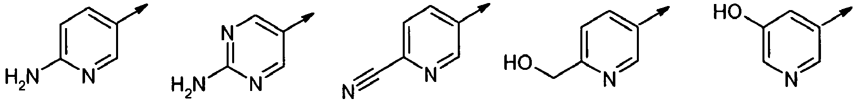 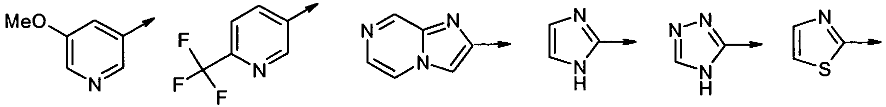 Plus particulièrement, lorsque Het ne représente pas un hétérocyle de type imidazol-2-yle, triazol-3-yle, benzimidazol-2-yle ou azabenzimidazol-2-yle, et est éventuellement substitué par un ou plusieurs radicaux R1, tels que définis précédemment, il est particulièrement avantageux selon l'invention de préparer les composés de formule générale (III)
- soit par couplage d'un composé de formule générale (II) dans lequel z représente un atome d'iode, un atome de brome ou le radical trifluorométhanesulfonyloxy avec un dérivé hétérocyclique boronique, acide ou ester,
- soit par couplage d'un composé de formule générale (II) dans lequel z représente un acide boronique ou un ester boronique, éventuellement cyclique - tel que l'ester de méthyle, de n-butyle, d'isopropyle ou de pinacole avec un bromo ou un iodo hétérocycle,
dans les conditions de la réaction de Suzuki, en présence d'un dérivé de Palladium(0) comme catalyseur, en opérant selon le schéma (5) :

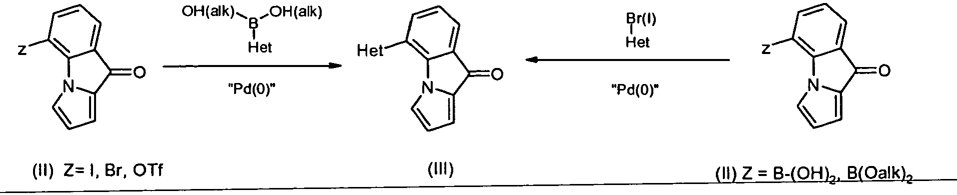

Plus particulièrement, lorsque l'hétérocycle Het est de type benzimidazole ou azabenzimidazole - ou bien encore de type benzoxazole ou azabenzoxazole, benzothiazole ou azabenzothiazole, lié par sa position 2 à la position 5 de la pyrrolo[1,2-a]indol-9-one, il est particulièrement avantageux de former ledit hétérocycle par couplage d'un dérivé d'orthophénylènediamine ou de diamino-pyridine - ou bien d'orthoaminophénol, d'orthoaminothiophénol ou d'amino-hydroxy-pyridine ou d'amino-mercapto-pyridine ortho-disubstituée - avec un acide, un chlorure d'acide, un ester de méthyle, ou un aldéhyde en position 5 de la pyrrolo[1,2-a]indol-9-one en opérant selon le schéma (6) : 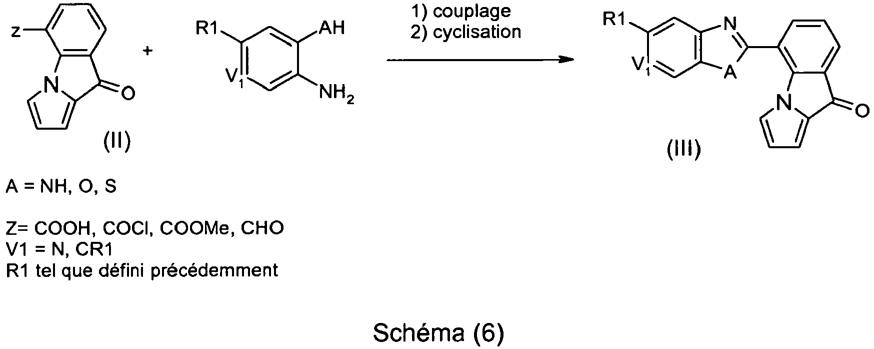 Lorsqu'on utilise le dérivé acide 5-carboxylique de la pyrrolo[1,2-a]indol-9-one, il est particulièrement avantageux d'activer cet acide à l'aide d'un agent de couplage connu de l'homme de l'art, tel que le chlorhydrate de 1-(3-diméthylaminopropyl)-3-éthylcarbodiimide (EDCI) en présence de 1-hydroxybenzotriazole (HOBT), du tétrafluoroborate de o-((éthoxy-carbonyl)cyanométhylèneamino)-N,N,N',N'-tetraméthyluronium (TOTU) ou de l'hexafluorophosphate de o-(1H-benzotriazole-1-yl)-N,N,N',N'-tetraméthyluronium (HBTU).
Lorsqu'on utilise un dérivé ester de méthyle de l'acide 5-carboxylique de la pyrrolo[1,2-a]indol-9-one, il est avantageux dans le cadre de l'invention, d'opérer en présence de triméthylaluminium dans un solvant organique halogéne, tel que le dichlorométhane ou le dichlorométhane.
Lorsqu'on utilise un dérivé 5-carboxaldéhyde de la pyrrolo[1,2-a]indol-9-one, il est avantageux, dans le cadre de l'invention, d'opérer :
- soit par chauffage micro-onde en présence de silice, selon Tetrahedron Lett. 1998, 39, 4481-84 ;
- soit en présence de dichloro-dicyano-benzoquinone (DDQ), selon Tetrahedron 1995, 51, 5813-18 ;
- soit en présence d'un mélange de chlorure de thionyle et de pyridine, selon E.P. 511187 ;
- soit en présence de chlorure ferrique, selon Eur. J. Med. Chem. 2006, 31, 635-42.
Diverses conditions de cyclisation du mélange d'amides intermédiaires peuvent être utilisées dans le cadre de l'invention, tels que l'acide acétique ou un mélange d'acide et d'anhydride trifluoroacétique. Il est également particulièrement avantageux, dans le cadre de l'invention, d'effectuer ce type de cyclisation thermique en milieu acide par chauffage dans un réacteur micro-onde.

Plus particulièrement, lorsque ledit hétérocycle est de type imidazole, oxazole, ou thiazole, lié par sa position 2 à la position 5 de la pyrrolo[1,2-a]indol-9-one, il est particulièrement avantageux de former ledit hétérocycle à partir d'un acide, d'un chlorure d'acide, d'un ester ou d'un aldéhyde en position 5 de la pyrrolo[1,2-a]indol-9-one, en opérant selon le schéma (7) : 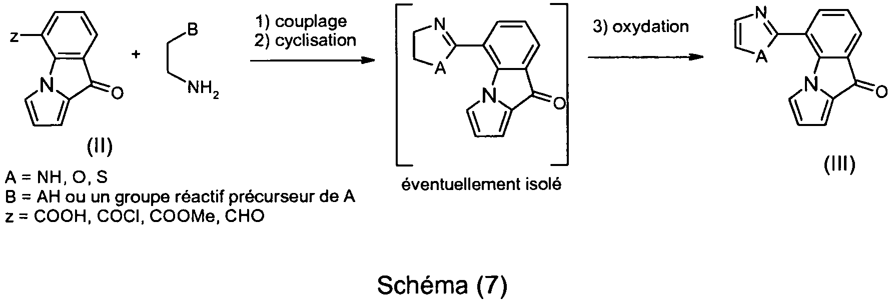 Dans le cadre de invention il est particulièrement avantageux d'opérer :
1. dans le cas où ledit hétérocycle est un imidazole ou une imidazoline :
   - à partir d'une 2-azido-éthylamine, selon Tetrahedron, 47(38), 1991, 8177-94,
   - d'une éthylènediamine, selon Biorg. Med. Chem Lett. 12(3), 2002, 471-75,
   - de glyoxal et d'ammoniaque, selon J. Med. Chem., 46(25), 2003, 5416-27 ;
2. dans le cas où ledit hétérocycle est un oxazole ou une oxazoline :
   - à partir d'un 2-azido-éthanol, selon J. Org. Chem., 61 (7), 1996, 2487-96,
   - d'un 2-aminoéthanol, selon J. Med. Chem. 47(8), 2004, 1969-86 ou Khim. Geterosikl. Soed. 1984(7), 881-4,
   - de diéthylacétal de 2-aminoacétaldéhyde, selon Heterocycles, 39(2), 1994, 767-78 ;
3. dans le cas où ledit hétérocycle est thiazole ou une thiazoline :
   - à partir d'une 2-chloro-éthylamine et de réactif de Lawesson, selon Helv. Chim. Acta, 88(2), 2005, 187-95,
   - d'un 2-aminoéthanethiol, selon J. Org. Chem. 69(3), 2004, 811-4, ou Tetrahedron Lett., 41(18), 2000, 3381-4,

D'une manière plus générale, il est avantageux, dans le cadre de l'invention, de former l'hétérocycle d'un produit de formule générale (III) à partir d'un triflate, d'un dérivé bromé ou iodé, d'un acide ou d'un ester boronique, d'un acide carboxylique, d'un chlorure d'acide, d'un ester d'acide carboxylique ou d'un aldéhyde, en position 5 de la pyrrolo[1,2-a]indol-9-one par l'une quelconque des méthodes de synthèse connues de l'homme de l'art, telles que celles décrites dans Comprehensive Organic Chemistry, par D. H.R. Barton et al. (Pergamon Press) ou Advances in Heterocyclic Chemistry (Academic Press) ou Heterocyclic Compounds (Wiley Intersciences).

### Préparation des composés de formule générale (IV)

La présente invention a également pour objet les méthodes de synthèse des produits de formule (IV), dans lesquels Z représente un groupe ester carboxylique, notamment ester de méthyle, un radical benzyloxy, un atome d'iode, un atome de brome, un radical triflate, un acide ou un ester boronique, un acide carboxylique, un carboxaldéhyde ou un radical hydroxyle.
Les produits de formule générale (IV) dans lesquels Z représente un ester carboxylique, un radical benzyloxy, un atome d'iode, un atome de brome, un radical triflate, un acide ou un ester boronique, un acide carboxylique, un carboxaldéhyde ou un radical hydroxyle peuvent être avantageusement préparés dans le cadre de l'invention en faisant réagir un produit de formule générale (II), dans lesquel Z représente un ester carboxylique, un radical benzyloxy, un atome d'iode ou un atome de brome selon les méthodes générales connues de l'homme de l'art, en particulier celles décrites dans Comprehensive Organic Chemistry, par D.Barton et al. (Pergamon Press), Advanced Organic Chemistry, par J. March (Wiley Interscience), ou Compendium of Organic Synthetic Methods (Wiley Interscience). Il est particulièrement avantageux d'utiliser la méthode décrite dans le schéma (8)

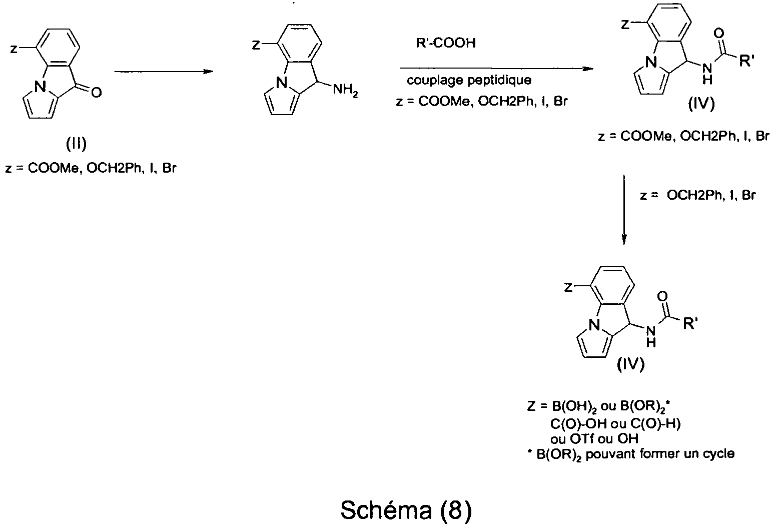

### Préparation des composés de formule générale (I)

### Méthode A à partir des composés (III) :

La présente invention a également pour objet les méthodes de synthèse des produits de formule (I), à partir des composés de formule générale (III) qui peut être effectuée selon les méthodes générales connues de l'homme de l'art, en particulier celles décrites dans Comprehensive Organic Chemistry, par D.Barton et al. (Pergamon Press), Advanced Organic Chemistry, par J.March (Wiley Interscience), ou Compendium of Organic Synthetic Methods (Wiley Interscience). Il est particulièrement avantageux d'utiliser la méthode du schéma (9) :

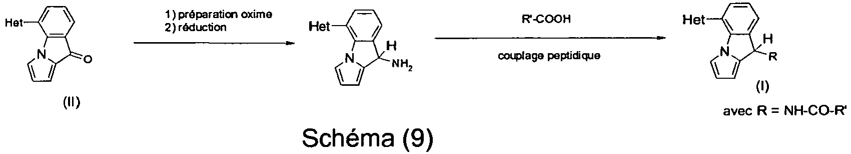

### Méthode B à partir des composés (IV) :

La présente invention a également pour objet les méthodes de synthèse des produits de formule (I), à partir des composés de formule générale (IV) qui peut être effectuée selon les méthodes générales connues de l'homme de l'art, en particulier celles décrites dans Comprehensive Organic Chemistry, par D.Barton et al. (Pergamon Press), Advanced Organic Chemistry, par J.March (Wiley Interscience), ou Compendium of Organic Synthetic Methods (Wiley Interscience).
Lorsque Het ne représente pas un hétérocyle de type imidazol-2-yle, triazol-3-yle, benzimidazol-2-yle ou azabenzimidazol-2-yle, et est éventuellement substitué par un ou plusieurs radicaux R1, tels que définis précédemment, il est particulièrement avantageux selon l'invention de préparer les composés de formule générale (I)
- soit par couplage d'un composé de formule générale (IV) dans lequel z représente un atome d'iode, un atome de brome ou le radical trifluorométhanesulfonyloxy avec un dérivé hétérocyclique boronique, acide ou ester,
- soit par couplage d'un composé de formule générale (IV) dans lequel z représente un acide boronique ou un ester boronique, éventuellement cyclique - tel que l'ester de méthyle, de n-butyle, d'isopropyle ou de pinacole avec un bromo ou un iodo hétérocycle,
dans les conditions de la réaction de Suzuki, en présence d'un dérivé de Palladium(0) comme catalyseur, en opérant selon le schéma (10) :

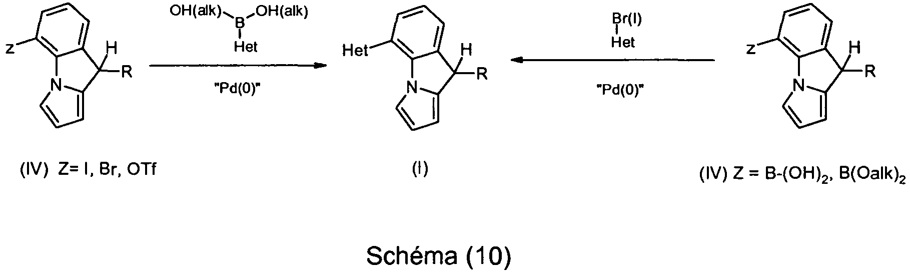

Plus particulièrement, lorsque l'hétérocycle Het est de type benzimidazole ou azabenzimidazole - ou bien encore de type benzoxazole ou azabenzoxazole, benzothiazole ou azabenzothiazole, lié par sa position 2 à la position 5 du pyrrolo[1,2-a]indole, il est particulièrement avantageux de former ledit hétérocycle par couplage d'un dérivé d'orthophénylènediamine ou de diamino-pyridine - ou bien d'orthoaminophénol, d'orthoaminothiophénol ou d'amino-hydroxy-pyridine ou d'amino-mercapto-pyridine ortho-disubstituée - avec un acide, un chlorure d'acide, un ester de méthyle, ou un carboxaldéhyde en position 5 du pyrrolo[1,2-a]indole en opérant selon le schéma (11) : 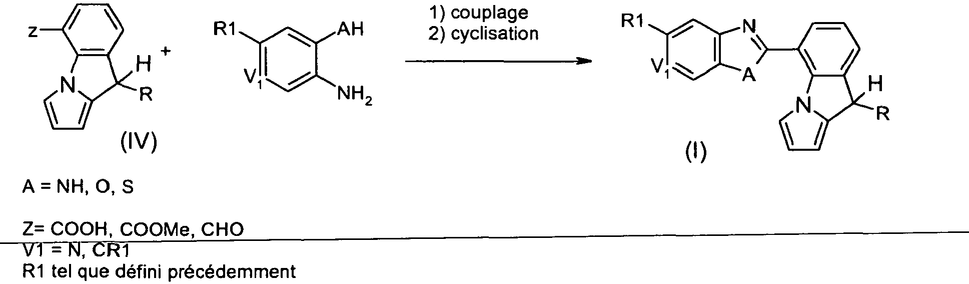 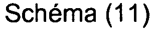 Lorsqu'on utilise le dérivé acide 5-carboxylique du pyrrolo[1,2-a]indole, il est particulièrement avantageux d'activer cet acide à l'aide d'un agent de couplage connu de l'homme de l'art, tel que le chlorhydrate de 1-(3-diméthylaminopropyl)-3-éthylcarbodiimide (EDCI) en présence de 1-hydroxybenzotriazole (HOBT), du tétrafluoroborate de o-((éthoxy-carbonyl)cyanométhylèneamino)-N,N,N',N'-tetraméthyluronium (TOTU) ou de l'hexafluorophosphate de o-(1H-benzotriazole-1-yl)-N,N,N',N'-tetraméthyluronium (HBTU).
Lorsqu'on utilise un dérivé ester de méthyle de l'acide 5-carboxylique du pyrrolo[1,2-a]indole, il est avantageux dans le cadre de l'invention, d'opérer en présence de triméthylaluminium dans un solvant organique halogéné, tel que le dichlorométhane ou le dichloroéthane.
Lorsqu'on utilise un dérivé 5-carboxaldéhyde du pyrrolo[1,2-a]indole, il est avantageux, dans le cadre de l'invention, d'opérer :
- soit par chauffage micro-onde en présence de silice, selon Tetrahedron Lett. 1998, 39, 4481-84 ;
- soit en présence de dichloro-dicyano-benzoquinone (DDQ), selon Tetrahedron 1995, 51, 5813-18 ;
- soit en présence d'un mélange de chlorure de thionyle et de pyridine, selon E.P. 511187 ;
- soit en présence de chlorure ferrique, selon Eur. J. Med. Chem. 2006, 31, 635-42.
Diverses conditions de cyclisation du mélange d'amides intermédiaires peuvent être utilisées dans le cadre de l'invention, tels que l'acide acétique ou un mélange d'acide et d'anhydride trifluoroacétique. Il est également particulièrement avantageux, dans le cadre de l'invention, d'effectuer ce type de cyclisation thermique en milieu acide par chauffage dans un réacteur micro-onde.

Plus particulièrement, lorsque ledit hétérocycle est de type imidazole, oxazole, ou thiazole, lié par sa position 2 à la position 5 du pyrrolo[1,2-a]indole, il est particulièrement avantageux de former ledit hétérocycle à partir d'un acide, d'un ester ou d'un aldéhyde en position 5 du pyrrolo[1,2-a]indole, en opérant selon le schéma (12) : 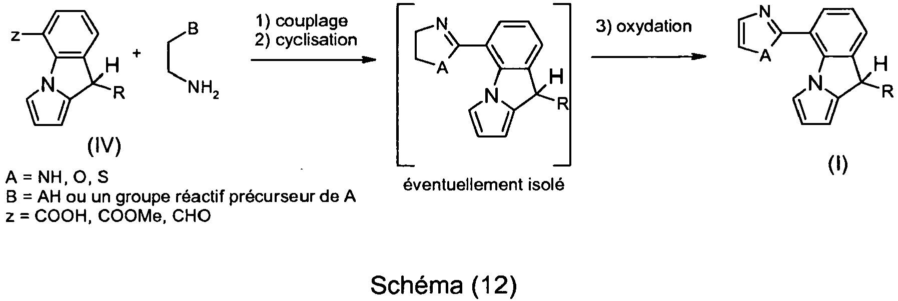 Dans le cadre de l'invention il est particulièrement avantageux d'opérer :
4. dans le cas où ledit hétérocycle est un imidazole ou une imidazoline :
   - à partir d'une 2-azido-éthylamine, selon Tetrahedron, 47(38), 1991, 8177-94,
   - d'une éthylènediamine, selon Biorg. Med. Chem Lett. 12(3), 2002, 471-75,
   - de glyoxal et d'ammoniaque, selon J. Med. Chem., 46(25), 2003, 5416-27 ;
5. dans le cas où ledit hétérocycle est un oxazole ou une oxazoline :
   - à partir d'un 2-azido-éthanol, selon J. Org. Chem., 61 (7), 1996, 2487-96,
   - d'un 2-aminoéthanol, selon J. Med. Chem. 47(8), 2004, 1969-86 ou Khim. Geterosikl. Soed. 1984(7), 881-4,
   - de diéthylacétal de 2-aminoacétaldéhyde, selon Heterocycles, 39(2), 1994, 767-78 ;
6. dans le cas où ledit hétérocycle est thiazole ou une thiazoline :
   - à partir d'une 2-chloro-éthylamine et de réactif de Lawesson, selon Helv. Chim. Acta, 88(2), 2005, 187-95,
   - d'un 2-aminoéthanethiol, selon J. Org. Chem. 69(3), 2004, 811-4, ou Tetrahedron Lett., 41(18), 2000, 3381-4,

D'une manière plus générale, il est avantageux, dans le cadre de l'invention, de former l'hétérocycle d'un produit de formule générale (IV) à partir d'un triflate, d'un dérivé bromé ou iodé, d'un acide ou d'un ester boronique, d'un acide carboxylique, d'un ester d'acide carboxylique ou d'un aldéhyde, en position 5 du pyrrolo[1,2-a]indole par l'une quelconque des méthodes de synthèse connues de l'homme de l'art, telles que celles décrites dans Comprehensive Organic Chemistry, par D. H.R. Barton et al. (Pergamon Press) ou Advances in Heterocyclic Chemistry (Academic Press) ou Heterocyclic Compounds (Wiley Intersciences).
La présente invention a ainsi également pour objet à titre de produits industriels nouveaux, les intermédiaires de synthèse de formules (II), (III) et (IV): 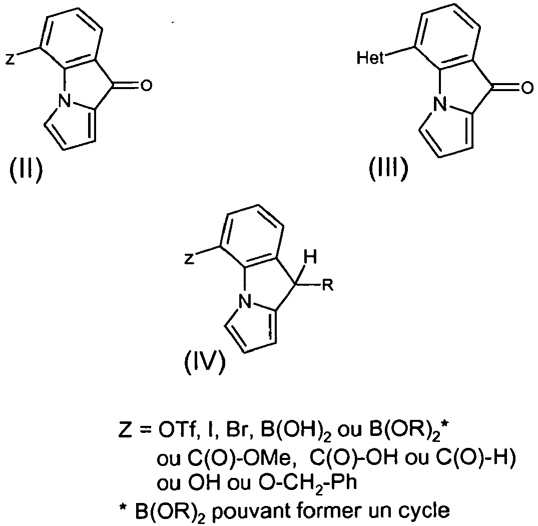 composés de formules (II), (III) et (IV) tels que définis ci dessus dans lesquels les substituants Het et R ont les significations indiquées ci-dessus pour les produits de formule (I) et z a la signification indiquée ci-dessus, à l'exclusion du composé de formule (II) dans lequel z représente un atome d'iode.

Les produits objet de la présente invention sont doués de propriétés pharmacologiques intéressantes: on a constaté qu'ils possédaient notamment des propriétés inhibitrices des activités des protéines chaperones et notamment de leurs activités ATPasiques.

Parmi ces protéines chaperones, on cite notamment la chaperone humaine HSP90.

Les produits répondant à la formule générale (I) tels que définis ci-dessus présentent ainsi une activité inhibitrice de la chaperone Hsp90 importante.

Des tests donnés dans la partie expérimentale ci-après illustrent l'activité inhibitrice de produits de la présente invention vis-à-vis de telles protéines chaperones.

Ces propriétés rendent donc les produits de formule générale (1) de la présente invention utilisables comme médicaments pour le traitement de tumeurs malignes.

Les produits de formule (I) peuvent également être utilisés dans le domaine vétérinaire.

L'invention a donc pour objet l'application, à titre de médicaments, des produits de formule (I) tels que définis ci-dessus.

L'invention a particulièrement pour objet l'application à titre de médicaments, des produits de formule (I) tels que définis ci-dessus dont les noms suivent :
- le [5-(6-fluoro-1H-benzimidazol-2-yl)-9H-pyrrolo[1 ,2-a]indol-9-yl]amide de l'acide 1H-pyrrolo[2,3-b]pyridine-4-carboxylique.
- le (5-quinoléin-3-yl-9H-pyrrolo[1,2-a]indol-9-yl)-amide de l'acide 1H-pyrrolo[2,3-b]pyridine-4-carboxylique.
- le [5-(3H-imidazo[4,5-c]pyridin-2-yl)-9H-pyrrolo[1,2-a]indol-9-yl]-amide de l'acide [1H-pyrrolo[2,3-b]pyridine-4-carboxylique.
- le [5-(3H-imidazo[4,5-c]pyridin-2-yl)-9H-pyrrolo[1,2-a]indol-9-yl]-amide de l'acide 6-chloro-1H-pyrrolo[2,3-b]pyridine-4-carboxylique,
ainsi que les sels d'addition avec les acides minéraux et organiques ou avec les bases minérales et organiques pharmaceutiquement aceptables desdits produits de formule (I).

L'invention a notamment pour objet à titre de médicaments, des produits de formule (I) tels que définis ci-dessus :
Het est choisi dans le groupe constitué par : 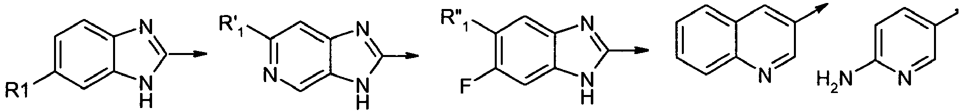 avec :
   R1 représente H, F, Cl, Br, CF3, NO2, CN, CH3, OH, OCH3, OCF3, CO2Me, CONH2, CONHMe, CONH-(CH2)3-OMe, CONH-(CH2)3-N(Me)2, NHC(O)Me, SO2NH2, SO2N(Me)2,
   R'1 représente H, CONH2, CONHMe et OMe,
   R"1 représente F, Cl, OH, OMe, CN, O-(CH2)3-OMe et O-(CH2)3-N(Me)2
et R est choisi dans le groupe constitué par : 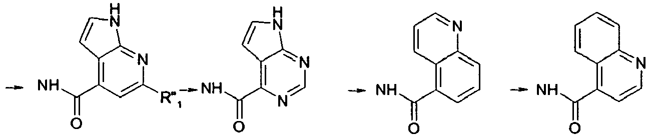 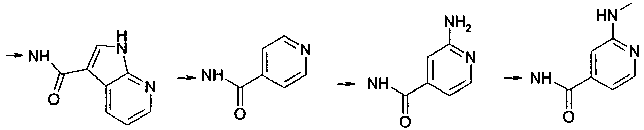 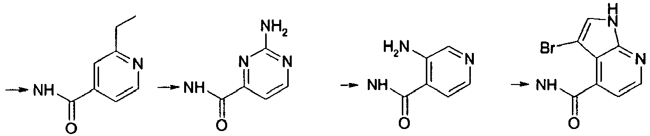 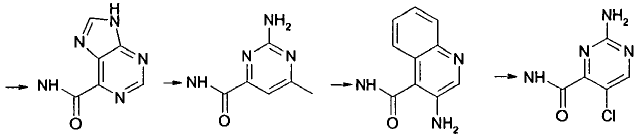 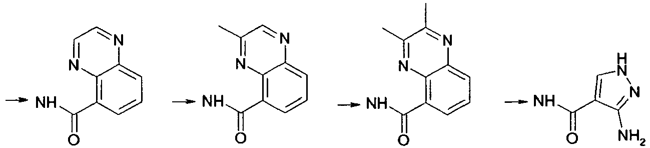 ainsi que leurs prodrogues, lesdits produits de formule (I) étant sous toutes les formes isomères possibles tautomères, racémiques, énantiomères et diastéréo-isomères, ainsi que les sels d'addition avec les acides minéraux et organiques ou avec les bases minérales et organiques pharmaceutiquement acceptables desdits produits de formule (I).

Les produits peuvent être administrés par voie parentérale, buccale, perlinguale ou topique.

L'invention a aussi pour objet les compositions pharmaceutiques, caractérisées en ce qu'elles renferment, à titre de principe actif, un au moins des médicaments de formule générale (1).

Ces compositions peuvent être présentées sous forme de solutions ou de suspensions injectables, de comprimés, de comprimés enrobés, de capsules, de sirops, de crèmes, de pommades et de lotions. Ces formes pharmaceutiques sont préparées selon les méthodes usuelles. Le principe actif peut être incorporé à des excipients habituellement employés dans ces compositions, tels que les véhicules aqueux ou non, le talc, la gomme arabique, le lactose, l'amidon, le stéarate de magnésium, les corps gras d'origine animale ou végétale, les dérivés paraffiniques, les glycols, les divers agents mouillants, dispersants ou émulsifiants, les conservateurs.

La dose usuelle, variable selon le sujet traité et l'affection en cause, peut être, par exemple, de 10 mg à 1 g par jour chez l'homme, par voie orale.

La présente invention concerne ainsi l'utilisation de produits de formule (I) tels que définis ci-dessus ou de sels pharmaceutiquement acceptables desdits produits de formule (I) pour la préparation de médicaments destinés à inhiber l'activité de protéines chaperones et notamment d'Hsp90.

La présente invention concerne ainsi particulièrement l'utilisation de produits de formule (I) telle que définie ci-dessus ou de sels pharmaceutiquement acceptables desdits produits de formule (I) dans laquelle la protéine chaperone est HSP90.

La présente invention concerne ainsi l'utilisation de produits de formule (I) telle que définie ci-dessus ou de sels pharmaceutiquement acceptables desdits produits de formule (I) pour la préparation d'un médicament destiné à prévenir ou traiter une maladie caractérisée par le dérèglement de l'activité d'une protéine chaperone de type Hsp90 et notamment une telle maladie chez un mammifère.

La présente invention concerne l'utilisation de produits de formule (I) telle que définie ci-dessus ou de sels pharmaceutiquement acceptables desdits produits de formule (I) pour la préparation d'un médicament destiné à prévenir ou traiter une maladie appartenant au groupe suivant: les maladies neurodégénératives telles que la maladie de Huntington, la maladie de Parkinson, l'ischémie cérébrale focale, la maladie d'Alzheimer la sclérose en plaques et la sclérose latérale amyotrophique, la malaria, les filarioses de Brugia et de Bancroft, la toxoplasmose, les mycoses résistantes aux traitements, l'hépatite B, l'hépatite C, le virus de l'Herpes, la dengue (ou grippe tropicale), l'atrophie musculaire spinale et bulbaire, désordres de la prolifération de cellules mésangiales, thromboses, rétinopathies, psoriasis, dégénération musculaire, maladies en oncologie, cancers.

La présente invention concerne ainsi l'utilisation de produits de formule (I) telle que définie ci-dessus ou de sels pharmaceutiquement acceptables desdits produits de formule (I) pour la préparation d'un médicament destiné à traiter des maladies en oncologie.

La présente invention concerne particulièrement l'utilisation de produits de formule (I) telle que définie ci-dessus ou de sels pharmaceutiquement acceptables desdits produits de formule (I) pour la préparation d'un médicament destiné à traiter des cancers.

Parmi ces cancers, la présente invention s'intéresse tout particulièrement au traitement des tumeurs solides et au traitement de cancers résistants aux agents cytotoxiques

La présente invention concerne ainsi notamment l'utilisation de produits de formule (I) telle que définie à l'une quelconque des revendications précédentes ou de sels pharmaceutiquement acceptables desdits produits de formule (I) pour la préparation d'un médicament destiné à traiter des cancers parmi lesquels les cancers du poumon, du sein et de l'ovaire, les glioblastomes, les leucémies myéloîdes chroniques, les leucémies lymphoblastiques aigûes, les cancers de la prostate, du pancréas et du colon, les mélanomes métastatiques, les tumeurs de la thyroîde et les carcinomes rénaux.

Aussi parmi les principales indications potentielles d'inhibiteurs d'Hsp90, peut-on citer, à titre non limitatif:
- les cancers du poumon « à non petites cellules », les cancers du sein, les cancers de l'ovaire ete les glioblastomes qui surexpriment EGF-R ou HER2 ;
- les leucémies myéloîdes chroniques qui surexpriment Bcr-Abl ;
- les leucémies lymphoblastiques aigûes qui surexpriment Flt-3 ;
- les cancers du sein, de la prostate, du poumon, du pancrés, du colon ou de l'ovaire qui surexpriment Akt ;
- les mélanomes metastatiques et les tumeurs de la thyroîde qui surexpriment la forme mutée de la protéine B-Raf ;
- les cancers de la prostate androgène-dépendants et androgène-indépendants ;
- les cancers du sein estrogène-dépendants et estrogène-indépendants ;
- les carcinomes rénaux qui surexpriment HIF-1α ou la protéine c-met mutée.

La présente invention s'intéresse encore plus particulièrement au traitement du cancer du sein, du colon et des poumons.

La présente invention concerne aussi l'utilisation de produits de formule (I) telle que définie ci-dessus ou de sels pharmaceutiquement acceptables desdits produits de formule (I) pour la préparation d'un médicament destiné à la chimiothérapie de cancers.

A titre de médicaments selon la présente invention destinés à la chimiothérapie de cancers, les produits de formule (I) selon la présente invention peuvent être utilisés seuls ou en association avec chimiothérapie ou radiothérapie ou alternativement en association avec d'autres agents thérapeutiques.

La présente invention concerne ainsi notamment les compositions pharmaceutiques telles que définies ci-dessus contenant en plus, des principes actifs d'autres médicaments de chimiothérapie contre le cancer.

De tels agents thérapeutiques peuvent être des agents anti-tumoraux utilisés communément.

Comme exemples d'inhibiteurs connus de protéines kinases, on peut citer notamment la butyrolactone, le flavopiridol, la 2-(2-hydroxyéthylamino)-6-benzylamino-9-méthylpurine, l'olomucine, le Glivec ainsi que l'Iressa.

Les produits de formule (I) selon la présente invention peuvent ainsi également être avantageusement utilisés en combinaison avec des agents anti-prolifératifs : à titre d'exemples de tels agents anti-prolifératifs mais sans toutefois se limiter à cette liste, on peut citer les inhibiteurs d'aromatase, les antiestrogènes, les inhibiteurs de topoisomérase, les agents actifs sur les microtubules, les agents d'alkylation, les inhibiteurs d'histone désacétylase, les inhibiteurs de farnésyl transférase, les inhibiteurs de COX-2, les inhibiteurs de MMP, les inhibiteurs de mTOR , les antimétabolites antinéoplastique, les composés du platine, les inhibiteurs de protéasome, les inhibiteurs d'Histone Déactylase (HDACs), et notamment les inhibiteurs d'HDAC6, les composés faisant décroître l'activité des protéines kinases et également les composés anti-angiogéniques, les agonistes de la gonadoréline, les anti-androgènes.

La présente invention concerne donc des produits de formule (I) comme inhibiteurs de la chaperone Hsp90, lesdits produits de formule (I) étant sous toutes les formes isomères possibles tautomères, racémiques, énantiomères et diastéréo-isomères, ainsi que les sels d'addition avec les acides minéraux et organiques ou avec les bases minérales et organiques pharmaceutiquement acceptables desdits produits de formule (I) .

La présente invention concerne particulièrement des produits de formule (I) tels que définis ci-dessus comme inhibiteurs de HSP90.

Les produits de formule (I) selon la présente invention peuvent être préparés par l'application ou l'adaptation de méthodes connues et notamment des méthodes décrites dans la littérature comme par exemple celles décrites par R.C.Larock dans: Comprehensive Organic Transformations, VCH publishers, 1989.

Dans les réactions décrites ci-après, il peut être nécessaire de protéger des groupes fonctionnels réactifs tels que par exemple des groupements hydroxy, amino, imino, thio ou carboxy, quand ceux-ci sont désirés dans le produit final mais quand leur participation n'est pas souhaitée dans les réactions de synthèse des produits de formule (I). On peut utiliser des groupes protecteurs conventionnels en accord avec les pratiques usuelles standard comme ceux décrits par exemple par T.W. Greene and P.G.M.Wuts dans "Protective Groups in Organic Chemistry" John Wiley and Sons, 1991.

La partie expérimentale ci-après donne des exemples non limitatifs de produits de départ: d'autres produits de départ peuvent être trouvés dans le commerce ou préparés selon les méthodes usuelles connues de l'homme du métier.

Exemples illustrant l'invention : Les exemples dont la préparation suit illustrent la présente invention sans toutefois la limiter.

Tous les exemples décrits ont été caractérisés par spectroscopie RMN du proton et par spectroscopie de masse, la majorité de ces exemples ont également été caractérisés en spectroscopie Infra Rouge.

Sauf conditions différentes spécifiquement décrites, les spectres de masse de type LC/MS, reportés dans la description des différents exemples ci-dessous, ont été effectués dans les conditions de chromatographie liquide suivantes :

### Méthode A :

Les spectres ont été obtenus sur un appareil Waters ZQ
Ionisation : électrospray en mode positif et/ou négatif (ES+/-)
Conditions chromatographiques :
- Colonne : XBridge C₁₈ 2,5 µm 3 x 50 mm
- Solvants : A : H₂O (0,1 % acide formique)
   B : CH₃CN (0,1 % acide formique)
- Température de colonne : 70 °C
- Débit : 0,9 ml/min
- Gradient (7 min) : de 5 à 100 % de B en 5,3 min ; 5,5 min : 100 % de B ; 6,3 min: 5%deB

### Méthode B :

Les spectres ont été obtenus sur un appareil WATERS QUATTRO PREMIER
Ionisation : électrospray en mode positif et/ou négatif (ES+/-)
Conditions chromatographiques :
- Colonne : ACQUITY BEH C18 1,7 µm - 2,1 x 50 mm
- Solvants : A : H2O (0,1 % acide formique)
   B : CH3CN (0,1 % acide formique)
- Température de colonne : 70°C
- Débit : 0,7 ml/min
- Gradient (3,7 min) : de 5 à 100 % de B en 3 min ; 3,6 min : 5 % de B

### Méthode C :

Les spectres ont été obtenus sur un appareil WATERS QUATTRO PREMIER
Ionisation : électrospray en mode positif et/ou négatif (ES+/-)

*Conditions chromatographiques :*
- Colonne : ACQUITY BEH C₁₈1,7 µm - 2,1 x 50 mm
- Solvants : A : H₂O (0,1 % acide formique)
   B : CH₃CN (0,1 % acide formique)
- Température de colonne : 70°C
- Débit : 0,7 ml/min
- Gradient (6 min) : de 5 à 100 % de B en 5 min ; 5,5 min : 5 % de B

### Méthode D :

Les spectres ont été obtenus sur un appareil WATERS UPLC-SQD
Ionisation : électrospray en mode positif et/ou négatif (ES+/-)
Conditions chromatographiques :
- Colonne : ACQUITY BEH C1 8 1,7 2,1 x 50 mm
- Solvants : A : H2O (0,1 % acide formique)
   B : CH3CN (0,1 % acide formique)
- Température de colonne : 50°C
- Débit : 1 ml/min
- Gradient (2mn) : de 5 à 50 % de B en 0,8 min ; 1,2 min : 100 % de B ; 1,85 m in : 100% de B ; 1,95: 5 % de B

### Exemple 1 : Synthèse du [5-(6-fluoro-1H-benzimidazol-2-yl)-9H-pyrrolo[1,2-a]indol-9-yl]amide de l'acide 1H-pyrrolo[2,3-b]pyridine-4-carboxylique.

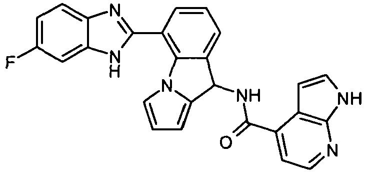

### Etape 1 :

Méthode 1 :Dans un autoclave on maintient pendant 16 heures à 50°C sous 2 bars de pression de monoxyde de carbone un mélange de 1,00g de 5-iodo-pyrrolo[1,2-a]indol-9-one (qui peut-être préparée selon J.Med.Chem. 2004, 47(6), 1448), 152mg d'acétate de palladium et de 280mg de 1,3-diphénylphosphinopropane et 0.47mL de triéthylamine dans 6mL de méthanol et 15mL de diméthylformamide. Après purge à l'argon, on filtre le milieu réactionnel en lavant l'insoluble avec du méthanol puis évapore à sec le filtrat. Le résidu brun orangé est chromatographié sur gel de silice (40-63µm) en éluant avec du dichlorométhane. On obtient 800mg d'ester de méthyle de l'acide 9-oxo-9H-pyrrolo(1,2-a]indole-5-carboxylique sous forme d'un solide beige dont les caractéristiques sont les suivantes :
- Spectre de RMN 1H (400MHz, δ en ppm, DMSO-d6) : 3.95 (s, 3H); 6.43 (dd, *J*=3.8 et 2.7 Hz, 1H); 6.98 (dd, *J*=3.8 et 1.0 Hz, 1H); 7.33 (t, *J*=7.5 Hz, 1H); 7.78 (dd, *J=*7.5 et 1.4 Hz, 1H); 7.97 à 8.03 (m, 2H).
- Spectre de masse (LC/MS Méthode A) : Temps de rétention Tr (min) = 3,48; [M+H]⁺ : m/z 228.

Méthode 2: Dans un ballon de 50mL on ajoute à température ambiante 3mL d'une solution 1M de tribromure de bore dans du dichlorométhane à un mélange de 260mg de diméthyl ester de l'acide 2-pyrrol-1-yl-isophthalique (qui peut être préparé selon Helvetica Chim.Acta 1983, 66(7), 2135) dissous dans 4mL de dichlorométhane. Le milieu réactionnel est agité à température ambiante pendant 2 heures puis on additionne 5mL d'eau. Après décantation, la phase aqueuse est réextraite avec 2 fois 15mL de dichlorométhane. Les phases organiques regroupées sont lavées avec 2 fois 20mL d'eau, séchées sur sulfate de magnésium et évaporées à sec sous vide. Le résidu est chromatographié sur gel de silice (15-40µm) en éluant avec un mélange de dichlorométhane et de cyclohexane (50/50 v/v). On obtient 122mg d'ester de méthyle de l'acide 9-oxo-9H-pyrrolo[1,2-a]indole-5-carboxylique sous forme d'un solide jaunâtre dont les caractéristiques sont identiques à celles du produit obtenu par la méthode 1.

Etape 2 : Dans un ballon de 250mL on chauffe au reflux pendant 3 heures un mélange de 800mg d'ester de méthyle de l'acide 9-oxo-9H-pyrrolo[1,2-a]indole-5-carboxylique obtenu selon l'étape précédente et de 740mg de monohydrate d'hydroxyde de lithium dans 25mL d'eau et 100mL de méthanol. Le milieu réactionnel est évaporé à sec sous vide, le résidu solide est repris dans 35mL d'eau et amené à pH 3 avec de l'acide chlorhydrique 5M. Un précipité apparaît et le milieu est extrait avec 3 fois 35mL d'un mélange de dichlorométhane et de méthanol (95/5 v/v), les extraits organiques réunis sont lavés avec 3 fois 15mL d'eau, séchés sur sulfate de magnésium et évaporés à sec sous vide. On obtient 630mg d'acide 9-oxo-9H-pyrrolo[1,2-a]indole-5-carboxylique sous forme d'un solide beige dont les caractéristiques sont les suivantes :
- Spectre de masse (LC/MS Méthode A) : Temps de rétention Tr (min) = 2,69; [M+H]⁺ : m/z 214; [M-H]⁻ : m/z 212.

,Etape 3: Dans un ballon de 250mL sous argon, on agite à température ambiante pendant 1 heure un mélange de 620mg d'acide 9-oxo-9H-pyrrolo[1,2-a]indole-5-carboxylique obtenu selon l'étape précédente, de 1.50g d' hexafluorophosphate de o-(1H-benzotriazole-1-yl)-N,N,N',N'-tetraméthyluronium (HBTU) et de 0.44mL de diisopropyléthylamine dans 55mL de tétrahydrofuranne. On ajoute alors 367mg de 4-fluoro-o-phénylènediamine et maintient l'agitation pendant 3 jours. Le milieu réactionnel est évaporé à sec sous vide et le résidu est repris avec 15mL d'un mélange de dichlorométhane et de méthanol (90/10 v/v). La phase organique est lavée avec 10mL d'une solution saturée de bicarbonate de sodium, puis avec 3 fois 5mL d'eau distillée et séchée sur sulfate de magnésium. Après évaporation à sec sous vide, le résidu est déposé en spot sec sur une colonne de gel de silice (40-63µm) et chromatographié en éluant avec un mélange de dichlorométhane et de méthanol (95/5 v/v). On obtient 379mg de (2-amino-4-fluoro-phényl)-amide de l'acide 9-oxo-9H-pyrrolo[1,2-a]indole-5-carboxylique sous la forme d'un solide beige dont les caractéristiques sont les suivantes :
- Spectre de RMN 1H (400MHz, δ en ppm, DMSO-d6) : 5.38 (s large, 2H); 6.35 (dd, *J*=3.5 et 2.5 Hz, 1H); 6.39 (td, *J*=8.6 et 2.9 Hz, 1H); 6.55 (dd, *J*=11.2 et 2.9 Hz, 1H); 6.92 (d large, *J*=3.5 Hz, 1H); 7.22 (dd, *J*=8.6 et 6.4 Hz, 1H); 7.33 (t, *J*=7.6 Hz, 1H); 7.57 (d large, *J*=2.5 Hz, 1H); 7.68 (d large, *J*=7.6 Hz, 1H); 7.94 (d large, *J*=7.6 Hz, 1H); 9.91 (s, 1H).
- Spectre de masse (LC/MS Méthode A) : Temps de rétention Tr (min) = 3,10; [M+H]+ _{:} m/z 322; [M-H]- : m/z 320.

Etape 4 : Dans un ballon de 250mL on porte au reflux pendant 3 heures un mélange de 375mg de (2-amino-4-fluoro-phényl)-amide de l'acide 9-oxo-9H-pyrrolo[1,2-a]indole-5-carboxylique obtenu selon l'étape précédente dans 40mL d'acide acétique glacial. Le milieu réactionnel est évaporé à sec sous vide et le résidu est chromatographié sur gel de silice (15-40µm) en éluant avec un gradient de méthanol (0% à 30% en volume) dans du dichlorométhane. On obtient 119mg de 5-(6-fluoro-1H-benzimidazol-2-yl)-pyrrolo[1,2-a]indol-9-one sous forme d'un solide jaunâtre dont les caractéristiques sont les suivantes :
- Spectre de RMN 1H (400MHz, δ en ppm, DMSO-d6) : 6.36 (dd, *J*=3.9 et 2.9 Hz, 1H); 6.96 (dd, *J*=3.9 et 1.0 Hz, 1H); 7.16 (td, *J*=9.5 et 2.4 Hz, 1H); 7.42 (t, *J*=7.6 Hz, 1H); 7.50 (d large, *J*=9.5 Hz, 1H); 7.69 (dd, *J*=7.6 et 1.0 Hz, 1H); 7.72 (m partiellement masqué, 1H); 7.99 (dd, *J*=7.6 et 1.0 Hz, 1H); 8.45 (dd, *J*=2.9 et 1.0 Hz, 1H); 13.30 (m étalé, 1H).
- Spectre de masse (LC/MS Méthode B) : Temps de rétention Tr (min) = 1,83; [M+H]+ : m/z 304; [M-H]-: m/z 302.

Etape 5 : Dans un ballon de 20mL on porte au reflux un mélange de 60mg de 5-(6-fluoro-1H-benzimidazol-2-yl)-pyrrolo[1,2-a]indol-9-one obtenu selon l'étape précédente et de 42mg de chlorhydrate d'hydroxylamine dans 5mL de pyridine pendant 6 heures. Le milieu réactionnel est évaporé à sec sous vide et le résidu repris avec 25mL d'une solution saturée de bicarbonate de sodium est extrait avec 3 fois 25mL d'acétate d'éthyle. Les phases organiques regroupées sont lavées avec 25mL d'une solution saturée de chlorure de sodium, séchées sur sulfate de magnésium et évaporées à sec sous vide. On obtient 97mg de 5-(6-fluoro-1H-benzimidazol-2-yl)-pyrrolo[1,2-a]indol-9-one oxime sous forme d'une huile jaunâtre dont les caractéristiques sont les suivantes :
- Spectre de RMN 1H (400MHz, δ en ppm, DMSO-d6) : 6.34 (dd, *J*=3.4 et 2.9 Hz, 1H); 6.78 (dd, *J*=3.4 et 1.0 Hz, 1H); 7.14 (td, *J*=10.5 et 3.0 Hz, 1H);7.37 (t, *J*=7.7 Hz, 1H); 7.48 (m étalé, 1H); 7.68 (m étalé, 1H); 7.82 (d, *J*=7.7 Hz, 2H); 8.10 (d large, *J*=2.9 Hz, 1H); 12.14 (s, 1H); 13.2 (m étalé, 1H) pour l'isomère E ou Z majoritaire.
- Spectre de masse (LC/MS Méthode A) : Temps de rétention Tr (min) = 3,65; [M+H]+ : m/z 319; [M-H]-: m/z 317.

Etape 6 : Dans un ballon de 100mL on agite à température ambiante 90mg de 5-(6-fluoro-1H-benzimidazol-2-yl)-pyrrolo[1,2-a]indol-9-one oxime obtenue selon l'étape précédente dans un mélange de 2mL d'acide acétique, 2mL d'éthanol et 2mL d'eau. On rajoute 19mg de zinc en poudre et maintient l'agitation pendant ½ heure. Le milieu réactionnel est filtré sur clarcel, lavé avec 80mL de méthanol et le filtrat est évaporé à sec sous vide. Le résidu est chromatographié sur gel de silice (15-40µm) en éluant avec un mélange de méthanol et de dichlorométhane (5/95 v/v). On obtient 42mg d'acétate de 5-(6-fluoro-1H-benzimidazol-2-yl)-9H-pyrrolo[1,2-a]indol-9-ylamine sous forme d'un solide beige dont les caractéristiques sont les suivantes :
- Spectre de RMN 1H (400MHz, δ en ppm, DMSO-d6) : 1.89 (s, 3H); 4.93 (s, 1H); 6.08 à 6.20 (m, 2H); 7.12 (t large, *J*=9.5 Hz, 1H); 7.24 à 7.80 (m très étalé, 2H); 7.29 (t, *J*=7.8 Hz, 1H); 7.53 (s large, 1H); 7.64 (d large, *J*=7.8 Hz, 1H); 7.71 (d large, *J*=7.8 Hz, 1H); 13.08 (m étalé, 1H).
- Spectre de masse (LC/MS Méthode A) : Temps de rétention Tr (min) = 2,51; [M+H]+ _{:} m/z 305; [M+H-NH3]+ _{:} m/z 288 (pic de base); [M-H]- : m/z 303.

Etape 7 : Dans un ballon de 100mL on agite un mélange de 42mg d'acétate de 5-(6-fluoro-1H-benzimidazol-2-yl)-9H-pyrrolo[1,2-a]indol-9-ylamine obtenu selon l'étape précédente, de 19mg d'acide 1H-pyrrolo[2,3-b]pyridine-4-carboxylique (qui peut-être obtenu selon WO 2003/000688), de 39mg de tétrafluoroborate de o-((éthoxycarbonyl)cyanométhylèneamino)-N,N,N',N'-tetraméthyluronium (TOTU) et de 16µL de diisopropyléthylamine dans 3mL de N-méthylpyrrolidone. Après 1 heure on évapore à sec le milieu réactionnel, reprend le résidu avec 30mL d'une solution saturée de bicarbonate de sodium et extrait la phase acqueuse par 4 fois 30mL d'acétate d'éthyle. Les phases organiques regroupées sont lavées avec 3 fois 30mL d'une solution saturée de chlorure de sodium, séchées sur sulfate de magnésium et évaporées à sec sous vide. Le résidu est chromatographié sur gel de silice (15-40µm) en éluant avec un gradient de méthanol (2% à 5% v/v) dans du dichlorométhane puis sur une deuxième colonne de gel de silice (15-40µm) en éluant avec de l'acétate d'éthyle pur. On obtient 12mg de [5-(6-fluoro-1H-benzimidazol-2-yl)-9H-pyrrolo[1,2-a]indol-9-yl]amide de l'acide 1H-pyrrolo[2,3-b]pyridine-4-carboxylique sous forme d'un solide brun dont les caractéristiques sont les suivantes :
- Spectre de RMN 1H : (400MHz, δ en ppm, DMSO-d6) : 6.22 à 6.28 (m, 2H); 6.40 (d, *J*=7.8 Hz, 1H); 6.89 (dd, *J*=3.4 et 2.0 Hz, 1H); 7.13 (m étalé, 1H); 7.33 (t, *J*=7.6 Hz, 1H); 7.38 (m étalé, 1H); 7.44 (d, *J*=4.9 Hz, 1H); 7.52 à 7.83 (m, 5H); 8.28 (d, *J*=4.9 Hz, 1H); 9.30 (d, *J*=7.8 Hz, 1H); 11.85 (m large, 1H); 13.14 (s, 1H).
- Spectre de masse (LC/MS Méthode A) : Temps de rétention Tr (min) = 3,53; [M+H]+ _{:} m/z 449; [2M+H]+ : m/z 897; [M+2H+CH₃CN]₂+: m/z 245,5 (pic de base).

### Exemple 2: Synthèse du (5-quinoléin-3-yl-9H-pyrrolo[1,2-a]indol-9-yl)-amide de l'acide 1H-pyrrolo[2,3-b]pyridine-4-carboxylique.

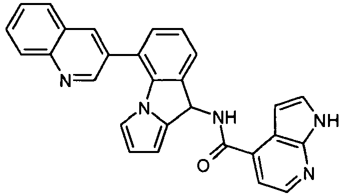

Etape 1 : Dans un ballon de 100mL sous argon, on chauffe à 123°C un mélange de 361 mg de 5-iodo-pyrrolo[1,2-a]indol-9-one (qui peut-être préparée selon J.Med.Chem. 2004, 47(6), 1448), de 214mg d'acide 3-quinoléine boronique, de 603mg de carbonate de césium et de 143mg de tetrakis(triphénylphosphine) palladium(0) dans 20mL de diméthylformamide. Après 3 heures, le milieu réactionnel est évaporé à sec sous vide et le résidu repris dans de l'acétate d'éthyle est filtré. Le filtrat est lavé avec de l'eau, 2 fois avec une solution saturée de bicarbonate de sodium puis avec une solution saturée de chlorure de sodium, séché avec du sulfate de magnésium et évaporé à sec sous vide. Le résidu est chromatographié sur gel de silice (15-40µm) en éluant avec un gradient d'acétate d'éthyle (20% à 30% v/v) dans du n-heptane. On obtient 231 mg de 5-quinoléin-3-yl-pyrrolo[1,2-a]indol-9-one sous forme d'un solide dont les caractéristiques sont les suivantes :
- Spectre de RMN 1H : (400MHz, δ en ppm, CDCl3) : 6.08 (dd, *J*=3.8 et 2.6 Hz, 1H); 6.33 (dd, *J*=2.6 et 0.9 Hz, 1H); 6.80 (dd, *J*=3.8 et 0.9 Hz, 1H); 7.27 (t, *J*=7.7 Hz, 1H); 7.41 (dd, *J*=7.7 et 1.3 Hz, 1H); 7.65 à 7.72 (m, 2H); 7.85 (m, 1H); 7.92 (d large, *J*=8.3 Hz, 1H); 8.24 (d large, *J*=8.3 Hz, 1H); 8.32 (d, *J*=2.2 Hz, 1H); 9.07 (d, *J*=2.2 Hz, 1H).

Etape 2 : Dans un ballon de 100mL on chauffe au reflux sous argon un mélange de 231 mg de 5-quinoléin-3-yl-pyrrolo[1,2-a]indol-9-one obtenue selon l'étape précédente et de 164mg de chlorhydrate d'hydroxylamine dans 20mL de pyridine. Après 1,5 heures de chauffage, le milieu réactionnel est évaporé à sec sous vide. Le résidu est repris dans une solution saturée de bicarbonate de sodium et extraite 2 fois avec de l'acétate d'éthyle. Les phases organiques regroupées sont lavées avec une solution saturée de bicarbonate de sodium puis une solution saturée de chlorure de sodium, séchées sur sulfate de magnésium et évaporées à sec sous vide. Le résidu est chromatographié sur gel de silice (15-40µm) en éluant avec un gradient de méthanol (2% à 3% v/v) dans le dichlorométhane. On obtient 277mg de chlohydrate de 5-quinoléin-3-yl-pyrrolo[1,2-a]indol-9-one oxime sous forme d'un solide jaunâtre dont les caractéristiques sont les suivantes :
- Spectre de RMN 1H : (400MHz, δ en ppm, DMSO-d6) : 6.20 (dd, *J*=3.5 et 2.9 Hz,1H); 6.24 (dd, *J*=2.9 et 1.1 Hz, 1H); 6.72
   (dd, *J*=3.5 et 1.1 Hz, 1H); 7.34 (t, *J*=7.7 Hz, 1H); 7.47 (dd, *J*=7.7 et 1.3 Hz, 1H); 7.72 (m, 1H); 7.78 (dd, *J*=7.7 et 1.3 Hz, 1H); 7.88 (m, 1H); 8.10 (d large, *J*=7.9 Hz, 1H); 8.15 (d large, *J*=7.9 Hz, 1H); 8.62 (d, *J*=2.2 Hz, 1H); 9.06 (d, *J*=2.2 Hz, 1H); 12.18 (m étalé, 1H) pour l'isomère E ou Z majoritaire.

Etape 3 : Dans un ballon de 100mL on agite à température ambiante un mélange de 277mg de chlohydrate de 5-quinoléin-3-yl-pyrrolo[1,2-a]indol-9-one oxime obtenue selon l'étape précédente dans 5mL d'acide acétique, 5mL d'éthanol et 5mL d'eau. On additionne 58mg de zinc en poudre et laisse agiter pendant 1,5 heures. On rajoute encore 58mg de zinc en poudre et laisse sous agitation pendant 3,5 heures. Le milieu réactionnel est filtré sur clarcel en lavant avec 100mL de méthanol. Le filtrat est évaporé à sec sous vide et chromatographié sur gel de silice (15-40µm) en éluant avec un gradient de méthanol (2% à 7% v/v) dans du dichlorométhane. On obtient 116mg d'acétate de 5-quinolin-3-yl-9H-pyrrolo[1,2-a]indol-9-ylamine sous forme d'un solide marron dont les caractéristiques sont les suivantes :
- Spectre de RMN 1H : (300MHz, δ en ppm, DMSO-d6) : 1.89 (s, 3H); 4.96 (s, 1H); 5.98 (d large, *J*=3.1 Hz, 1H); 6.05 (t, *J*=3.1 Hz, 1H); 6.14 (dt, *J*=3.1 et 1.2 Hz, 1H); 7.28 (t, *J*=7.5 Hz, 1H); 7.35 (dd, *J*=7.5 et 1.2 Hz, 1H); 7.65 à 7.74 (m, 2H); 7.86 (m, 1H); 8.09 (d large, *J*=8.0 Hz, 1H); 8.14 (d large, *J*=8.0 Hz, 1H); 8.53 (d, *J*=2.3 Hz, 1H); 9.00 (d, *J*=2.3 Hz, 1H).

Etape 4: Dans un ballon de 100mL sous argon, on agite à température ambiante pendant 18 heures un mélange de 116mg d'acétate de 5-quinolin-3-yl-9H-pyrrolo[1,2-a]indol-9-ylamine obtenue selon l'étape précédente, de 63mg d'acide 1H-pyrrolo[2,3-b]pyridine-4-carboxylique (qui peut-être obtenu selon WO 2003/000688), de 82mg de chlorhydrate de N-(3-diméthylaminopropyl)-N'-éthylcarbodiimide (EDCI) et de 58mg de 1-hydroxybenzotriazole (HOBt) dans 7.5mL de diméthylformamide. Le milieu réactionnel est évaporé à sec sous vide et le résidu est additionné de 50mL d'eau et la suspension est agitée pendant 1 heure. Le solide est filtré et lavé avec de l'eau, puis une solution saturée de bicarbonate de sodium et à nouveau avec de l'eau. On essore toute une nuit puis reprend le solide dans un mélange de méthanol et de dichlorométhane (10/90 v/v) et le solvant évaporé à sec sous vide. Le résidu est chromatographié sur gel de silice (15-40µm) en éluant avec un mélange de méthanol et de dichlorométhane (3/97 v/v). On obtient 53mg de (5-quinoléin-3-yl-9H-pyrrolo[1,2-a]indol-9-yl)-amide de l'acide 1H-pyrrolo[2,3-b]pyridine-4-carboxylique sous forme d'un solide beige dont les caractéristiques sont les suivantes :
- Spectre de RMN 1H : (300MHz, δ en ppm, DMSO-d6) : 6.09 (d large, *J*=3.2 Hz, 1H); 6.13 (t, *J*=3.2 Hz, 1H); 6.24 (dm, *J*=3.2 Hz, 1H); 6.44 (d large, *J*=8.3 Hz, 1H); 6.90 (d large, *J*=3.3 Hz, 1H); 7.32 (t, *J*=7.7 Hz, 1H); 7.41 à 7.47 (m, 2H); 7.60 à 7.67 (m, 2H); 7.73 (m, 1H); 7.88 (m, 1H); 8.10 à 8.18 (m, 2H); 8.29 (d, *J*=5.0 Hz, 1H); 8.57 (m étalé, 1H); 9.04 (m large, 1H); 9.33 (d, J=8.3 Hz, 1H); 11.89 (m étalé, 1H).
- Spectre de masse (LC/MS Méthode C) : Temps de rétention Tr (min) = 2,36; [M+H]+ m/z 442; [M-H]- m/z 440

### Exemple 3: Synthèse du [5-(3H-imidazo[4,5-c]pyridin-2-yl)-9H-pyrrolo[1,2-a]indol-9-yl]-amide de l'acide [1H-pyrrolo[2,3-b]pyridine-4-carboxylique.

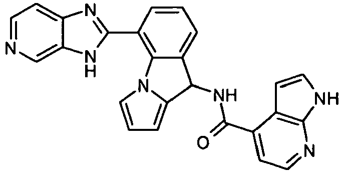

Etape 1 : Dans un ballon de 500mL on agite pendant 19 heures à température ambiante un mélange de 730mg d'acide 9-oxo-9H-pyrrolo[1,2-a]indole-5-carboxylique obtenu selon l'étape 2 de l'exemple 1, de 373mg de 3,4-diaminopyridine, de 721 mg de chlorhydrate de N-(3-diméthylaminopropyl)-N'-éthylcarbodiimide (EDCI) et de 508mg de 1-hydroxybenzotriazole (HOBt) dans 60mL de diméthylformamide. Le milieu réactionnel est évaporé à sec sous vide et le résidu solide est repris dans 50mL d'eau. Le précipité est filtré et le solide est lavé successivement avec 3 fois 80mL d'eau, 3 fois 80mL d'une solution saturée de bicarbonate de sodium et encore avec 3 fois 80mL d'eau. Le solide est dissous dans du méthanol et le solvant évaporé à sec sous vide. Le résidu est chromatographié sur gel de silice (15-40µm) en éluant avec un gradient de méthanol (5% à 10% v/v) dans du dichlorométhane puis un mélange de dichlorométhane et de méthanol ammoniacal 5N (90/10 v/v). On obtient 220mg de (4-amino-pyridin-3-yl)-amide de l'acide 9-oxo-9H-pyrrolo[1,2-a]indole-5-carboxylique sous forme d'un solide dont les caractéristiques sont les suivantes :
- Spectre de RMN 1H : (400MHz, δ en ppm, DMSO-d6) : 6.02 (s large, 2H); 6.35 (dd, *J*=3.7 et 2.7 Hz, 1H); 6.66 (d, *J*=5.6 Hz, 1H); 6.92 (dd, *J*=3.7 et 1.1 Hz, 1H); 7.33 (t, *J*=7.7 Hz, 1H); 7.58 (dd, *J*=2.7 et 1.1 Hz, 1H); 7.68 (dd, *J*=7.7 et 1.3 Hz, 1H); 7.95 (d, *J*=5.6 Hz, 1H); 8.00 (dd, *J*=7.7 et 1.3 Hz, 1H); 8.15 (s, 1H); 10.00 (s large, 1H).

Etape 2 : Dans un réacteur à micro-ondes de 20mL on chauffe à 200°C pendant 30 minutes un mélange de 256mg de (4-amino-pyridin-3-yl)-amide de l'acide 9-oxo-9H-pyrrolo[1,2-a]indole-5-carboxylique obtenu selon l'étape précédente dans 20mL d'acide acétique glacial. Le milieu réactionnel est filtré sur clarcel en lavant avec de l'acide acétique glacial. Le filtrat est évaporé à sec sous vide et le résidu repris 2 fois avec du toluène et réévaporé à chaque fois à sec sous vide. Le solide est trituré dans de l'oxyde d'isopropyle, filtré et séché sous vide à 40°C pendant 1 nuit. On obtient 111mg de 5-(3H-imidazo[4,5-c]pyridin-2-yl)-pyrrolo[1,2-a]indol-9-one sous forme d'un solide dont les caractéristiques sont les suivantes :
- Spectre de RMN 1H : (400MHz, δ en ppm, DMSO-d6) : 6.38 (dd, *J*=3.9 et 2.6 Hz, 1H); 6.98 (dd, *J*=3.9 et 1.1 Hz, 1H); 7.43 (t, *J*=7.6 Hz, 1H); 7.72 (dd, *J*=7.6 et 1.4 Hz, 1H); 7.76 (d large, *J*=5.6 Hz, 1H); 8.11 (d large, *J*=7.6 Hz, 1H); 8.38 (d, *J*=5.6 Hz, 1H); 8.50 (m étalé, 1H); 9.11 (s large, 1H); 13.8 (m très étalé, 1H).

Etape 3 : Dans un ballon de 250mL sous argon on chauffe au reflux pendant 3 heures un mélange de 444mg de 5-(3H-imidazo[4,5-c]pyridin-2-yl)-pyrrolo[1,2-a]indol-9-one obtenue selon l'étape précédente et de 323mg de chlorhydrate d'hydroxylamine dans 60mL de pyridine. Le milieu réactionnel est évaporé à sec sous vide et le résidu est repris dans une solution saturée de bicarbonate de sodium et le précipité est filtré et lavé avec de l'eau. Le solide est dissous dans du méthanol et le solvant évaporé à sec sous vide. On reprend le résidu dans du toluène puis réévapore à sec sous vide. On obtient 380mg de 5-(3H-imidazo[4,5-c]pyridin-2-yl)-pyrrolo[1,2-a]indol-9-one oxime sous forme d'un solide dont les caractéristiques sont les suivantes :
- Spectre de RMN 1H : (400MHz, δ en ppm, DMSO-d6) : 6.25 (dd, *J*=3.5 et 2.9 Hz, 1H); 6.72 (dd, *J*=3.5 et 1.3 Hz, 1H); 7.18 (t, *J*=7.7 Hz, 1H); 7.34 (dd, *J*=5.5 et 1.2 Hz, 1H); 7.59 (dd, *J*=7.7 et 1.4 Hz, 1H); 7.86 (d, *J*=5.5 Hz, 1H); 8.29 (dd, *J*=7.7 et 1.4 Hz, 1H); 8.69 (d, *J*=1.2 Hz, 1H); 9.23 (dd, *J*=2.9 et 1.3 Hz, 1H) pour l'isomère E ou Z majoritaire.

Etape 4 : Dans un ballon de 100mL on agite à température ambiante un mélange de 200mg de 5-(3H-imidazo[4,5-c]pyridin-2-yl)-pyrrolo[1,2-a]indol-9-one oxime obtenue selon l'étape précédente dans 5mL d'acide acétique glacial, 5mL d'éthanol et 5mL d'eau. On rajoute 43mg de zinc en poudre et maintient l'agitation à température ambiante pendant 2 heures. Le milieu réactionnel est filtré sur clarcel avec lavage avec du méthanol. Le filtrat est évaporé à sec sous vide et le résidu est est chromatographié sur gel de silice (15-40µm) en éluant avec un mélange de dichlorométhane et de méthanol ammoniacal 5N (95/5 v/v). On obtient 51 mg de 5-(3H-imidazo[4,5-c]pyridin-2-yl)-9H-pyrrolo[1,2-a]indol-9-ylamine sous forme d'un solide dont les caractéristiques sont les suivantes :
- Spectre de RMN 1H : (400MHz, δ en ppm, DMSO-d6) : 4.94 (s, 1H); 6.14 à 6.20 (m, 2H); 7.31 (t, *J*=7.6 Hz, 1H); 7.54 (s large, 1H); 7.64 (d, *J*=5.6 Hz, 1H); 7.70 (d, *J*=7.6 Hz, 1H); 7.74 (d, *J*=7.6 Hz, 1H); 8.36 (d, *J*=5.6 Hz, 1H); 9.01 (s, 1H).
- Spectre de masse (LC/MS Méthode A) : Temps de rétention Tr (min) = 0,32; [M+H]+: m/z 288; [M+H-NH₃]+: m/z 271; [M-H]-: m/z 286.

Etape 5 : Dans un ballon de 100mL on agite pendant 4 heures sous argon à température ambiante un mélange de 51 mg de 5-(3H-imidazo[4,5-c]pyridin-2-yl)-9H-pyrrolo[1,2-a]indol-9-ylamine obtenue selon l'étape précédente, de 28mg d'acide 1H-pyrrolo[2,3-b]pyridine-4-carboxylique (qui peut-être obtenu selon WO 2003/000688), de 37mg de chlorhydrate de N-(3-diméthylaminopropyl)-N'-éthylcarbodiimide (EDCI) et de 26mg de 1-hydroxybenzotriazole (HOBt) dans 5mL de diméthylformamide. Le milieu réactionnel est évaporé à sec sous vide. Le résidu est trituré dans 20mL d'eau puis filtré. Le solide est lavé avec une solution saturée de bicarbonate de sodium, repris dans un mélange de dichlorométhane et de méthanol et la solution est évaporée à sec sous vide. Le résidu est chromatographié sur gel de silice (15-40µm) en éluant avec un mélange de dichlorométhane et de méthanol (98/2 v/v). On obtient 47mg de [5-(3H-imidazo[4,5-c]pyridin-2-yl)-9H-pyrrolo[1,2-a]indol-9-yl]-amide de l'acide 1H-pyrrolo[2,3-b]pyridine-4-carboxylique sous forme d'un solide dont les caractéristiques sont les suivantes :
- Spectre de RMN 1H : (400MHz, δ en ppm, DMSO-d6) : 6 22 à 6.98 (m, 2H); 6.40 (d, *J*=8.2 Hz, 1H); 6.89 (dd, *J*=3.4 et 2.0 Hz, 1H); 7.33 (t, *J*=7.7 Hz, 1H); 7.44 (d, *J*=5.1 Hz, 1H); 7.61 (dd, *J*=3.4 et 2.5 Hz, 1H); 7.64 (d, *J*=6.2 Hz, 1H); 7.67 (d large, *J*=7.7 Hz, 1H); 7.84 (d, *J*=7.7 Hz, 1H); 7.85 (m étalé, 1H); 8.28 (d, *J*=5.1 Hz, 1H); 8.33 (d, *J*=6.2 Hz, 1H); 9.01 (s, 1H); 9.29 (d, *J*=8.2 Hz, 1H); 11.84 (m large, 1H); 13.47 (m étalé, 1H).
- Spectre de masse (LC/MS Méthode A) : Temps de rétention Tr (min) = 2,39; [M+H]+ : m/z 432; [M-H]- : m/z 430.

### Exemple 4 : Synthèse du [5-(3H-imidazo[4,5-c]pyridin-2-yl)-9H-pyrrolo[1,2-a]indol-9-yl]-amide de l'acide 6-chloro-1H-pyrrolo[2,3-b]pyridine-4-carboxylique.

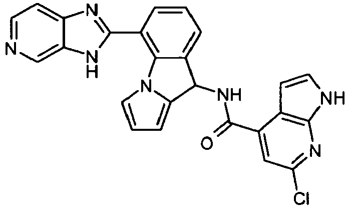

Etape 1 : Dans un tricol de 100mL on additionne en l'espace de 5 minutes sous argon à -5°C une solution de 1,55g d'acide 3-chloroperoxybenzoique dans 5mL d'acétate d'éthyle à un mélange de 881 mg d'ester de méthyle de l'acide 1H-pyrrolo[2,3-b]pyridine-4-carboxylique (qui peut- être obtenu selon WO 2003/000688) dans 10mL d'acétate d'éthyle. Après 1 heure d'agitation à -5°C on laisse revenir à température ambiante. Le solide marron est filtré, lavé avec 3 fois 3mL d'acétate d'éthyle et séché. On obtient 844mg d'ester de méthyle de l'acide 7-oxy-1H-pyrrolo[2,3-b]pyridine-4-carboxylique sous forme d'un solide marron dont les caractéristiques sont les suivantes :
- Spectre de RMN 1H : (400MHz, δ en ppm, DMSO-d6) : 3.93 (s, 3H); 6.93 (d, *J*=2.9 Hz, 1H); 7.63 (d, *J*=2.9 Hz, 1H); 7.64 (d, *J*=6.8 Hz, 1H); 8.23 (d, *J*=6.8 Hz, 1H); 12.79 (m étalé, 1H).
- Spectre de masse (LC/MS Méthode A) : Temps de rétention Tr (min) = 1,97; [M+H]+ : m/z 193; [M-H]-: m/z 191.

Etape 2 : Dans un tricol de 100mL en maintenant la température sous 25°C on additionne goutte à goutte 2,17mL de chlorure de méthanesulfonyle à une suspension de 4,49g d'ester de méthyle de l'acide 7-oxy-1H-pyrrolo[2,3-b]pyridine-4-carboxylique obtenu selon l'étape précédente, dans 30mL de diméthylformamide. Le milieu réactionnel est ensuite chauffé à 80°C pendant 1 heure. Après refroidissement on rajoute encore 2,17mL de chlorure de méthanesulfonyle en maintenant la température sous 35°C. On chauffe à nouveau le milieu réactionnel à 80°C pendant 1 heure. Après refroidissement on additionne 50mL d'eau en maintenant la température sous 30°C. Le milieu réactionnel très visqueux est refroidi à 5°C et le précipité est filtré. Le solide beige est lavé avec 5 fois 15mL d'eau et séché. On obtient 3,53g d'ester de méthyle de l'acide 6-chloro-1H-pyrrolo[2,3-b]pyridine-4-carboxylique sous forme d'un solide beige dont les caractéristiques sont les suivantes :
- Spectre de RMN 1H : (400MHz, δ en ppm, DMSO-d6) : 3.96 (s, 3H); 6.88 (dd, *J*=3.4 et 2.0 Hz, 1H); 7.57 (s, 1H); 7.75 (dd, *J*=3.4 et 2.5 Hz, 1H); 12.27 (m étalé, 1H).

Etape 3: Dans un ballon de 100mL on additionne à température ambiante 7,5mL d'une solution aqueuse 1 M d'hydroxyde de lithium à une suspension de 527mg d'ester de méthyle de l'acide 6-chloro-1H-pyrrolo[2,3-b]pyridine-4-carboxylique obtenu selon l'étape précédente, dans 15mL de méthanol. Après 2,5 heures d'agitation à température ambiante, on ajoute goutte à goutte 7,5mL d'acide chlorhydrique 1 N. Après ½ heure d'agitation, on filtre le solide, le lave avec 25mL d'eau et le sèche. On obtient 475mg d'acide 6-chloro-1H-pyrrolo[2,3-b]pyridine-4-carboxylique sous forme d'un solide beige dont les caractéristiques sont les suivantes :
- Spectre de RMN 1H : (400MHz, δ en ppm, DMSO-d6) : 6.87 (dd, *J*=3.4 et 2.0 Hz, 1H); 7.53 (s, 1H); 7.70 (dd, *J*=3.4 et 2.5 Hz, 1H); 12.19 (m étalé, 1H); 13.66 (m très étalé, 1H).
- Spectre de masse (LC/MS Méthode A) : Temps de rétention Tr (min) = 2,35; [M+H]+ : m/z 197; [M-H]- : m/z 195.

Etape 4 : Dans un ballon de 100mL on agite pendant 3 heures sous argon à température ambiante un mélange de 69mg de 5-(3H-imidazo[4,5-c]pyridin-2-yl)-9H-pyrrolo[1,2-a]indol-9-ylamine obtenue selon l'étape 4 de l'exemple 3, de 48mg d'acide 6-chloro-1H-pyrrolo[2,3-b]pyridine-4-carboxylique obtenu selon l'étape précédente, de 51 mg de chlorhydrate de N-(3-diméthylaminopropyl)-N'-éthylcarbodiimide (EDCI) et de 36mg de 1-hydroxybenzotriazole (HOBt) dans 5mL de diméthylformamide. Le milieu réactionnel est évaporé à sec sous vide et le résidu est chromatographié sur gel de silice (15-40µm) en éluant avec un gradient de méthanol (4% à 6% v/v) dans le dichlorométhane. Les fractions intéressantes sont regroupées, évaporées à sec sous vide et le résidu trituré dans une solution de bicarbonate de sodium, filtré et le solide lavé avec de l'eau. Le précipité est dissous dans du méthanol et le solvant est évaporé à sec sous vide. On obtient 11 mg de [5-(3H-imidazo[4,5-c]pyridin-2-yl)-9H-pyrrolo[1,2-a]indol-9-yl]-amide de l'acide 6-chloro-1H-pyrrolo[2,3-b]pyridine-4-carboxylique sous forme d'un solide dont les caractéristiques sont les suivantes :
- Spectre de RMN 1H : (300MHz, δ en ppm, DMSO-d6) : 6.21 à 6.28 (m, 2H); 6.36 (d large, *J*=8.3 Hz, 1H); 6.91 (d, *J*=3.7 Hz, 1H); 7.31 (t, *J*=7.8 Hz, 1H); 7.54 (s, 1H); 7.56 à 7.68 (m, 3H); 7.90 (d large, *J*=7.8 Hz, 1H); 8.04 (m étalé, 1H); 8.26 (d, *J*=6.0 Hz, 1H); 8.96 (s, 1H); 9.43 (d, *J*=8.3 Hz, 1H); 12.08 (m étalé, 1H).
- Spectre de masse (LC/MS Méthode D) : Temps de rétention Tr (min) = 0,59; [M+H]+ : m/z 466; [M-H]- : m/z 464.

### Exemple 5 : composition pharmaceutique

On a préparé des comprimés répondant à la formule suivante :

| | |
|---|---|
| Produit de l'exemple 1 | ........................ 0,2 g |
| Excipient pour un comprimé terminé à | ..... 1 g |

(détail de l'excipient : lactose, talc, amidon, stéarate de magnésium).

La présente invention comprend également toutes les compositions pharmaceutiques préparées avec tout produit de formule (I) selon la présente invention.

### Tests biologiques permettant de caractériser biologiquement les produits de l'invention :

Le phosphate inorganique libéré au cours de l'hydrolyse de l'ATP par l'activité ATPasique de Hsp82 est quantifié par la méthode du green Malachite. En présence de ce réactif, il y a formation du complexe phosphate inorganique-molybdate-vert de malachite qui absorbe à une longueur d'onde de 620 nm.

Les produits à évaluer sont incubés dans un volume réactionnel de 30 µl, en présence de 1 µM Hsp82 et de 250 µM de substrat (ATP) dans un tampon composé de 50 mM Hepes-NaOH (pH 7.5), 1 mM DTT, 5 mM MgCl2 et 50 mM KCl à 37 °C pendant 60 min. Parallèlement, une gamme de phosphate inorganique comprise entre 1 à 40 µM est constituée dans le même tampon.

L'activité ATPasique est ensuite révélée par l'addition de 60 µl du réactif biomol green (Tebu). Après 20 min d'incubation à température ambiante, l'absorbance des différents puits est mesurée à l'aide d'un lecteur de microplaque à 620 nm. La concentration en phosphate inorganique de chaque échantillon est alors calculée à partir de la courbe d'étalonnage.

L'activité ATPasique d'Hsp82 est exprimée en concentration de phosphate inorganique produit en 60 minutes. L'effet des divers produits testés est exprimé en pourcentage d'inhibition de l'activité ATPasique.

La formation d'ADP due à l'activité ATPasique de Hsp82 a été utilisée pour mettre au point une autre méthode d'évaluation de l'activité enzymatique de cette enzyme par application d'un système de couplage enzymatique faisant intervenir la pyruvate kinase (PK) et la lactate deshydrogensase (LDH). Dans cette méthode spectrophotométrique de type cinétique, la PK catalyse la formation d'ATP et de pyruvate à partir de phosphoenol-pyruvate (PEP) et de l'ADP produit par Hsp82. Le pyruvate formé, substrat de la LDH, est ensuite transformé en lactate en présence de NADH. Dans ce cas, la diminution de la concentration en NADH, mesurée par la diminution de l'absorbance à la longueur d'onde de 340 nm est proportionnelle à la concentration en ADP produit par Hsp82.

Les produits testés sont incubés dans un volume réactionnel de 100 µl de tampon composé de 100 mM Hepes-NaOH (pH 7.5), 5 mM MgCl2, 1 mM DTT, 150 mM KCI, 0.3 mM NADH, 2.5 mM PEP et 250 µM ATP. Ce mélange est pré-incubé à 37°C pendant 30 minutes avant addition de 3.77 unités de LDH et 3.77 unités de PK. La réaction est initiée par addition du produit à évaluer, en concentrations variables, et de Hsp82, à la concentration de 1 µM. La mesure de l'activité enzymatique de Hsp82 est alors réalisée, en continu, dans un lecteur de microplaque, à 37°C, à la longueur d'onde de 340nm. La vitesse initiale de la réaction est obtenue par la mesure de la pente de la tangente à l'origine de la courbe enregistrée. L'activité enzymatique est exprimée en µM d'ADP formé par minute. L'effet des divers produits testés est exprimé en capacité à inhiber l'activité ATPasique selon la codification ci-dessous :
A : IC50 < 1 µM
B : 1 µM < IC50 < 10 µM
C: 10 µM < IC50 < 100 µM

**Tableau de résultats**

| Ex | Structure | Hsp82 ATPase IC50 µM | Ex | Structure | Hsp82 ATPase IC50 µM |
|---|---|---|---|---|---|
| 1 | 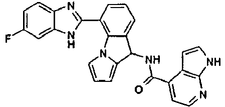 | A | 2 | 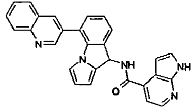 | B |
| 3 | 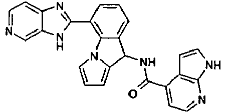 | A | 4 | 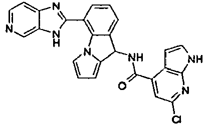 | B |

## Revendications

1. Produits de formule (I) 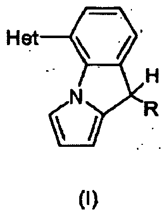 dans lesquels :
Het représente un hétérocycle aromatique ou partiellement insaturé - de type dihydro ou tétrahydro - mono ou bicyclique, de 5 à 11 chaînons, contenant de 1 à 4 hétéroatomes, choisis parmi N, O ou S, éventuellement substitué par un ou plusieurs radicaux R1 ou R'1 identiques ou différents tels que décrits ci-dessous,
R est choisi dans le groupe constitué par X-(A-B)n-CONH2, X-(A-B)n-O-CONH2, X-(A-B)n-NH-CONH2, X-(CH2)m-hétérocycloalkyle, X-(CH2)m-aryle et X-(CH2)m-hétéroaryle avec X représente -O-C(O), -NH-C(O), NH-CS, -NH-CO-CH2-O- ; -NH-CO-CH2-S-CH2-CO-NH- ; -NH-CO-(CH2)2-SO2- ; -NH-CO-CH2-N(CH3)-CO- ; A et B identiques ou différents représentent indépendamment une simple liaison, CH2, CH-alkyle, CH-aralkyle, n = 1,2 et m=0, 1,
R1 et/ou R'1 identiques ou différents sont dans le groupe constitué par H, halogène, CF3, nitro, cyano, alkyle, hydroxy, mercapto, amino, alkylamino, dialkylamino, alkoxy, alkylthio, carboxy libre ou estérifié par un radical alkyle, carboxamide, CO-NH(alkyl), CON(alkyl)2, NH-CO-alkyl, sulfonamide, NH-SO2-alkyl, S(O)2-NHalkyl, S(O2)-N(alkyl)2, tous les radicaux alkyle, alcoxy et alkylthio étant eux-mêmes éventuellement substitués par un ou plusieurs radicaux identiques ou différents choisi(s) parmi halogène, hydroxy, alkoxy, amino, alkylamino, et dialkylamino ;
lesdits produits de formule (I) étant sous toutes les formes tautomères et isomères possibles, racémiques, énantiomères et diastéréoisomères, ainsi que comme sels d'addition avec les acides minéraux et organiques ou avec les bases minérales et organiques des produits de formule (I).

2. Produits de formule (I) tels que définis à la revendication 1 dans lesquels :
Het est choisi dans le groupe constitué par : 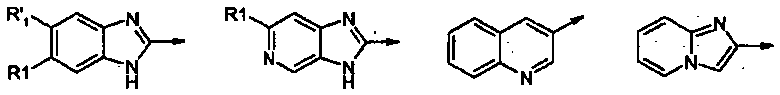 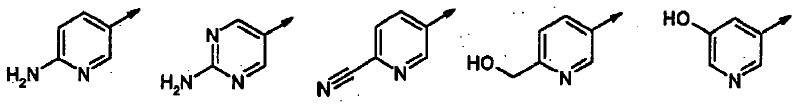 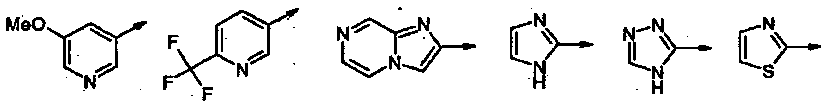
R1 et/ou R'1 identiques ou différents sont dans le groupe constitué par H, halogène, CF3, nitro, cyano, alkyle, hydroxy, mercapto, amino, alkylamino, dialkylamino, alkoxy, alkylthio (méthylthio), carboxy libre ou estérifié par un radical alkyle, carboxamide, CO-NH(alkyl) et CON(alkyl)2, NH-CO-alkyl, sulfonamide, NH-SO2-alkyl, S(O)2-NH(alkyl), S(O)2-N(alkyl)2, tous les radicaux alkyle, alcoxy et alkylthio étant eux-mêmes éventuellement substitués par un ou plusieurs radicaux identiques ou différents choisi(s) parmi halogène, hydroxy, alkoxy, amino, alkylamino et dialkylamino ;
le substituant R desdits produits de formule (I) étant choisi parmi les valeurs définies à l'une quelconque des autres revendications,
lesdits produits de formule (I) étant sous toutes les formes tautomères et isomères possibles, racémiques, énantiomères et diastéréoisomères, ainsi que comme sels d'addition avec les acides minéraux et organiques ou avec les bases minérales et organiques des produits de formule (I).

3. Produits de formule (I) tels que définis à l'une quelconque des autres revendications dans lesquels Het représente un hétérocycle tel que défini à la revendication 1 ou 2 éventuellement substitué par un ou plusieurs radicaux R1 ou R'1 ou R"1 identiques ou différents tels que définis à l'une quelconque des autres revendications,
et R est choisi dans le groupe constitué par : 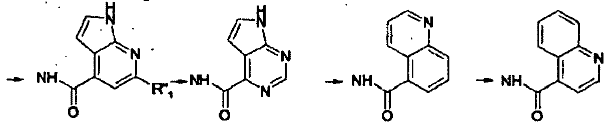 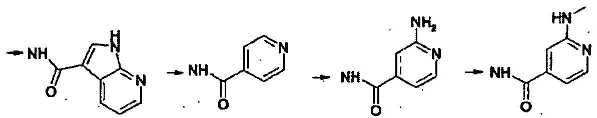 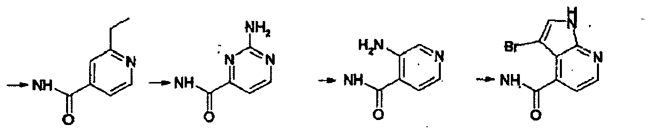 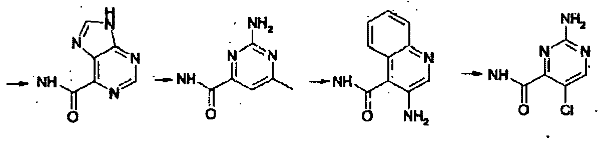 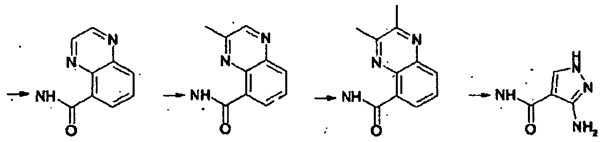 lesdits produits de formule (I) étant sous toutes les formes tautomères et isomères possibles, racémiques, énantiomères et diastéréoisoméres, ainsi que comme sels d'addition avec les acides minéraux et organiques ou avec les bases minérales et organiques des produits de formule (I).

4. Produits de formule (I) tels que définis à l'une quelconque des autres revendications dans lesquels :
Het est choisi dans le groupe constitué par : 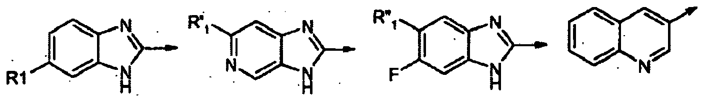 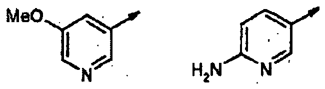 R est choisi dans le groupe constitué par : 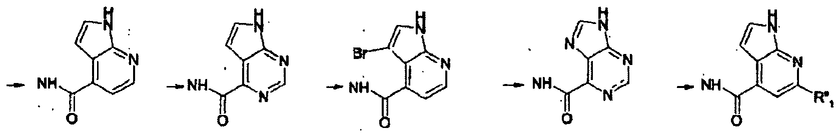
R1 est dans le groupe constitué par H, F, Cl, Br, CF3, NO2, CN, CH3, OH, OCH3, OCF3, CO2Me, CONH2, CONHMe, CONH-(CH2)3-OMe, CONH-(CH2)3-N(Me)2, NHC(O)Me, SO2NH2, SO2N(Me)2 ;
R'1 est dans le groupe constitué par H, CONH2, CONHMe et OMe ;
R"1 est dans le groupe constitué par F, Cl, OH, OMe, CN, O-(CH2)3-OMe et O-(CH2)3-N(Me)2 ;
lesdits produits de formule (I) étant sous toutes les formes tautomères et isomères possibles, racémiques, énantiomères et diastéréoisoméres, ainsi que comme sels d'addition avec les acides minéraux et organiques ou avec les bases minérales et organiques des produits de formule (I).

5. Produits de formule (I) tels que définis à l'une quelconque des autres revendications dans lesquels:
Het est choisi dans le groupe constitué par : 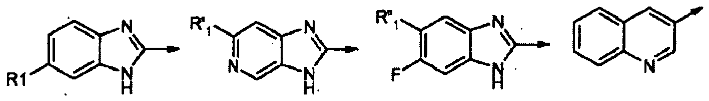 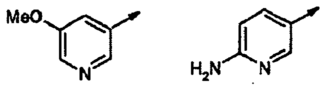
R est choisi dans le groupe constitué par : 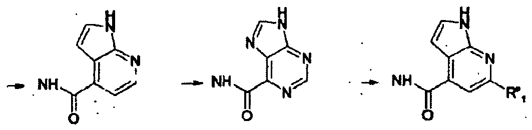
R1 est dans le groupe constitué par H, F, Cl, Br, CF3, NO2, CN, CH3, OH, OCH3, OCF3, CO2Me, CONH2, CONHMe, CONH-(CH2)3-OMe, CONH-(CH2)3-N(Me)2, NHC(O)Me, SO2NH2, SO2N(Me)2,
R'1 est dans le groupe constitué par H, CONH2, CONHMe et OMe,
R"1 est dans le groupe constitué par F, Cl, OH, OMe, CN, O-(CH2)3-OMe et O-(CH2)3-N(Me)2
lesdits produits de formule (I) étant sous toutes les formes isomères possibles racémiques, énantiomères et diastéréo-isomères, ainsi que les sels d'addition avec les acides minéraux et organiques ou avec les bases minérales et organiques.

6. Produits de formule (I) tels que définis à l'une quelconque des autres revendications dans lesquels :
Het est choisi dans le groupe constituée par : 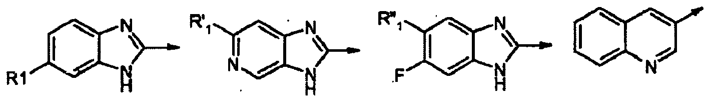 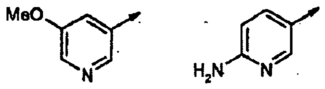 avec :
R1 représente H, F, Cl, Br, CF3, NO2, CN, CH3, OH, OCH3, OCF3, CO2Me, CONH2, CONHMe, CONH-(CH2)3-OMe, CONH-(CH2)3-N(Me)2, NHC(O)Me, SO2NH2, SO2N(Me)2;
R'1 représente H, CONH2, CONHMe et OMe;
R"1 représente F, Cl, OH, OMe; CN, O-(CH2)3-OMe et O-(CH2)3-N(Me)2 ;
et R est choisi dans le groupe constitué par : 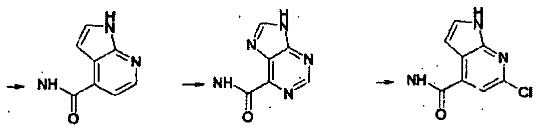 ainsi que leurs prodrogues, lesdits produits de formule (I) étant sous toutes les formes isomères possibles tautomères, racémiques, énantiomères et diastéréo-isoméres, ainsi que les sels d'addition avec les acides minéraux et organiques ou avec les bases minérales et organiques desdits produits de formule (I).

7. Produits de formule. (I) tels que définis à l'une quelconque des autres revendications dont les noms suivent :
- le [5-(6-fluoro-1H-benzimidazol-2-yl)-9H-pyrrolo[1,2-a]indol-9-yl]amide de l'acide 1H-pyrrolo[2,3-b]pyridine-4-carboxylique.
- le (5-quinoléin-3-yl-9H-pyrrolo[1,2-a]indol-9-yl)-amide de l'acide 1H-pyrrolo[2,3-b]pyridine-4-carboxylique.
- le [5-(3H-imidazo[4,5-c]pyridin-2-yl)-9H-pyrrolo[1,2-a]indol-9-yl]-amide de l'acide [1H-pyrrolo[2,3-bjpyridine-4-carboxylique.
- le [5-(3H-imidazo[4,5-c]pyridin-2-yl)-9H-pyrrolo[1,2-a]indol-9-yl]-amide de l'acide 6-chloro-1H-pyrrolo[2,3-b]pyridine-4-carboxylique.
ainsi que les sels d'addition avec les acides minéraux et organiques ou avec les bases minérales et organiques desdits produits de formule (I).

8. Procédé de préparation des produits de formule (1) tels que définis dans les revendications précédentes, **caractérisé par** le schéma (I) ci après:
selon lequel les produits de formule générale (I) peuvent être préparés à partir d'un dérivé de formule générale (II), soit par l'intermédiaire d'un dérivé de formule générale (III) (méthode A) soit par l'intermédiaire d'un dérivé de formule générale (IV) (méthode B) selon le schéma général (1) ci-dessous : 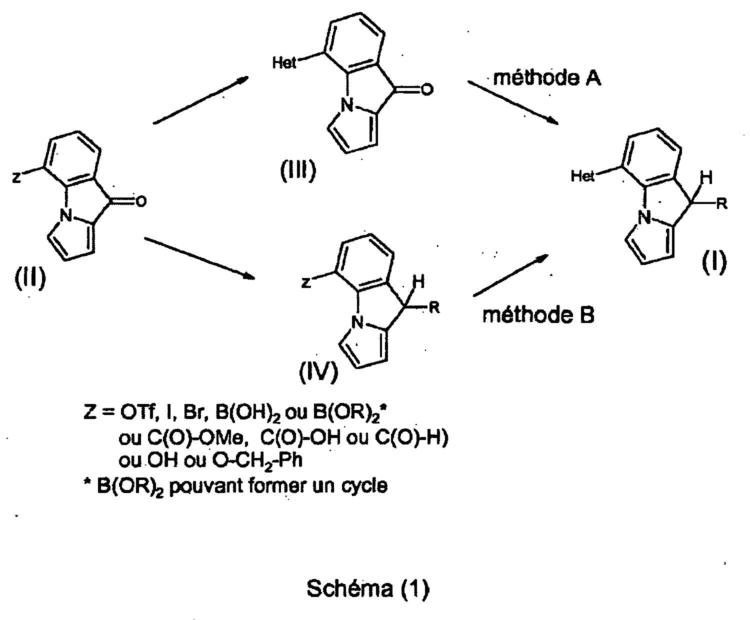 dans lequel les substituants Het et R ont les significations indiquées pour les produits de formule (I) tels que définis dans les revendications précédentes et z a la signification indiquée ci-dessus dans le schéma (1),

9. A titre de produits industriels nouveaux, les intermédiaires de synthèse de formules (II), (III) et (IV) : 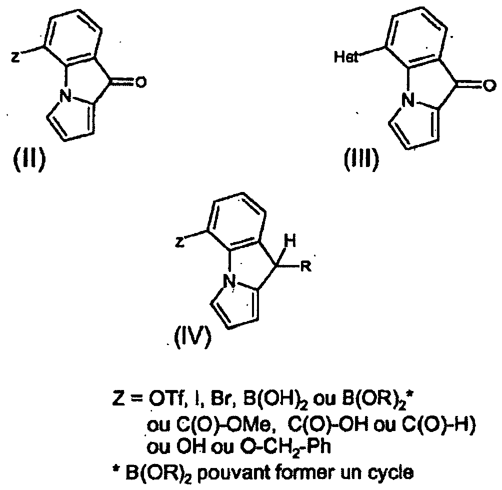 composés de formules (II), (III) et (IV) dans lesquels les substituants Het et R ont les significations indiquées pour les produits de formule (I) à l'une quelconque des autres revendications et z a la signification indiquée ci-dessus, à l'exclusion du composé de formule (II) dans lequel z représente un atome d'iode.

10. A titre de médicaments, les produits de formule (I) telle que définie aux revendications 1 à 7), lesdits produits de formule (I) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (I).

11. A titre de médicaments, les produits de formule (I) telle que définie à la revendication 6), lesdits produits de formule (I) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (I).

12. A titre de médicaments, les produits de formule (I) telle que définie à la revendication 7), lesdits produits de formule (I) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (I).

13. Les compositions pharmaceutiques contenant à titre de principe actif, l'un au moins des médicaments tels que définis aux revendications 10 à 12.

14. Compositions pharmaceutiques telles que définies aux revendications précédentes contenant en plus, des principes actifs d'autres médicaments de chimiothérapie contre le cancer.

15. Compositions pharmaceutiques selon l'une quelconque des revendications précédentes **caractérisées en ce qu'**elles sont utilisées comme médicaments, en particulier pour la chimiothérapie de cancers.

16. Utilisation de produits de formule (I) tels que définis à l'une quelconque des revendications précédentes ou de sels pharmaceutiquement acceptables desdits produits de formule (I) pour la préparation de médicaments destinés à inhiber l'activité de protéines chaperones et notamment d'Hsp90.

17. Utilisation de produits de formule (I) telle que définie à l'une quelconque des revendications précédentes ou de sels pharmaceutiquement acceptables desdits produits de formule (I) pour la préparation d'un médicament destiné à prévenir ou traiter une maladie **caractérisée par** le dérèglement de l'activité d'une protéine chaperone de type Hsp90.

18. Utilisation de produits de formule (I) telle que définie à l'une quelconque des revendications précédentes ou de sels pharmaceutiquement acceptables desdits produits de formule (I) pour la préparation d'un médicament destiné à prévenir ou traiter une maladie appartenant au groupe suivant: les maladies neurodégénératives telles que la maladie de Huntington, la maladie de Parkinson, l'ischémie cérébrale focale, la maladie d'Alzheimer, la sclérose en plaques et la sclérose latérale amyotrophique, la malaria, filarioses de Brugia et de Bancroft, la toxoplasmose, les mycoses résistantes aux traitements, l'hépatite B, l'hépatite C, le virus de l'Herpes, la dengue (ou grippe tropicale), l'atrophie musculaire spinale et bulbaire, désordres de la prolifération de cellules mésangiales, thromboses, rétinopathies, psoriasis, dégénération musculaire, maladies en oncologie, cancers.

19. Utilisation de produits de formule (I) telle que définie à l'une quelconque des revendications précédentes ou de sels pharmaceutiquement acceptables desdits produits de formule (I) pour la préparation d'un médicament destiné à traiter des cancers.

20. Utilisation de produits de formule (I) selon la revendication précédente dans laquelle la maladie à traiter est un cancer de tumeurs solides ou liquides.

21. Utilisation de produits de formule (I) selon la revendication précédente dans laquelle la maladie à traiter est un cancer résistant aux agents cytotoxiques.

22. Utilisation de produits de formule (I) telle que définie à l'une quelconque des revendications précédentes ou de sels pharmaceutiquement acceptables desdits produits de formule (I) pour la préparation d'un médicament destiné à traiter des cancers parmi lesquels les cancers du poumon, du sein et de l'ovaire, les glioblastomes, les leucémies myéloides chroniques, les leucémies lymphoblastiques aigûes, les cancers de la prostate, du pancréas et du colon, les mélanomes métastatiques, les tumeurs de la thyroïde et les carcinomes rénaux..

23. Utilisation de produits de formule (I) telle que définie à l'une quelconque des revendications précédentes ou de sels pharmaceutiquement acceptables desdits produits de formule (I) pour la préparation d'un médicament destiné à la chimiothérapie de cancers.

24. Utilisation de produits de formule (I) telle que définie à l'une quelconque des revendications précédentes ou de sels pharmaceutiquement acceptables desdits produits de formule (I) pour la préparation de médicaments destinés à la chimiothérapie de cancers utilisés seuls ou en association.

25. Utilisation de produits de formule (I) telle que définie à l'une quelconque des revendications précédentes ou de sels pharmaceutiquement acceptables desdits produits de formule (I) pour la préparation de médicaments destinés à être utilisés seuls ou en association avec chimiothérapie ou radiothérapie ou alternativement en association avec d'autres agents thérapeutiques.

26. Utilisation de produits de formule (I) selon la revendication précédente dans laquelle les agents thérapeutiques peuvent être des agents anti-tumoraux utilisés communément.

27. Produits de formule (I) tels que définis à l'une quelconque des revendications précédentes comme inhibiteurs de HSP90, lesdits produits de formule (I) étant sous toutes les formes tautomères et/ou isomères possibles racémiques, énantiomères et diastéréo-isomères, ainsi que les sels d'addition avec les acides minéraux et organiques ou avec les bases minérales et organiques pharmaceutiquement acceptables desdits produits de formule (I).

## Claims

1. Products of formula (I) 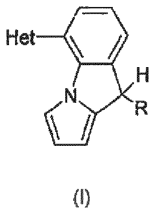 in which:
Het represents a monocyclic or bicyclic, aromatic or partially unsaturated heterocycle - of dihydro or tetrahydro type -, with from 5 to 11 ring members, containing from 1 to 4 heteroatoms chosen from N, O or S, optionally substituted with one or more radicals R1 or R'1, which may be identical or different, as described below,
R is chosen from the group constituted of X-(A-B)ₙ-CONH₂, X-(A-B)ₙ-O-CONH₂, X- (A-B)ₙ-NH-CONH₂, X- (CH₂) ₘ-heterocycloalkyl, X-(CH₂)ₘ-aryl and X-(CH₂)ₘ-heteroaryl where X represents -O-C(O), -NH-C(O), NH-CS, -NH-CO-CH₂-O-; -NH-CO-CH₂-S-CH₂-CO-NH-; -NH-CO-(CH₂)₂-SO₂-; -NH-CO-CH₂-N(CH₃)-CO-; A and B, which may be identical or different, independently represent a single bond, CH₂, CH-alkyl or CH-aralkyl, n = 1 or 2 and m = 0 or 1;
R1 and/or R'1, which may be identical or different, are in the group constituted of H, halogen, CF₃, nitro, cyano, alkyl, hydroxyl, mercapto, amino, alkylamino, dialkylamino, alkoxy, alkylthio, carboxyl in free form or esterified with an alkyl radical, carboxamide, CO-NH(alkyl), CON(alkyl)₂, NH-CO-alkyl, sulphonamide, NH-SO₂-alkyl, S(O)₂-NHalkyl and S(O₂) -N(alkyl)₂, all the alkyl, alkoxy and alkylthio radicals being themselves optionally substituted with one or more radicals, which may be identical or different, chosen from halogen, hydroxyl, alkoxy, amino, alkylamino and dialkylamino;
said products of formula (I) being in all the possible tautomeric and isomeric forms: racemic, enantiomeric and diastereoisomeric, and also as addition salts with inorganic and organic acids or with inorganic and organic bases of the products of formula (I).

2. Products of formula (I) as defined in Claim 1, in which:
Het is chosen from the group constituted of: 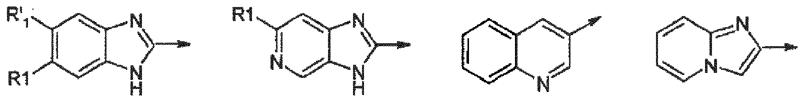 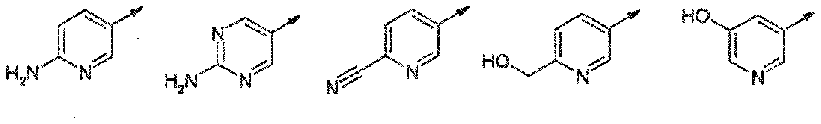 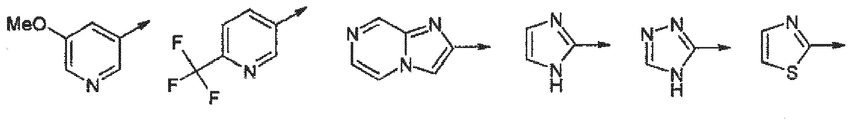
R1 and/or R'1, which may be identical or different, are in the group constituted of H, halogen, CF₃, nitro, cyano, alkyl, hydroxyl, mercapto, amino, alkylamino, dialkylamino, alkoxy, alkylthio (methylthio), carboxyl in free form or esterified with an alkyl radical, carboxamide, CO-NH(alkyl) an CON(alkyl)₂, NH-CO-alkyl, sulphonamide, NH-SO₂-alkyl, S(O)₂-NHalkyl and S(O₂)-N(alkyl)₂, all the alkyl, alkoxy and alkylthio radicals being themselves optionally substituted with one or more radicals, which may be identical or different, chosen from halogen, hydroxyl, alkoxy, amino, alkylamino and dialkylamino;
the substituent R of said products of formula (I) being chosen from the values defined in any one of the other claims,
said products of formula (I) being in all the possible tautomeric and isomeric forms: racemic, enantiomeric and diastereoisomeric, and also as addition salts with inorganic and organic acids or with inorganic and organic bases of the products of formula (I).

3. Products of formula (I) as defined in any one of the other claims, in which Het represents a heterocycle as defined in Claim 1 or 2, optionally substituted with one or more radicals R1 or R'1 or R"1, which may be identical or different, as defined in any one of the other claims,
and R is chosen from the group constituted of: 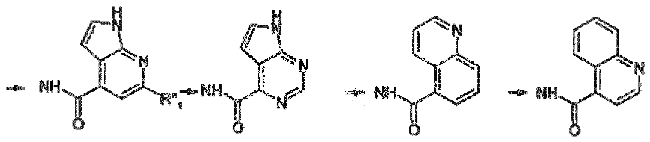 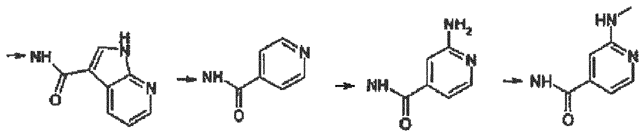 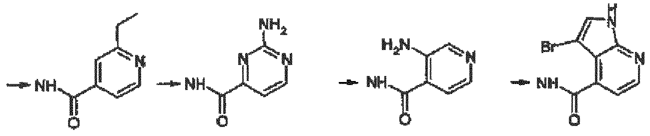 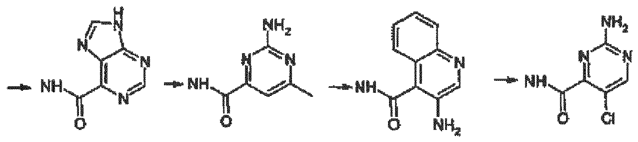 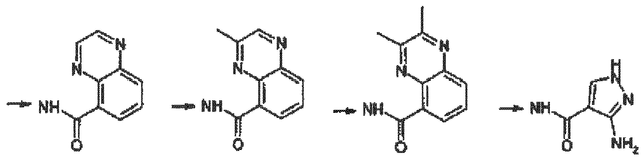 said products of formula (I) being in all the possible tautomeric and isomeric forms: racemic, enantiomeric and diastereoisomeric, and also as addition salts with inorganic and Organic acids or with inorganic and organic bases of the products of formula (I).

4. Products of formula (I) as defined in any one of the other claims, in which:
Het is chosen from the group constituted of: 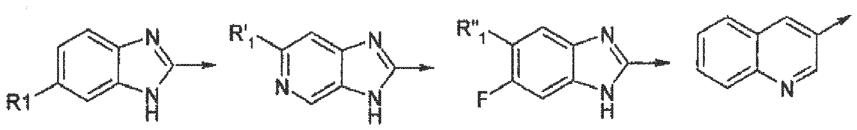 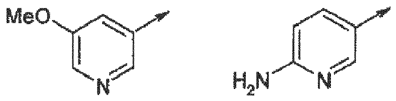
R is chosen from the group constituted of: 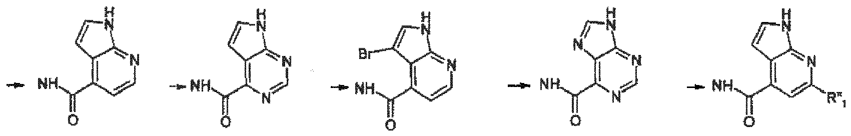
R1 is in the group constituted of H, F, Cl, Br, CF₃, NO₂, CN, CH₃, OH, OCH₃, OCF₃, CO₂Me, CONH₂, CONHMe, CONH-(CH₂)₃-OMe, CONH-(CH2)₃-N(Me)₂, NHC(O) Me, SO₂NH₂ and SO₂N(Me)₂;
R'1 is in the group constituted of H, CONH₂, CONHMe and OMe;
R"1 is in the group constituted of F, Cl, OH, OMe, CN, O-(CH₂)₃-OMe and O- (CH₂)₃-N (Me)₂;
said products of formula (I) being in all the possible tautomeric and isomeric forms: racemic, enantiomeric and diastereoisomeric, and also as addition salts with inorganic and organic acids or with inorganic and organic bases of the products of formula (I).

5. Products of formula (I) as defined any one of the other claims, in which:
Het is chosen from the group constituted of: 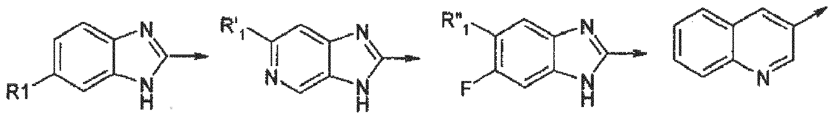 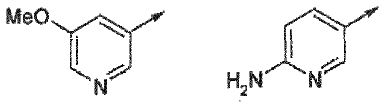
R is chosen from the group constituted of: 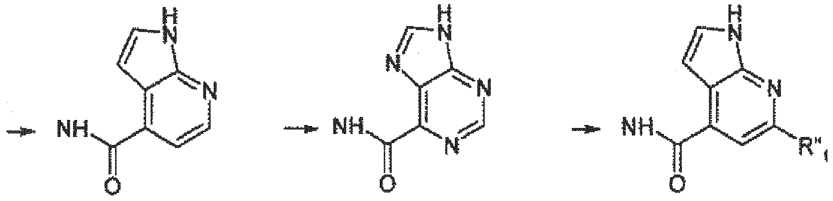
R1 is in the group constituted of H, F, Cl, Br, CF₃, NO₂, CN, CH₃, OH, OCH₃, OCF₃, CO₂Me, CONH₂, CONHMe, CONH-(CH₂)₃ - OMe, CONH - (CH₂)₃ - N(Me)₂, NHC (O) Me, SO₂NH₂ and SO₂N (Me)₂,
R'1 is in the group constituted of H, CONH₂, CONHMe and OMe,
R" 1 is in the group constituted of F, Cl, OH, OMe, CN, O-(CH₂)₃ - OMe and O- (CH₂)₃ - N(Me)₂
said products of formula (I) being in all the possible isomeric forms: racemic, enantiomeric and diastereoisomeric; and also the addition salts with inorganic and organic acids or with inorganic and organic bases.

6. Products of formula (I) as defined in any one of the other claims, in which:
Het is chosen from the group constituted of: 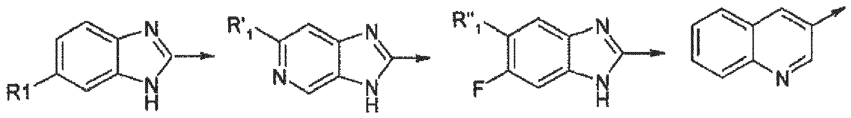 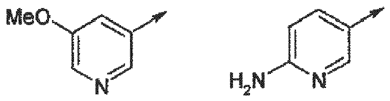 with:
R1 represents H, F, Cl, Br, CF₃, NO₂, CN, CH₃, OH, OCH₃, OCF₃, CO₂Me, CONH₂, CONHMe, CONH-(CH₂)₃-OMe, CONH-(CH₂)₃-N(Me)_{2,} NHC(O)Me, SO₂NH₂ or SO₂N(Me)₂;
R'1 represents H, CONH₂, CONHMe or OMe;
R"1 represents F, Cl, OH, OMe, CN, 0-(CH₂)₃-OMe or O- (CH₂)₃ -N(Me)₂;
and R is chosen from the group constituted of: 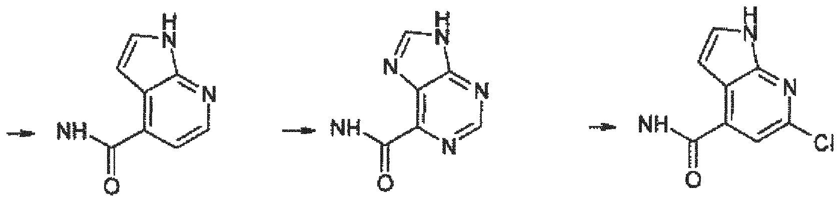
an also the prodrugs thereof, said products of formula (I) being in all the possible isomeric forms: tautomeric, racemic, enantiomeric and diastereoisomeric, and also the addition salts with inorganic and organic acids or with inorganic and organic bases of said products of formula (I).

7. Products of formula (I) as defined in any one of the other claims, the names of which are given below:
- 1H-pyrrolo[2,3-b]pyridine-4-carboxylic acid [5-(6-fluoro-1H-benzimidazol-2-yl)-9H-pyrrolo[1,2-a]indol-9-yl]amide,
- 1H-pyrrolo[2,3-b]pyridine-4-carboxylic acid (5-quinolin-3-yl-9H-pyrrolo[1,2-a]indol-9-yl)amide,
- 1H-pyrrolo[2,3-b]pyridine-4-carboxylic acid [5-(3H-imidazo[4,5-c]pyridin-2-yl)-9H-pyrrolo[1,2-a]indol-9-yl]amide,
- 6-chloro-1H-pyrrolo[2,3-b]pyridine-4-carboxylic acid [5-(3H-imidazo[4,5-c]pyridin-2-yl)-9H-pyrrolo[1,2-a] indol-9-yl]amide,
and also the addition salts with inorganic and organic acids or with inorganic and organic bases of said products of formula (I).

8. Method for preparing the products of formula (I) as defined in the preceding claims, **characterized by** scheme (1) hereinafter:
according to which the products of general formula (I) can be prepared from a derivative of general formula (II), either by using a derivative of general formula (III) (method A) or by using a derivative of general formula (IV) (method B) according to general scheme (1) below: 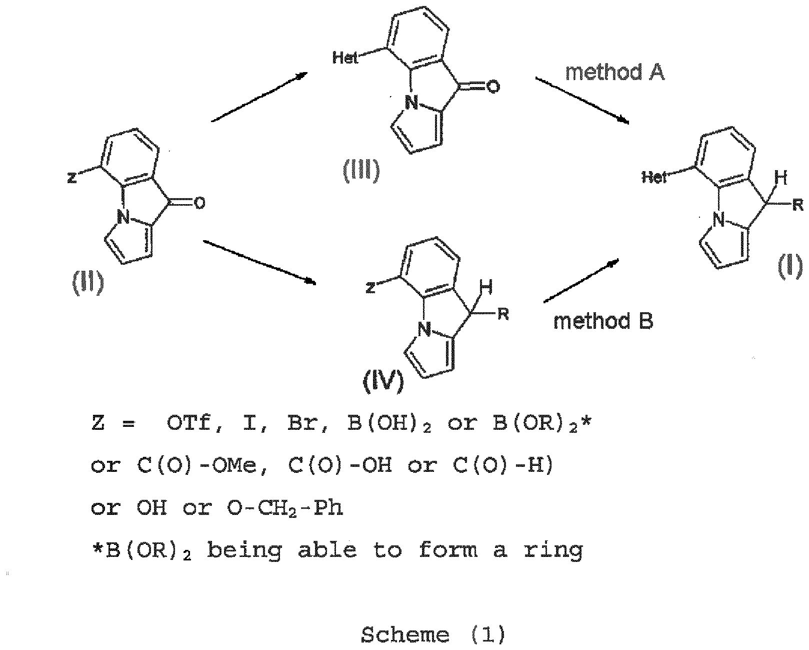 in which the substituents Heat and R have the meanings indicated for the products of formula (I) as defined in the preceding claims and z has the meaning indicated above in scheme (1).

9. As novel industrial products, the synthesis intermediates of formulae (II), (III) and (IV) : 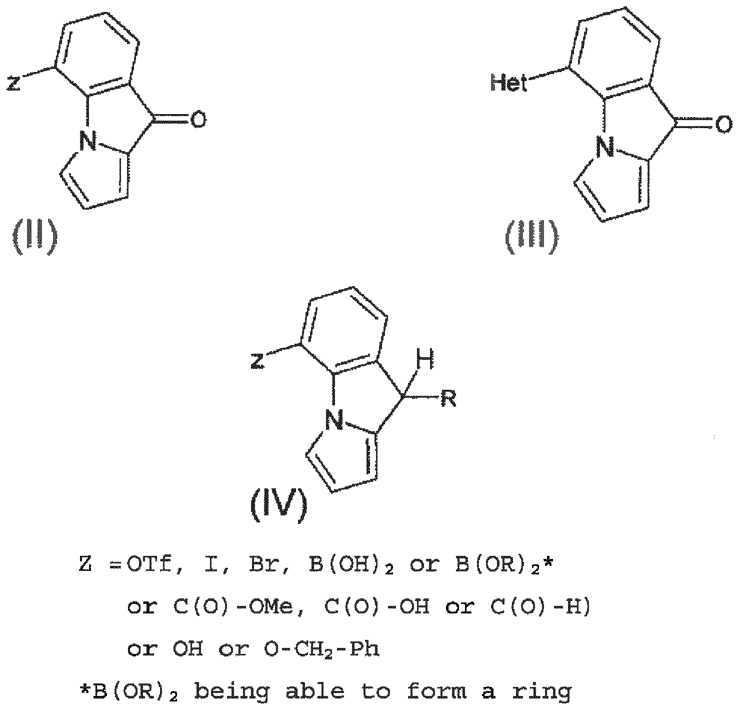 in which compounds of formulae (II), (III) and (IV), the substituents Heat and R have the meanings indicated for the products of formula (I) in any one of the other claims and z has the meaning indicated above with the exception of the compound of formula (II) in which z represents an iodine atom.

10. As medicaments, the products of formula (I) as defined in Claims 1 to 7, said products of formula (I) being in all the possible isomeric forms: racemic, enantiomeric an diastereoisomeric, and also the pharmaceutically acceptable addition salts with inorganic an organic acids or with inorganic and organic bases of said products of formula (I).

11. As medicaments, the products of formula (I) as define in Claim 6, said products of formula (I) being in all the possible isomeric forms: racemic, enantiomeric and diastereoisomeric, and also the pharmaceutically acceptable addition salts with inorganic and organic acids or with inorganic and organic bases of said products of formula (I).

12. As medicaments, the products of formula (I) as defined in Claim 7, said products of formula (I) being in all the possible isomeric forms: racemic, enantiomeric and diastereoisomeric, and also the pharmaceutically acceptable addition salts with inorganic and organic acids or with inorganic and organic bases of said products of formula (I).

13. Pharmaceutical compositions containing, as active ingredient, at least one of the medicaments as defined in Claims 10 to 12.

14. Pharmaceutical compositions as defined in the preceding claims, additionally containing active ingredients of other medicaments for anticancer chemotherapy.

15. Pharmaceutical compositions according to any one of the preceding claims, **characterized in that** they are used as medicaments, in particular for cancer chemotherapy.

16. Use of products of formula (I) as defined in any one of the preceding claims or pharmaceutically acceptable salts of said products of formula (I), for the preparation of medicaments for inhibiting the activity of chaperone proteins, and in particular of Hsp90.

17. Use of products of formula (I) as defined in any one of the preceding claims or of pharmaceutically acceptable salts of said products of formula (I), for the preparation of a medicament for preventing or treating a disease **characterized by** the disturbance of the activity of a chaperone protein of Hsp90 type.

18. Use of products of formula (I) as defined in any one of the preceding claims or of pharmaceutically acceptable salts of said products of formula (I), for the preparation of a medicament for preventing or treating a disease belonging to the following group: neurodegenerative diseases such as Huntington's disease, Parkinson's disease, focal cerebral ischaemia, Alzheimer's disease, multiple sclerosis and amyotrophic lateral sclerosis, malaria, Brugia filariasis, Bancroft's filariasis, toxoplasmosis, treatment-resistant mycoses, hepatitis B, hepatitis C, the Herpes virus, dengue (or tropical flu), spinal and bulbar muscular atrophy, mesangial cell proliferation disorders, thromboses, retinopathies, psoriasis, muscle degeneration, diseases in oncology and cancers.

19. Use of products of formula (I) as defined in any one of the preceding claims or of pharmaceutically acceptable salts of said products of formula (I), for the preparation of a medicament for treating cancers.

20. Use of products of formula (I) according to the preceding claim, in which the disease to be treated is a cancer of solid or liquid tumours.

21. Use of products of formula (I) according to the preceding claim, in which the disease to be treated is a cancer resistant to cytotoxic agents.

22. Use of products of formula (I) as defined in any one of the preceding claims, or of pharmaceutically acceptable salts of said products of formula (I), for the preparation of a medicament for treating cancers, among which are lung cancer, breast cancer and ovarian cancer, glioblastomas, chronic myeloid leukaemias, acute lymphoblastic leukaemias, prostate cancer, pancreatic cancer and colon cancer, metastatic melanomas, thyroid tumours and renal carcinomas.

23. Use of products of formula (I) as defined in any one of the preceding claims or of pharmaceutically acceptable salts of said products of formula (I), for the preparation of a medicament for use in cancer chemotherapy.

24. Use of products of formula (I) as defined in any one of the preceding claims or of pharmaceutically acceptable salts of said products of formula (I), for the preparation of medicaments for use in cancer chemotherapy, which are used alone or in combination.

25. Use of products of formula (I) as defined in any one of the preceding claims or of pharmaceutically acceptable salts of said products of formula (I), for the preparation of medicaments intended to be used alone or in combination with chemotherapy or radiotherapy, or alternatively in combination with other therapeutic agents.

26. Use of products of formula (I) according to the preceding claim, in which the therapeutic agents can be commonly used anti-tumour agents.

27. Products of formula (I) as defined in any one of the preceding claims, as Hsp90 inhibitors, said products of formula (I) being in all the possible tautomeric and/or isomeric forms: racemic, enantiomeric and diastereoisomeric, and also the pharmaceutically acceptable addition salts with inorganic and organic acids or with inorganic and organic bases of said products of formula (I).

## Patentansprüche

1. Produkte der Formel (I) 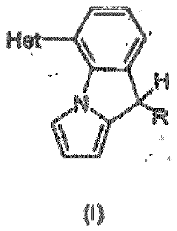 worin:
Het für einen 5- bis 11-gliedrigen mono- oder bicyclischen Heterocyclus, der aromatisch oder teilweise ungesättigt - vom Dihydro- oder Tetrahydro-Typ - ist, 1 bis 4 aus N, O oder S ausgewählte Heteroatome enthält und gegebenenfalls durch einen oder mehrere gleiche oder verschiedene Reste R1 oder R'1 gemäß nachstehender Beschreibung substituiert ist,
R ausgewählt ist aus der Gruppe bestehend aus X-(A-B)ₙ-CONH₂, X- (A-B)ₙ-O-CONH₂, X- (A-B)ₙ-NH-CONH₂, X-(CH₂)ₘ-Heterocycloalkyl, X-(CH₂)ₘ-Aryl und X-(CH₂)ₘ-Heteroaryl, wobei X für -O-C(O), -NH-C(O), NH-CS, -NH-CO-CH₂-O-; -NH-CO-CH₂-S-CH₂-CO-NH-; -NH-CO-(CH₂)₂-SO₂- oder -NH-CO-CH₂-N(CH₃)-CO- steht; A und gleich oder verschieden sind und unabhängig voneinander für eine Einfachbindung, CH₂, CH-Alkyl oder CH-Aralkyl stehen, n = 1 oder 2 und m = 0 oder 1;
R1 und/oder R'1 gleich oder verschieden sind und aus der Gruppe bestehend aus aus H, Halogen, CF₃, Nitro, Cyano, Alkyl, Hydroxy, Mercapto, Amino, Alkylamino, Dialkylamino, Alkoxy, Alkylthio, freiem Carboxy oder mit einem Alkylrest verestertem Carboxy, Carboxamid, CO-NH(Alkyl), CON(Alkyl)₂, NH-CO-Alkyl, Sulfonamid, NH-SO₂-Alkyl, S(O)₂-NH-Alkyl und S(O₂)-N(alkyl)₂ stammen, wobei alle Alkyl-, Alkoxy- und Alkylthioreste gegebenenfalls selbst durch einen oder mehrere gleiche oder verschiedene Reste, die aus Halogen, Hydroxy, Alkoxy, Amino, Alkylamino und Dialkylamino ausgewählt sind, substituiert sind;
wobei die Produkte der Formel (I) in allen möglichen tautomeren und racemischen, enantiomeren oder diastereoisomeren isomeren Formen sowie als Additionssalze der Produkte der Formel (I) mit anorganischen und organischen Säuren oder mit anorganischem und organischen Basen vorliegen.

2. Produkte der Formel (I) nach Anspruch 1, worin:
Het ausgewählt ist aus der Gruppe bestehend aus: 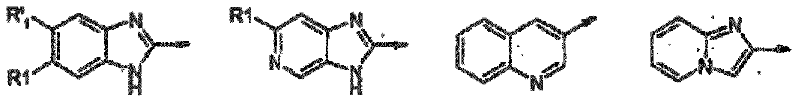 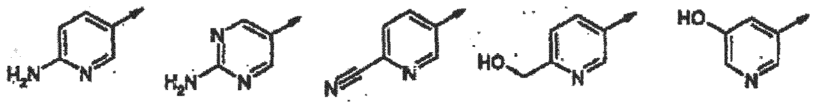 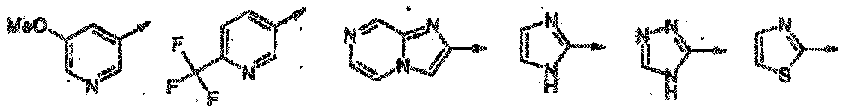
R1 und/oder R'1 gleich oder verschieden sind und aus der Gruppe bestehend aus aus H, Halogen, CF₃, Nitro, Cyano, Alkyl, Hydroxy, Mercapto, Amino, Alkylamino, Dialkylamino, Alkoxy, Alkylthio (Methylthio), freiem Carboxy oder mit einem Alkylrest verestertem Carboxy, Carboxamid, CO-NH(Alkyl) und CON(Alkyl)₂, NH-CO-Alkyl, Sulfonamid, NH-SO₂-Alkyl, S(O)₂-NH-Alkyl und S(O)₂-N(alkyl)₂ stammen, wobei alle Alkyl-, Alkoxy- und lkylthioreste gegebenenfalls selbst durch einen oder mehrere gleiche oder verschiedene Reste, die aus Halogen, Hydroxy, Alkaxy, Amino, Alkylamino und Dialkylamino ausgewählt sind, substituiert sind;
wobei der Substituent R der Produkte der Formel (I) aus den in einem der anderen Ansprüche definierten Werten ausgewählt ist,
wobei die Produkte der Formel (I) in allen möglichen tautomeren und racemischen, enantiomeren oder diastereoisomeren isomeren Formen sowie als Additionssalze der Produkte der Formel (I) mit anorganischen und organischen Säuren oder mit anorganischen und organischen Basen vorliegen.

3. Produkte der Formel (I) nach einem der anderen Ansprüche, worin Het für einen Heterocyclus gemäß Anspruch 1 oder 2 steht, der gegebenenfalls durch einen oder mehrere gleiche oder verschiedene Reste R1 oder R'1 oder R''1 gemäß einem der anderen Ansprüche substituiert ist,
und R ausgewählt ist aus der Gruppe bestehend aus: 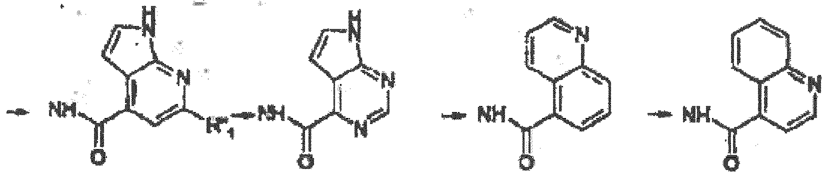 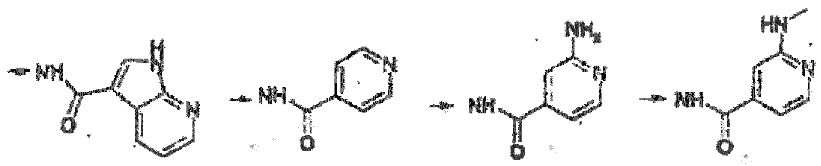 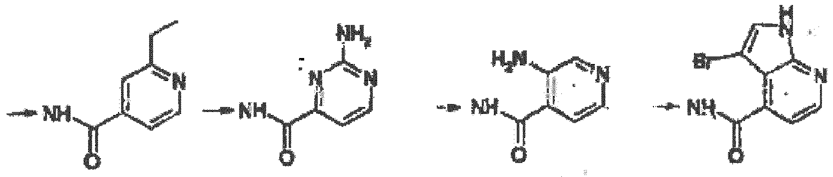 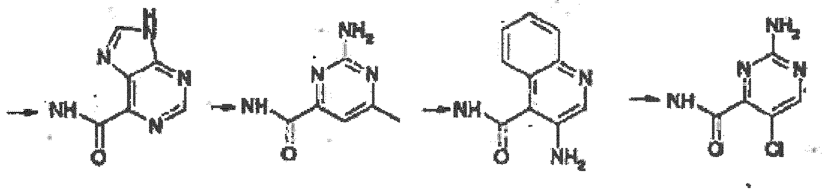 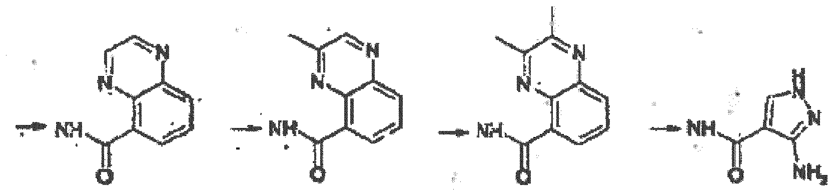 wobei die Produkte der Formel (I) in allen möglichen tautomeren und racemischen, enantiomeren oder diastereoisomeren isomeren Formen sowie als Additionssalze der Produkte der Formel (I) mit anorganischen und organischen Säuren oder mit anorganischen und organischen Basen vorliegen.

4. Produkte der Formel (I) nach einem der anderen Ansprüche, worin
Het ausgewählt ist aus der Gruppe bestehend aus: 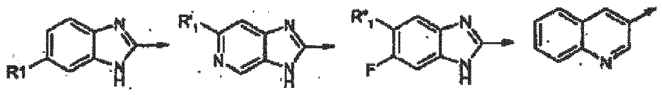 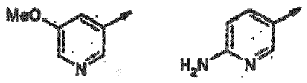
R ausgewählt ist aus der Gruppe bestehend aus: 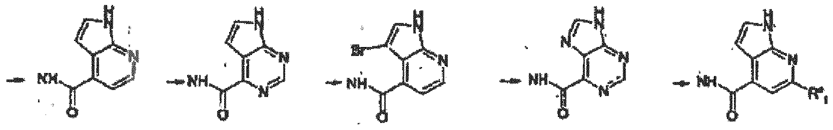
R1 aus der Gruppe bestehend aus H, F, Cl, Br, CF₃, NO₂, CN, CH₃, OH, OCH₃, OCF₃, CO₂Me, CONH₂, CONHMe, CONH-(CH₂)₃-OMe, CONH-(CH₂)₃-N(Me)₂, NHC(O)Me, SO₂NH₂ und SO₂N(Me)2 stammt;
R'1 aus der Gruppe bestehend aus H, CONH₂, CONHMe und OMe stammt,
R''1 aus der Gruppe bestehend aus F, Cl, OH, OMe, CN, O-(CH₂)₃-OMe und O-(CH₂)₃-N(Me)₂ stammt;
wobei die Produkte der Formel (I) in allen möglichen tautomeren und racemische, enantiomeren oder diastereoisomeren isomeren Formen sowie als Additionssalze der Produkte der Formeln (I) mit anorganischen und organischem Säuren oder mit anorganischen und organischem Basen vorliegen.

5. Produkte der Formel (I) nach einem der anderen Anspruche, worin
Het ausgewählt ist aus der Gruppe bestehend aus: 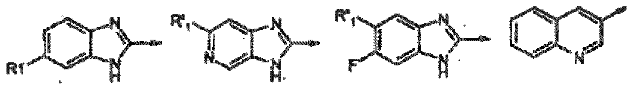 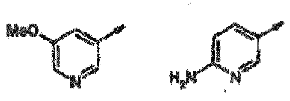
Ausgewählt ist aus der Gruppe bestehend aus: 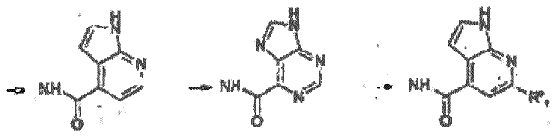
R1 aus der Gruppe bestehend aus H, F, Cl, Br, CF₃, NO₂, CN, CH₃, OH, OCH₃, OCF₃, CO₂Me, CONH₂, CONHMe, CONH-(CH2)3-OMe, CONH-(CH₂)₃-N(Me)₂, NHC(O)Me, SO₂NH₂ und SO₂N(Me)₂ stammt;
R'1 aus der Gruppe bestehend aus H, CONH₂, CONHMe und OMe stammt,
R''1 aus Gruppe bestehend aus F, Cl, OH, OMe, CN, O-(CH₂)₃-OMe und O-(CH₂)₃-N(Me)₂ stammt;
wobei die Produkte der Formel (I) in allen möglichen racemischen, enantiomeren oder diastereoisomeren isomeren Formen vorlieben, sowie Additionssalze mit anorganischem und organischen Säuren oder mit anorganischen und organischen Basen.

6. Produkte der Formel (I) nach einem der anderen Ansprüche, worin
Heft ausgewählt ist aus der Gruppe bestehend aus: 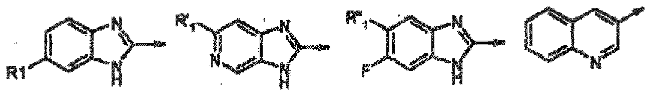 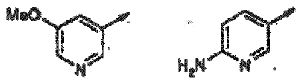 wobei:
R1 für H, F, Cl, Br, CF₃, NO₂, CN, CH₃, OH, OCH₃, OCF₃, CO₂Me, CONH₂, CONHMe, CONH-(CH₂)₃-OMe, CONH-(CH₂)₃-N(Me)2_{,} NHC(O)Me, SO₂NH₂ oder SO₂N(Me)₂ steht;
R'1 für H, CONH₂, CONHMe oder OMe steht,
R''1 für F, Cl, OH, OMe, CN, O-(CH₂)₃-OMe oder O-(CH₂)₃-N(Me)₂ steht;
und R ausgewählt ist der Gruppe bestehend aus: 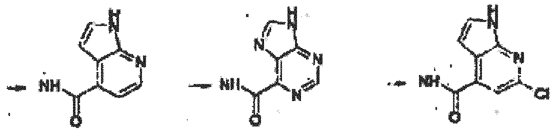 sowie deren Prodrugs, wobei die Produkte der Formel (I) in allen möglichen tautomeren, racemischen, enantiomeres oder diastereoisomeren isomeren Formen vorliegen, sowie die Additionssalze der Produkte der Formel (I) mit anorganischen und organischen Säuren oder mit anorganischen und organischen Basen.

7. Produkte der Formel (I) nach einem der anderen Ansprüche den folgenden Namen:
1H-Pyrrolo[2,3-b]pyridin-4-carbonsäure[5-(6-fluor-1H-benzimidazol-2-yl)-9H-pyrrolo[1,2-a]indol-9-yl]amid,
1H-Pyrrolo[2,3-b]pyridin-4-carbonsäure(5-chinolin-3-yl-9H-pyrrolo[1,2-a]indo1-9-yl)amid,
1H-Pyrrolo[2,3-b]pyridin-4-carbonsäure[5-(3H-imidazo[4,5-c]pyridin-2-yl)-9H-pyrrolo[1,2-a]-indol-9-yl]amid,
6-Chlor-1H-pyrrolo[2,3-b]pyridin-4-carbonsäure[5-(3H-imidazo[4,5-c]pyridin-2-yl)-9H-pyrrolo[1,2-a]-indol-9-yl]amid,
sowie die Additionssalze der Produkte der Formel (I) mit anorganischen und organischen Säuren oder mit anorganischen und organischen Basen.

8. Verfahren zur Herstellung der Produkte der Formel (I) nach einem der vorhergehenden Ansprüche, **gekennzeichnet durch** das nachstehende Schema (1):
gemäß dem die Produkte der allgemeinen Formel (I) ausgehend von einem Derivat der allgemeinen Formel (II) entweder über ein Derivat der allgemeinen Formel (III) (Methode A) oder über ein Derivat der allgemeinen Formel (IV) (Methode B) gemäß nachstehendem allgemeinem Schema (1) hergestellt werden können: 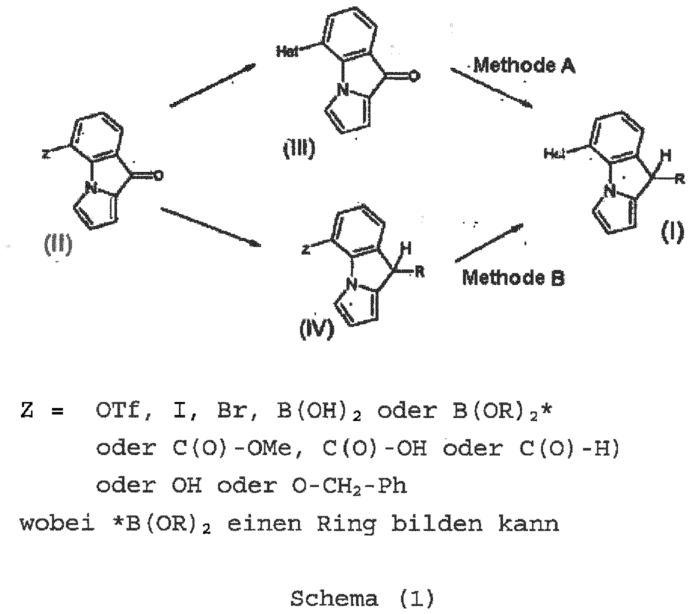 worin die Substituenten Het und R die für die Produkte der Formel (I) gemäß den vorhergehenden Ansprüchen angegebenen Bedeutungen besitzen and z die oben in Schema (1) angegebene Bedeutung besetzt.

9. Synthesezwischenprodukte der Formeln (II), (III) und (IV) als neue technische Produkte: 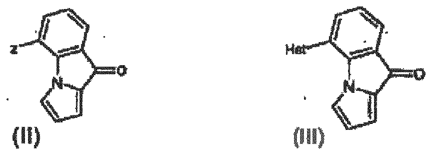 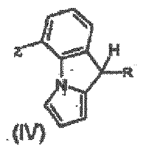 Z = OTf, I, Br, B(OH)₂ oder B(OR)₂*
oder C(O)-OMe, C(O)-OH oder C(O)-H)
oder O oder O-CH₂-Ph
wobei *B(OR)₂ einen Ring bilden kann wobei in den Verbindungen der Formeln (II), (III) und (IV) die Substituenten Heft und R die für die Produkte der Formel (I) gemäß einem der anderen Ansprüche angegebenen Bedeutungen besitzen und z die oben angegebene Bedeutung besitzt, mit Ausnahme der Verbindung der Formel (II), worin z für ein Iodatom steht.

10. Produkte der Formel (I) nach den Ansprüchen 1 bis 7, wobei die Produkte der Formel (I) in allen möglichen racemischen, enantiomeren oder diastereoisomeren isomeren Formen vorliegen, sowie die pharmazeutisch unbedenklichen Additionssalze der Produkte der Formel (I) mit anorganischen und organischen Säuren oder mit anorganischen und organischen Basen als Medikamente.

11. Produkte der Formel (I) nach Anspruch 6, wobei die Produkte der Formel (I) in allen möglichen racemischen, enantiomeren oder diastereoisomeren isomeren Formen vorliegen, sowie die pharmazeurisch unbedenklichen Additionssalze der Produkte der Formel (I) mit anorganischen und organischen Säuren oder mit anorganischen und organischen Basen als Medikamente.

12. Produkte der Formel (I) nach Anspruch 7, wobei die Produkte der Formel (I) in allen möglichen racemischen, enantiomeren oder diastereoisomeren isomeren Formen vorliegen, sowie die pharmazeutisch unbedenklichen Additionssalze der Produkte der Formel (I) mit anorganischen und organischen Säuren oder mit anorganischen und organischen Basen als Medikamente.

13. Pharmazeutische Zusammensetzungen, die als irkstoff mindestens eines der Medikamente nach den Ansprüchen 10 bis 12 entfalten.

14. Pharmazeutische Zusammensetzungen nach den vorhergehenden Ansprüchen, die außerdem Wirkstoffe anderer Medikamente für die Krebschemotherapie enthalten.

15. Pharmazeutische Zusammensetzungen nach einem der vorhergehenden Ansprüche, **dadurch gekennzeichnet, dass** sie als Medikamente verwendet werden, insbesondere für die Krebschemotherapie.

16. Verwendung von Produkten der Formel (I) nach einem der vorhergehenden Ansprüche oder von pharmazeutisch unbedenklichen Salzen der Produkte der Formel (I) zur Herstellung von Medikamenten zur Inhibierung der Aktivität von Chaperon-Proteinen und insbesondere Hsp90.

17. Verwendung von Produkten der Formel (I) nach eine der vorhergehenden Ansprüche oder von pharmazeutisch unbedenklichen Salzen der Produkte der Formel (I) zur Herstellung eines Medikamentes zur rävention oder Behandlung einer Erkrankung, die durch die Störun der Aktivität eines Chaperon-Proteins vom Hsp90-Typ gekennzeichnet ist,

18. Verwendung von Produkten der Formel (I) nach einem der vorhergehenden Ansprüche oder von pharmazeutisch unbedenklichen Salzen der Produkte der Formel (I) zur Herstellung eines Medikaments zur Prävention oder Behandlung einer Erkrankung aus der folgenden Gruppe: neurodegenerative Erkrankengen wie Chorea Huntington, Parkinson-Krankheit, fokale Hirnischämie, Alzheimer-Krankheit, multiple Sklerose und amyotrophe Lateralsklerose, Malaria, Brugia- und Bancroft-Filarie, Toxoplasmose, behandlungsresistente Mykosen, Hepatitis B, Hepatitis C, Herpesvirus, Dengue (oder Tropengrippe), spinale und bulbäre Muskelatrophie, Störungen der Mesangialzellenproliferation, Thrombosen, Retinopathien, Psoriasis, Muskeldegeneration, onkologische Erkrankungen, Krebserkrankungen.

19. Verwendung von Produkten der Formel (I) nach einem der vorhergehenden Ansprüche oder von pharmazeutisch unbedenklichen Salzen der Produkte der Formel (I) zur Herstellung eines Medikamentes zur Behandlung von Krebserkrankungen.

20. Verwendung von Produkten der Formel (I) nach dem vorhergehenden Anspruch, wobei es sich bei der zu behandelnden Erkrankung um einen Krebs mit soliden oder flüssigen Tumoren handelt.

21. Verwendung von Produkten der Formel (I) nach dem vorhergehenden Anspruch, wobei es sich bei der zu handelnden Erkrankung um einen Krebs handelt, der gegenüber Cytotoxika resistent ist.

22. Verwendung von Produkten der Formel (I) nach einem der vorhergehenden Ansprüche oder von harmazeutisch unbedenklichen Salzen der Produkte der Formel (I) zur Herstellung eines Medikaments zur Behandlung von Krebserkrankungen, u.a. Lungen-, Brust- und Eierstockkrebs, Glioblastomen, chronischen myeloischen Leukämien, akuten lymphoblastischen Leukämien, Prostatakrebs, Bauchspeicheldrüsenkrebs und Kolonkrebs, metastatischen Melanomen, Schilddrüsentumoren und Nierenkarzinomen.

23. Verwendung von Produkten der Formel (I) nach einem der vorhergehenden Ansprüche oder von pharmazeutisch unbedenklichen Salzen der Produkte der Formel (I) zur Herstellung eines Medikaments für die Krebschemotherapie.

24. Verwendung von Produkten der Formel (I) nach einem der vorhergehenden Ansprüche oder von pharmazeutisch unbedenklichen Salzen der Produkte der Formel (I) zur Herstellung von Medikamenten für die Krebschemotherapie, die allein oder in Kombination verwendet werden.

25. Verwendung von Produkten der Formel (I) nach einem der vorhergehenden Ansprüche oder von pharmazeutisch unbedenklichen Salzen der Produkte der Formel (I) zur Herstellung von Medikamenten zur alleinigen Verwendung oder zur Verwendung in Kombination mit Chemotherapie oder Strahlentherapie oder alternativ dazu in Kombination mit anderen Therapeutika.

26. Verwendung von Produkten der Formel (I) nach dem vorhergehenden Anspruch, wobei es sich bei den Therapeutika um gängige Antitumormittel handeln kann.

27. Produkte der Formel (I) nach eine der vorhergehenden Ansprüche als HSP90-Inhibitoren, wobei die Produkte der Formel (I) in allen möglichen tautomeren und/oder racemischen, enantiomeren oder diastereoisomeren isomeren Formen vorliegen, sowie Additionssalze der Produkte der Formel (I) mit anorganischen und organischen Säuren oder mit anorganischen organischen Basen.
